(19) Europäisches Patentamt
European Patent Office
Office européen des brevets

(11) **EP 3 527 287 A1**

(12) **EUROPÄISCHE PATENTANMELDUNG**

(43) Veröffentlichungstag:
**21.08.2019 Patentblatt 2019/34**

(51) Int Cl.:
*B01L 3/00* (2006.01)  *C12Q 1/68* (2018.01)
*G01N 21/03* (2006.01)  *G01N 21/64* (2006.01)
*B01L 7/00* (2006.01)

(21) Anmeldenummer: **18189028.6**

(22) Anmeldetag: **06.05.2005**

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **06.05.2004 DE 102004022263**

(62) Dokumentnummer(n) der früheren Anmeldung(en) nach Art. 76 EPÜ:
**10172432.6 / 2 256 478**
**05739547.7 / 1 761 641**

(71) Anmelder: **Alere Technologies GmbH**
**07749 Jena (DE)**

(72) Erfinder:
• **DWORRAK, Alexandra**
**07745 Jena (DE)**
• **ELLINGER, Thomas**
**07745 Jena (DE)**
• **ERMANTRAUT, Eugen**
**07749 Jena (DE)**
• **SCHULZ, Torsten**
**07749 Jena (DE)**
• **ULLRICH, Thomas**
**07745 Jena (DE)**
• **KAISER, Thomas**
**07743 Jena (DE)**
• **BICKEL, Ralf**
**07745 Jena (DE)**

(74) Vertreter: **Maiwald Patent- und Rechtsanwaltsgesellschaft mbH**
**Elisenhof**
**Elisenstraße 3**
**80335 München (DE)**

Bemerkungen:
Diese Anmeldung ist am 14-08-2018 als Teilanmeldung zu der unter INID-Code 62 erwähnten Anmeldung eingereicht worden.

(54) **VERFAHREN ZUM NACHWEIS VON MOLEKULAREN WECHSELWIRKUNGEN**

(57) Die Erfindung betrifft Vorrichtungen und Verfahren zum Nachweis von spezifischen Wechselwirkungen zwischen Sonden- und Targetmolekülen.

Insbesondere betrifft die Erfindung eine Vorrichtung zum qualitativen und/oder quantitativen Nachweis von molekularen Wechselwirkungen zwischen Sonden- und Targetmolekülen, umfassend:

a) einen Mikroarray mit einem Substrat, auf dem auf Array-Elementen Sondenmoleküle immobilisiert sind, wobei der Mikroarray auf einer ersten Fläche der Vorrichtung angeordnet ist; und

b) eine Reaktionskammer, die zwischen der ersten Fläche mit dem darauf angeordneten Mikroarray und einer zweiten Fläche gebildet ist,

wobei der Abstand zwischen dem Mikroarray und der zweiten Fläche veränderbar ist.

Abbildung 5

EP 3 527 287 A1

**Beschreibung**

Die Erfindung betrifft Vorrichtungen und Verfahren zum Nachweis von spezifischen Wechselwirkungen zwischen Target- und Sondenmolekülen.

Biomedizinische Tests basieren häufig auf dem Nachweis einer Wechselwirkung zwischen einem Molekül, das in bekannter Menge und Position vorhanden ist (der molekularen Sonde) und einem nachzuweisenden, unbekannten Molekül bzw. nachzuweisenden, unbekannten Molekülen (den molekularen Ziel- oder Targetmolekülen). Bei modernen Tests sind die Sonden in Form einer Substanzbibliothek auf Trägern, den so genannten Microarrays oder Mikroarrays oder Chips abgelegt, so dass eine Probe parallel an mehreren Sonden gleichzeitig analysiert werden kann (siehe z.B. D. J. Lockhart, E. A. Winzeler, Genomics, gene expression and DNA arrays; Nature 2000, 405, 827-836). Für die Herstellung der Microarrays werden die Sonden dabei üblicherweise in vorgegebener Art und Weise auf einer geeigneten, beispielsweise in WO 00/12575 beschriebenen Matrix immobilisiert (siehe z.B. US 5,412,087, WO 98/36827) bzw. synthetisch erzeugt (siehe z.B. US 5,143,854).

Voraussetzung für die Bindung eines beispielsweise mit einer Fluoreszenzgruppe markierten Targetmoleküls in Form eines DNA- oder RNA-Moleküls an eine Nukleinsäuresonde des Mikroarrays ist, dass sowohl Targetmolekül als auch Sondenmolekül in Form einer einzelsträngigen Nukleinsäure vorliegen. Nur zwischen solchen Molekülen kann eine effiziente und spezifische Hybridisierung stattfinden. Einzelsträngige Nukleinsäureziel- und Nukleinsäuresondenmoleküle erhält man in der Regel durch Hitzedenaturierung und optimale Wahl von Parametern wie Temperatur, Ionenstärke und Konzentration helixdestabilisierender Moleküle. Somit wird gewährleistet, dass nur Sonden mit nahezu perfekt komplementären, d.h. einander entsprechenden Sequenzen mit der Zielsequenz gepaart bleiben (A.A. Leitch, T. Schwarzacher, D. Jackson, I. J. Leitch, 1994, In vitro Hybridisierung, Spektrum Akademischer Verlag, Heidelberg/Berlin/Oxford).

Ein typisches Beispiel für die Verwendung von Mikroarrays in biologischen Testverfahren ist der Nachweis von Mikroorganismen in Proben in der biomedizinischen Diagnostik. Dabei macht man sich die Tatsache zunutze, dass die Gene für ribosomale RNA (rRNA) ubiquitär verbreitet sind und über Sequenzabschnitte verfügen, die für die jeweilige Spezies charakteristisch sind. Diese Spezies-charakteristischen Sequenzen werden in Form von einzelsträngigen DNA-Oligonukleotiden auf ein Mikroarray aufgebracht. Die zu untersuchenden Target-DNA-Moleküle werden zunächst aus der zu untersuchenden Probe isoliert und mit Markern, beispielsweise fluoreszierenden Markern versehen. Anschließend werden die markierten Target-DNA-Moleküle in einer Lösung mit den auf dem Mikroarray aufgebrachten Sonden inkubiert, unspezifisch auftretende Wechselwirkungen werden durch entsprechende Waschschritte entfernt und spezifische Wechselwirkungen durch fluoreszenzoptische Auswertung nachgewiesen. Auf diese Art und Weise ist es möglich, mit einem einzigen Test in einer Probe gleichzeitig z.B. mehrere Mikroorganismen nachzuweisen. Die Anzahl der nachweisbaren Mikroorganismen hängt bei diesem Testverfahren theoretisch nur von der Anzahl der spezifischen Sonden ab, die auf dem Mikroarray aufgebracht worden sind.

Zur Detektion molekularer Wechselwirkungen mit Hilfe von Mikroarrays bzw. Sonden-Arrays auf festen Oberflächen sind eine Reihe von Methoden und technischen Systemen beschrieben, von denen einige auch kommerziell erhältlich sind.

Klassische Systeme zur Detektion molekularer Wechselwirkungen beruhen auf dem Vergleich der Fluoreszenzintensitäten spektral selektiv angeregter, mit Fluorophoren markierter Targetmoleküle. Fluoreszenz ist die Eigenschaft von bestimmten Molekülen, bei Anregung mit Licht einer bestimmten Wellenlänge selber Licht zu emittieren. Dabei ergibt sich ein charakteristisches Absorptions- und Emissionsverhalten. Bei der Analyse wird mit steigender markierter Moleküldichte auf der funktionalisierten Oberfläche, z.B. durch steigende Effizienz der molekularen Wechselwirkung zwischen Target- und Sondenmolekülen, eine proportionale Zunahme des Fluoreszenzsignals angenommen.

Die insbesondere quantitative Detektion von Fluoreszenzsignalen wird mit modifizierten Verfahren der Fluoreszenzmikroskopie vorgenommen. Dabei wird das Licht der Absorptionswellenlänge von dem der Emissionswellenlänge mittels Filter oder Dichroiten getrennt und das Messsignal mittels optischer Elemente wie Objektiven und Linsen auf geeignete Detektoren wie z.B. zweidimensionale CCD-Arrays bildgebend abgebildet. Die Analyse erfolgt im Allgemeinen durch digitale Bildverarbeitung.

Bislang bekannte technische Lösungen unterscheiden sich hinsichtlich ihres optischen Aufbaus und der verwendeten Komponenten. Probleme und Limitationen können resultieren aus dem Signalrauschen (dem Hintergrund), das durch Effekte wie Bleichen und Quenching der verwendeten Farbstoffe, Autofluoreszenz der Medien, Assemblierungselemente und optischen Komponenten sowie Streuungen, Reflexionen und Fremdlicht im optischen Aufbau wesentlich bestimmt wird.

Daraus resultiert ein hoher technischer Aufwand zum Aufbau hochsensiver Fluoreszenz-Detektoren zum qualitativen und quantitativen Vergleich von Sonden-Arrays. Insbesondere zum Screening mit mittleren und hohen Durchsätzen sind speziell angepasste Detektionssysteme erforderlich, die einen gewissen Automatisierungsgrad besitzen.

Zur Optimierung von Standardepifluoreszenz-Aufbauten zum Auslesen molekularer Arrays sind CCD-basierte Detektoren bekannt, die zur Diskriminierung von optischen Effekten wie Streuung und Reflexionen die Anregung der Fluorophore im Dunkelfeld durch Auflicht oder Durchlicht realisieren (siehe z.B. C. E. Hooper et al., Quantitative Photon Imaging in the Life Sciences Using Intensified CCD Cameras, Journal of Bioluminescence and Chemiluminescence (1990), S. 337-344). Die Abbildung der Arrays erfolgt dabei entweder in einer Belichtung oder durch ein Rastern unter Verwendung von höher auflösender Optik. Die Verwendung von multispektralen Belichtungsquellen ermöglicht einen relativ einfachen Zugang zu verschiedenen Fluorophoren durch die Verwendung verschiedener Anregungsfilter (-kombinationen). Nachteilig ist allerdings, dass Autofluoreszenz und systembedingte optische Effekte wie die Beleuchtungshomogenität über dem Array komplizierte Beleuchtungsoptiken und Filtersysteme erfordern.

Weitere Methoden zur quantitativen Detektion von Fluoreszenzsignalen basieren auf der konfokalen Fluoreszenzmikroskopie. Beispielsweise in US 5,304,810 beschriebene konfokale Scanning-Systeme beruhen auf der Selektion der Fluoreszenzsignale entlang der optischen Achse mittels zweier Pinholes. Daraus ergibt sich ein hoher Justieraufwand der Proben bzw. die Etablierung eines leistungsfähigen Autofokussystems. Solche Systeme sind in der technischen Lösung hochkomplex. Erforderliche Komponenten wie Laser, Pinholes, gegebenenfalls gekühlte Detektoren wie z.B. PMT, Avalanche-Dioden oder CCD, komplexe hochgenaue mechanische Translationselemente und Optiken müssen mit erheblichem Aufwand aufeinander optimiert und integriert werden (siehe z.B. US 5,459,325; US 5,192,980; US 5,834,758). Miniaturisierungsgrad und Preis sind durch die Vielzahl und Funktionalität der Komponenten limitiert.

Analysen basierend auf Sonden-Arrays werden zum derzeitigen Zeitpunkt in der Regel fluoreszenzoptisch ausgelesen (siehe z.B. A. Marshall und J. Hodgson, DNA Chips: An array of possibilities, Nature Biotechnology, 16, 1998, 27-31; G. Ramsay, DNA Chips: State of the Art, Nature Biotechnology, 16, Jan. 1998, 40-44). Nachteilig an den vorstehend beschriebenen Detektionsvorrichtungen und -verfahren ist jedoch der hohe Signalhintergrund, der zu einer eingeschränkten Genauigkeit führt, der teilweise erhebliche technische Aufwand sowie die hohen Kosten, die mit den Nachweisverfahren verbunden sind.

Es sind eine Reihe insbesondere konfokaler Systeme bekannt, die für die Detektion von niederintegrierten Substanzbibliotheken im Array-Format geeignet sind, die in fluidischen Kammern angebracht sind (siehe z.B. US 5,324,633, US 6,027,880, US 5,585,639, WO 00/12759).

Zur Detektion von hochintegrierten molekularen Arrays, die insbesondere in fluidischen Systemen angebracht sind, sind die oben beschriebenen Methoden und Systeme vor allem wegen der dort auftretenden Streuungen, Reflexionen und optischen Aberrationen allerdings nur sehr bedingt adaptierbar. Ferner werden bei derartigen hochintegrierten Arrays hohe Anforderungen hinsichtlich der räumlichen Auflösung gestellt, die jedoch bislang technisch nicht realisiert werden konnten.

Es besteht folglich ein Bedarf an hoch integrierten Arrays, mit denen mit relativ geringem technischem Aufwand die Wechselwirkung zwischen Sonden und Targets mit hoher Genauigkeit qualitativ und/oder quantitativ nachgewiesen werden kann. Die Erhöhung der Selektivität und der Zugang zu alternativen Komponenten motiviert die Etablierung alternativer Imaging-Technologien wie Fluoreszenz-Polarisation und zeitaufgelöste Fluoreszenz für festkörpergebundene Assays. Der Effekt der Verdrehung der Polarisationsachse durch polarisiert angeregte Fluorophore wird zur Quantifizierung im Mikrotiter-Format angewandt. Es gibt ferner Ansätze, durch die Verwendung entsprechend modifizierter Polymerfolien als Polarisationsfilter kostengünstige Systeme mit hohem Durchsatz (HTS-Systeme) aufzubauen (siehe I. Gryczcynski et al., Polarisation sensing with visual detection, Anal. Chem. 1999, 71, 1241-1251).

Neuere Entwicklungen nutzen die Fluoreszenz von anorganischen Materialien, wie zum Beispiel von Lanthaniden (M. Kwiatowski et al., Solid-phase synthesis of chelate-labelled oligonucleotides: application in triple-color ligase-mediated gene analysis, Nucleic Acids Research, 1994, 22, 13) und Quantendots (M. P. Bruchez et. al., Semiconductor nanocrystals as fluorescent biological labels, Science 1998, 281, 2013). Farbstoffe mit langer Emissionsdauer im Mikrosekunden-Bereich wie Lanthanid-Gelate erfordern eine Umwandlung der Farbstoffe in eine mobile Phase, so dass eine ortsaufgelöste Detektion nicht möglich ist.

In der internationalen Patentanmeldung WO 00/72018 sind optische Aufbauten zur Detektion von mittels Goldbeads markierten Proben und deren Sichtbarmachung mittels Silberverstärkung beschrieben. Die dortigen Vorrichtungen sind allerdings lediglich für eine Detektion bei statischer Messung geeignet. Bei der statischen Messung wird nach der Wechselwirkung der Targets mit dem auf dem Sondenarray angeordneten Sonden sowie nach Beginn der Reaktion, die zu einem Niederschlag auf Array-Elementen führt, an denen eine Wechselwirkung stattgefunden hat, ein Bild aufgenommen und den gemessenen, von dem Grad der Niederschlagsbildung abhängigen Grauwerten Konzentrationen zugeordnet.

In WO 02/02810 wurde ein Verfahren zum qualitativen und/oder quantitativen Nachweis von Targets in einer Probe durch molekulare Wechselwirkungen zwischen Sonden und Targets auf Sonden-Arrays bereitgestellt, bei dem der zeitliche Verlauf der Niederschlagsbildung an den Array-Elementen in Form von Signalintensitäten detektiert wird, d.h. eine dynamische Messung durchgeführt wird. Jedem Array-Element wird dann anhand einer Kurvenfunktion, die die Niederschlagsbildung als Funktion der Zeit beschreibt, ein Wert zugeordnet, der die Wechselwirkung zwischen Sonde und Target auf einem Array-Element und damit die Menge an gebundenen Targets quantifiziert.

Für eine derartige dynamische Messung ist die Aufnahme von Bilderserien unter z.B. bestimmten thermischen Bedingungen bzw. in einem bestimmten Verfahrensstadium, z.B. bei Vorhandensein bestimmter Lösungen zur Zeit der Aufnahme, erforderlich. Dies bedingt ein komplexes Zusammenwirken der einzelnen Bauteile eines hochintegrierten Arrays insbesondere bei Anwendungen im Bereich des Genotyping.

Bei vielen Tests in der biomedizinischen Diagnostik tritt ferner das Problem auf, dass die Targetmoleküle zunächst nicht in einer für eine Detektion ausreichenden Menge vorhanden sind und deshalb häufig zunächst vor dem eigentlichen Testverfahren aus der Probe vervielfältigt werden müssen. Die Vervielfältigung von DNA-Molekülen geschieht typischerweise durch die Polymerase-Kettenreaktion (PCR). Für die Vervielfältigung von RNA müssen die RNA-Moleküle durch reverse Transkription in entsprechend komplementäre DNA (cDNA) umgewandelt werden. Diese cDNA kann dann ebenfalls durch PCR vervielfältigt (amplifiziert) werden. Bei der PCR handelt es sich um eine Labor-Standardmethode (wie z.B. in Sambrook et al. (2001) Molecular Cloning: A laboratory manual, 3rd edition, Cold Spring Harbor, N.Y., Cold Spring Harbor Laboratory Press).

Die Vervielfältigung von DNA durch PCR ist verhältnismäßig schnell, ermöglicht durch miniaturisierte Verfahren einen hohen Probendurchsatz in geringen Ansatzvolumina und ist durch Automatisierung arbeitseffizient.

Eine Charakterisierung von Nukleinsäuren durch eine alleinige Vervielfältigung ist jedoch nicht möglich. Vielmehr ist es notwendig, nach der Amplifikation Analysemethoden wie Nukleinsäuresequenzbestimmungen, Hybridisierung und/oder elektrophoretische Trenn- und Isolationsverfahren zur Charakterisierung der PCR-Produkte einzusetzen.

Generell sollten Vorrichtungen und Verfahren zur Amplifikation von Nukleinsäuren und deren Nachweis so konzipiert sein, dass möglichst wenige Eingriffe seitens eines Experimentators notwendig sind. Die Vorteile von Verfahren, die eine Vervielfältigung von Nukleinsäuren und deren Nachweis ermöglichen und in deren Verlauf ein Experimentator nur minimal eingreifen muss, liegen auf der Hand. Zum einen werden Kontaminationen vermieden. Zum anderen ist die Reproduzierbarkeit solcher Verfahren wesentlich erhöht, da sie einer Automatisierung zugänglich sind. Dies ist auch im Hinblick auf die arzneimittelrechtliche Zulassung von diagnostischen Verfahren extrem wichtig.

Es gibt gegenwärtig eine Vielzahl von Verfahren zur Amplifikation von Nukleinsäuren und deren Nachweis, bei denen zunächst das Target-Material durch PCR-Amplifikation vervielfältigt wird und die Identität bzw. der genetische Zustand der Zielsequenzen anschließend durch Hybridisierung gegen einen Sondenarray bestimmt wird. Die Amplifikation der nachzuweisenden Nukleinsäure- bzw. Target-Moleküle ist in der Regel notwendig, um ausreichende Mengen für einen qualitativen und quantitativen Nachweis im Rahmen der Hybridisierung zur Verfügung zu haben.

Sowohl die PCR-Amplifikation von Nukleinsäuren als auch deren Nachweis durch Hybridisierung ist einer Reihe von grundlegenden Problemen unterworfen. Dies gilt in gleicher Weise für Verfahren, die eine PCR-Amplifikation von Nukleinsäuren und deren Nachweis durch Hybridisierung kombinieren.

Werden in einem Verfahren, das eine PCR-Amplifikation und deren Nachweis durch Hybridisierung kombiniert, detektierbare Marker beispielsweise in Form von Fluoreszenz-markierten Primern in die nachzuweisenden Nukleinsäuren bzw. Target-Moleküle eingebracht, wird üblicherweise vor der eigentlichen Detektion ein Waschschritt durchgeführt. Ein derartiger Waschschritt dient der Entfernung der im Vergleich zum Amplifikationsprodukt in großem Überschuss vorliegenden, nicht umgesetzten Primer sowie solcher mit einem Fluoreszenzmarker versehenen Nukleotide, die nicht an der Nachweisreaktion teilnehmen bzw. nicht spezifisch mit den Nukleinsäuresonden des Mikroarrays hybridisieren. Auf diese Weise soll der hohe Signalhintergrund vermindert werden, der durch diese Moleküle verursacht wird. Durch einen derartigen zusätzlichen Verfahrensschritt wird allerdings das Nachweisverfahren deutlich verlangsamt. Ferner wird das detektierbare Signal auch für die nachzuweisenden Nukleinsäuren deutlich verringert, die spezifisch mit den Nukleinsäuresonden des Mikroarrays hybridisieren. Letzteres beruht vor allem darauf, dass das Gleichgewicht zwischen den durch Hybridisierung gebundenen und in Lösung befindlichen Targets nach dem Waschschritt nicht mehr gegeben ist. Nukleinsäuren, die bereits mit den auf dem Array befindlichen Nukleinsäuresonden hybridisiert hatten, werden von der Bindungsstelle durch das Waschen abgelöst und somit mit den in Lösung befindlichen Molekülen weggewaschen. Es bleibt überhaupt nur dann ein detektierbares Signal zurück, wenn der Wasch- bzw. Spülschritt der in Lösung befindlichen Moleküle schneller vollzogen wird als die Ablösung der bereits hybridisierten Nukleinsäuren erfolgt.

Es besteht folglich ein Bedarf an hochintegrierten Arrays, mit denen mit relativ geringem technischem Aufwand die Wechselwirkung zwischen Sonden und Targets mit hoher Genauigkeit qualitativ und/oder quantitativ nachgewiesen werden kann. Es besteht ferner ein Bedarf an Vorrichtungen, die die Durchführung von PCR und Analysereaktion, wie z.B. einer Hybridisierungsreaktion, in einem Reaktionsraum ermöglichen.

Aufgabe der vorliegenden Erfindung ist es somit, die vorstehend genannten Probleme des Standes der Technik, die sich insbesondere durch die mangelnde Kompatibilität des Assays mit dem Testsystem ergeben, zu überwinden.

Insbesondere ist es eine Aufgabe der vorliegenden Erfindung, Vorrichtungen bzw. Verfahren zur Verfügung zu stellen, mit denen molekulare Wechselwirkungen zwischen Sonden und Targets auf Sonden-Arrays mit hoher Genauigkeit und hoher Empfindlichkeit und auf einfache und kostengünstige Weise qualitativ und/oder quantitativ nachgewiesen werden können.

Ferner ist es eine Aufgabe der vorliegenden Erfindung, Verfahren bzw. Vorrichtungen zur Amplifikation und zum qualitativen und quantitativen Nachweis von Nukleinsäuren zur Verfügung zu stellen, bei denen die Eingriffe seitens des Experimentators in das Nachweisverfahren minimiert werden können.

Eine weitere Aufgabe der vorliegenden Erfindung ist es, Verfahren bzw. Vorrichtungen zum qualitativen und/oder quantitativen Nachweis von Targetmolekülen zur Verfügung zu stellen, mit denen ein hohes Signal-zu-Rausch-Verhältnis bei der Detektion von Wechselwirkungen auf dem Mikroarray gewährleistet wird, ohne die Wechselwirkung zwischen den Target- und den Sondenmolekülen auf dem Array zu beeinträchtigen.

Eine weitere Aufgabe der vorliegenden Erfindung ist es, Vorrichtungen bzw. Verfahren zur Verfügung zu stellen, mit denen eine hohe dynamische Auflösung bei der Detektion erreicht wird, d.h. der Nachweis schwacher Sonden/Target-Wechselwirkungen neben starken Signalen gewährleistet bleibt.

Der Erfindung liegt ferner die Aufgabe zugrunde, Vorrichtungen bzw. Verfahren zur Verfügung zu stellen, die eine nahezu gleichzeitige Vervielfältigung und Charakterisierung von Nukleinsäuren mit einem hohen Durchsatz ermöglichen.

Diese und weitere Aufgaben der vorliegenden Erfindung werden durch die Bereitstellung der in den Patentansprüchen gekennzeichneten Ausführungsformen gelöst.

Erfindungsgemäß werden Verfahren zum qualitativen und/oder quantitativen Nachweis von molekularen Wechselwirkungen zwischen Sonden- und Targetmolekülen zur Verfügung gestellt, bei denen auf den Austausch und/oder die Entfernung von Lösungen, d.h. insbesondere auf Wasch- bzw. Spülschritte, verzichtet werden kann.

Derartige erfindungsgemäße Verfahren umfassen insbesondere die folgenden Schritte:

a) Einbringen einer Probe enthaltend Targetmoleküle in eine Reaktionskammer, die einen Mikroarray aufweist, wobei der Mikroarray ein Substrat mit darauf auf Array-Elementen immobilisierten Sondenmoleküle umfasst;
b) Detektieren einer Wechselwirkung zwischen den Targetmolekülen und den auf dem Substrat immobilisierten Sondenmolekülen,

wobei nach dem Einbringen der Probe enthaltend Targetmoleküle und vor und während dem Detektieren kein Austauschen von Lösungen in der Reaktionskammer und/oder Entfernen von Lösungen aus der Reaktionskammer erfolgt.

Ferner werden im Rahmen der vorliegenden Erfindung Vorrichtungen bereitgestellt, die zur Durchführung derartiger Verfahren geeignet sind.

Insbesondere wird im Rahmen der vorliegenden Erfindung eine Vorrichtung zum qualitativen und/oder quantitativen Nachweis von molekularen Wechselwirkungen zwischen Sonden- und Targetmolekülen zur Verfügung gestellt, umfassend:

a) einen Mikroarray mit einem Substrat, auf dem auf Array-Elementen Sondenmoleküle immobilisiert sind, wobei der Mikroarray auf einer ersten Fläche der Vorrichtung angeordnet ist; und
b) eine Reaktionskammer, die zwischen der ersten Fläche mit dem darauf angeordneten Mikroarray und einer zweiten Fläche gebildet ist,

wobei der Abstand zwischen dem Mikroarray und der zweiten Fläche veränderbar ist.

Insbesondere ermöglicht der Variabilität des Abstands zwischen Mikroarray und zweiter Fläche, die üblicherweise die Detektionsebene der erfindungsgemäßen Vorrichtung darstellt, dass der Signalhintergrund, der durch markierte Targetmoleküle verursacht wird, die keine spezifische Affinität zu den Sondenmolekülen des Mikroarrays aufweisen und deshalb nicht mit diesen wechselwirken, wesentlich vermindert bzw. vollständig vermieden werden kann.

Erfindungsgemäß wird ferner ein Verfahren zum qualitativen und/oder quantitativen Nachweis von molekularen Wechselwirkungen zwischen Sonden- und Targetmolekülen zur Verfügung gestellt, das die folgenden Schritte umfasst:

a) Einbringen einer Probenlösung umfassend Targetmoleküle in eine Reaktionskammer einer wie vorstehend beschriebenen erfindungsgemäßen Vorrichtung; und
b) Detektieren einer Wechselwirkung zwischen den Targetmolekülen und den auf dem Substrat immobilisierten Sondenmolekülen.

Die erfindungsgemäßen Verfahren und Vorrichtungen zum Nachweis von Targetmolekülen sind so konzipiert, dass zur Durchführung des Nachweisverfahrens und ggf. einer Amplifikation der Targetmoleküle möglichst wenige Eingriffe seitens eines Experimentators in die Reaktionskammer notwendig sind. Dies bietet den wesentlichen Vorteil, dass dadurch Kontaminationen vermieden werden. Ferner ist die Reproduzierbarkeit der erfindungsgemäßen Verfahren im Vergleich zu herkömmlichen Verfahren wesentlich erhöht, da das erfindungsgemäße Verfahren aufgrund der Minimierung externer Eingriffe einer Automatisierung zugänglich ist. Die vorstehend genannten Vorteile spielen im Hinblick auf die Zulassung von diagnostischen Verfahren eine bedeutende Rolle.

Zur Beschreibung der vorliegenden Erfindung werden ferner unter anderen folgende Definitionen verwendet: Unter einer Sonde bzw. einem Sondenmolekül bzw. einer molekularen Sonde wird im Rahmen der vorliegenden Erfindung ein Molekül verstanden, das zum Nachweis anderer Moleküle durch ein bestimmtes, charakteristisches Bindungsverhalten bzw. eine bestimmte Reaktivität verwendet wird. Für die auf dem Array angeordneten Sonden kommt jede Art von Molekülen in Frage, die sich an feste Oberflächen koppeln lassen und eine spezifische Affinität aufweisen. In einer bevorzugten Ausführungsform handelt es sich um Biopolymere, insbesondere um Biopolymere aus den Klassen der Peptide, Proteine, Antigene, Antikörper, Kohlenhydrate, Nukleinsäuren und/oder deren Analoga und/oder Mischpolymere der vorstehend genannten Biopolymere. Besonders bevorzugt sind die Sonden Nukleinsäuren und/oder Nukleinsäureanaloga.

Als Sonde werden insbesondere Nukleinsäuremoleküle definierter und bekannter Sequenz bezeichnet, die benutzt werden, um in Hybridisierungsverfahren Target-Moleküle nachzuweisen. Als Nukleinsäuren können sowohl DNA- als auch RNA-Moleküle verwendet werden. Beispielsweise kann es sich bei den Nukleinsäurensonden bzw. Oligonukleotidsonden um Oligonukleotide mit einer Länge von 10 bis 100 Basen, vorzugsweise 15 bis 50 Basen und besonders bevorzugt von 20 bis 30 Basen Länge handeln. Typischerweise handelt es sich erfindungsgemäß bei den Sonden um einzelsträngige Nukleinsäuremoleküle oder Moleküle von Nukleinsäureanaloga, bevorzugt einzelsträngige DNA-Moleküle oder RNA-Moleküle, die mindestens über einen Sequenzbereich verfügen, der zu einem Sequenzbereich der Target-Moleküle komplementär ist. Je nach Nachweisverfahren und Anwendung können die Sonden auf einem festen Trägersubstrat, z.B. in Form eines Mikroarrays immobilisiert sein. Darüber hinaus können sie je nach Nachweisverfahren radioaktiv oder nicht radioaktiv markiert sein, so dass sie über im Stand der Technik übliche Nachweisverfahren nachgewiesen werden können.

Unter einem Target bzw. einem Targetmolekül wird im Rahmen der vorliegenden Erfindung das mit einer molekularen Sonde nachzuweisende Molekül verstanden. Bei einer bevorzugten Ausführungsform der vorliegenden Erfindung handelt es sich bei den zu detektierenden Targets um Nukleinsäuren. Der erfindungsgemäße Sonden-Array kann jedoch analog auch zum Nachweis von Peptid/Sonden-Wechselwirkungen, Protein/Sonden-Wechselwirkungen, Kohlenhydrat/Sonden-Wechselwirkungen, Antikörper/Sonden-Wechselwirkungen, usw. eingesetzt werden.

Falls es sich im Rahmen der vorliegenden Erfindung bei den Targets um Nukleinsäuren bzw. Nukleinsäuremoleküle handelt, die durch eine Hybridisierung gegen auf einem Sondenarray angeordnete Sonden nachgewiesen werden, umfassen diese Target-Moleküle in der Regel Sequenzen mit einer Länge von 40 bis 10.000 Basen, bevorzugt von 60 bis 2.000 Basen, ebenfalls bevorzugt von 60 bis 1.000 Basen, insbesondere bevorzugt von 60 bis 500 Basen und am meisten bevorzugt von 60 bis 150 Basen. Ihre Sequenz beinhaltet gegebenenfalls die Sequenzen von Primern, sowie die durch die Primer definierten Sequenzbereiche des Templates. Bei den Target-Molekülen kann es sich insbesondere um einzel- oder doppelsträngige Nukleinsäuremoleküle handeln, von denen ein Strang oder beide Stränge radioaktiv oder nicht radioaktiv markiert sind, so dass sie in einem der im Stand der Technik üblichen Nachweisverfahren nachgewiesen werden können.

Als Target-Sequenz wird erfindungsgemäß der Sequenzbereich des Targets bezeichnet, der durch Hybridisierung mit der Sonde nachgewiesen wird. Erfindungsgemäß wird auch davon gesprochen, dass dieser Bereich durch die Sonde adressiert wird.

Unter einer Substanzbibliothek wird im Rahmen der vorliegenden Erfindung eine Vielzahl von unterschiedlichen Sondenmolekülen verstanden, vorzugsweise mindestens zwei bis 1.000.000 unterschiedliche Moleküle, besonders bevorzugt mindestens 10 bis 10.000 unterschiedliche Moleküle und am meisten bevorzugt zwischen 100 und 1.000 unterschiedlichen Molekülen. Bei speziellen Ausgestaltungen kann eine Substanzbibliothek auch nur mindestens 50 oder weniger oder mindestens 30.000 unterschiedliche Moleküle umfassen. Die Substanzbibliothek ist vorzugsweise als Array auf einem Träger in der Reaktionskammer der erfindungsgemäßen Vorrichtung angeordnet.

Unter einem Sonden-Array wird im Rahmen der vorliegenden Erfindung eine Anordnung von molekularen Sonden bzw. einer Substanzbibliothek auf einem Träger verstanden, wobei die Position einer jeden Sonde separat bestimmt ist. Vorzugsweise umfasst der Array definierte Stellen bzw. vorbestimmte Bereiche, so genannte Array-Elemente, die besonders bevorzugt in einem bestimmten Muster angeordnet sind, wobei jedes Array-Element üblicherweise nur eine Spezies an Sonden beinhaltet. Die Anordnung der Moleküle bzw. Sonden auf dem Träger kann durch kovalente oder nicht kovalente Wechselwirkungen erzeugt werden. Die Sonden sind dabei auf der dem Reaktionsraum zugewandten Seite des Trägers angeordnet. Eine Position innerhalb der Anordnung, d.h. des Arrays, wird üblicherweise als Spot bezeichnet.

Unter einem Array-Element bzw. einem vorbestimmten Bereich bzw. einem Spot bzw. einem Array-Spot wird im Rahmen der vorliegenden Erfindung ein für die Deposition einer molekularen Sonde bestimmtes Areal auf einer Oberfläche verstanden, die Summe aller belegten Array-Elemente ist das Sonden-Array.

Unter einem Trägerelement bzw. Träger bzw. Substanzbibliothekenträger bzw. Substrat wird im Rahmen der vorliegenden Erfindung ein Festkörper verstanden, auf dem das Sonden-Array aufgebaut ist. Bei dem Träger, der üblicherweise auch als Substrat oder Matrix bezeichnet wird, kann es sich z.B. um Objektträger oder Wafer oder aber auch keramische Materialien handeln. Bei einer speziellen Ausgestaltung können die Sonden auch direkt auf der ersten Fläche, vorzugsweise auf einem Teilbereich der ersten Fläche, immobilisiert sein.

Die Gesamtheit aus in Array-Anordnung auf dem Substrat abgelegten Molekülen bzw. der in Array-Anordnung auf dem Substrat bzw. der Detektionsfläche abgelegten Substanzbibliothek und dem Träger bzw. Substrat wird häufig auch als "Chip", "Mikroarray", "Microarray", "DNA-Chip", Sondenarray", "Sonden-Array" etc. bezeichnet.

Als Detektionsebene wird im Rahmen der vorliegenden Erfindung die zweite Fläche der erfindungsgemäßen Vorrichtung bezeichnet. Vorzugsweise befinden sich die auf dem Mikroarray abgelegten Sonden bei der Detektion der Wechselwirkung zwischen Sonden und Targets im Wesentlichen in der Detektionsebene, insbesondere dadurch, dass der Abstand zwischen Mikroarray und zweiter Fläche auf etwa null verringert ist.

Unter einem Kammerkörper wird im Rahmen der vorliegenden Erfindung der die Reaktionskammer bildende Festkörper verstanden. Der Substanzbibliothekenträger bzw. der Chip ist üblicherweise Teil des Kammerkörpers, wobei der Substanzbibliothekenträger aus einem anderen Material gebildet sein kann als der übrige Kammerkörper.

Unter einer Reaktionskammer bzw. einem Reaktionsraum wird im Rahmen der vorliegenden Erfindung der Raum bezeichnet, der zwischen Mikroarray und zweiter Fläche bzw. Detektionsebene gebildet ist und vorzugsweise als veränderbarer Kapillarspalt ausgestaltet ist. Der Reaktionsraum ist seitlich durch Seitenwände begrenzt, die z.B. als elastische Dichtungen ausgeführt sein können. Die auf dem Mikroarray immobilisierten Sonden befinden sich auf der dem Innenraum der Reaktionskammer zugewandten Seite. Die Grundfläche der Reaktionskammer bzw. des Reaktionsraums ist durch die erste Fläche bzw. die zweite Fläche des Arrays definiert. Als Dicke des Reaktionsraums bzw. der Reaktionskammer bzw. des Kapillarspalts wird insbesondere der Abstand zwischen zweiter Fläche bzw. Detektionsebene und Oberfläche des Substrats bzw. des Mikroarrays bezeichnet. Üblicherweise weist ein Reaktionsraum im Rahmen der vorliegenden Erfindung eine geringe Dicke auf, beispielsweise eine Dicke von höchstens 1 cm, vorzugsweise von höchstens 5 mm, besonders bevorzugt von höchstens 3 mm und am meisten bevorzugt von höchstens 1 mm.

Unter dem Abstand zwischen dem Mikroarray und der zweiten Fläche wird im Rahmen der vorliegenden Erfindung der Abstand zwischen der Oberfläche des Mikroarray-Substrats, d.h. der dem Reaktionsraum zugewandten Seite des Mikroarrays, und der dem Reaktionsraum zugewandten Seite der zweiten Fläche verstanden. Ist der Abstand zwischen Mikroarray und zweiter Fläche etwa null, bedeutet dies, dass die Oberfläche des Substrats bündig auf der zweiten Fläche aufliegt.

Unter einem Kapillarspalt wird im Rahmen der vorliegenden Erfindung ein Reaktionsraum bezeichnet, der durch Kapillarkräfte, die zwischen dem Mikroarray und der zweiten Fläche wirken, befüllbar ist. Üblicherweise weist ein Kapillarspalt eine geringe Dicke z.B. von höchstens 1 mm, vorzugsweise von höchstens 750 $\mu$m und besonders bevorzugt von höchstens 500 $\mu$m auf. Als Dicke des Kapillarspalts ist erfindungsgemäß ferner eine Dicke im Bereich von 10 bis 300 $\mu$m, von 15 $\mu$m bis 200 $\mu$m bzw. von 25 $\mu$m bis 150 $\mu$m bevorzugt. Bei speziellen Ausgestaltungen der vorliegenden Erfindung weist der Kapillarspalt eine Dicke von 50 $\mu$m, 60 $\mu$m, 70 $\mu$m, 80 $\mu$m oder 90 $\mu$m auf. Weist der Reaktionsraum bzw. die Reaktionskammer eine Dicke von mehr als 2 mm auf, wird der Reaktionsraum bzw. die Reaktionskammer im Rahmen der vorliegenden Erfindung nicht mehr als Kapillarspalt bezeichnet.

Unter einer Kartusche oder Reaktionskartusche wird im Rahmen der vorliegenden Erfindung eine Einheit aus der Reaktionskammer mit einem Kammerkörper und einer entsprechenden Umhausung verstanden.

Unter einem konfokalen Fluoreszenzdetektionssystem wird im Rahmen der vorliegenden Erfindung ein Fluoreszenzdetektionssystem verstanden, in der das Objekt in der Brennebene des Objektivs durch eine Punktlichtquelle beleuchtet wird. Punktlichtquelle, Objekt und Punktlichtdetektor liegen dabei in exakt optisch konjugierten Ebenen. Beispiele für konfokale Systeme sind in A. Diaspro, Confocal and 2-photon-microscopy: Foundations, Applications and Advances, Wiley-Liss, 2002 beschrieben.

Unter einem das gesamte Volumen der Reaktionskammer abbildenden fluoreszenzoptischen System wird im Rahmen der vorliegenden Erfindung ein nichtkonfokales Fluoreszenzdetektionssystem verstanden, also ein Fluoreszenzdetektionssystem, in der die Beleuchtung durch die Punktlichtquelle nicht auf das Objekt begrenzt ist. Ein derartiges Fluoreszenzdetektionssystem weist somit keine fokale Begrenzung auf.

Herkömmliche Arrays bzw. Mikroarrays im Rahmen der vorliegenden Erfindung umfassen etwa 50 bis 10.000, vorzugsweise 150 bis 2.000 unterschiedliche Spezies von Sondenmolekülen auf einer, vorzugsweise quadratischen, Fläche von z.B. 1 mm bis 4 mm x 1 mm bis 4 mm, vorzugsweise von 2 mm x 2 mm. In weiteren Ausgestaltungen umfassen Mikroarrays im Rahmen der vorliegenden Erfindung etwa 50 bis etwa 80.000, vorzugsweise etwa 100 bis etwa 65.000, besonders bevorzugt etwa 1.000 bis etwa 10.000 unterschiedliche Spezies von Sondenmolekülen auf einer Fläche von mehreren mm$^2$ bis mehreren cm$^2$, vorzugsweise etwa 1 mm$^2$ bis 10 cm$^2$, besonders bevorzugt 2 mm$^2$ bis 1 cm$^2$ und am meisten bevorzugt etwa 4 mm$^2$ bis 6,25 mm$^2$. Beispielsweise weist ein herkömmliches Mikroarray von 100 bis 65.000 unterschiedliche Spezies von Sondenmolekülen auf einer Fläche von 2 mm x 2 mm auf.

Eine Markierung oder ein Marker bezeichnet im Rahmen der vorliegenden Erfindung eine detektierbare Einheit, beispielsweise ein Fluorophor oder eine Ankergruppe, an die eine detektierbare Einheit gekoppelt werden kann.

Eine Vervielfältigungsreaktion bzw. eine Amplifikationsreaktion umfasst im Rahmen der vorliegenden Erfindung üblicherweise 10 bis 50 oder mehr Amplifikationszyklen, vorzugsweise etwa 25 bis 45 Zyklen, besonders bevorzugt etwa 40 Zyklen. Eine zyklische Amplifikationsreaktion ist im Rahmen der vorliegenden Erfindung vorzugsweise eine Polymerase-Kettenreaktion (polymerase chain reaction, PCR).

Als Amplifikationszyklus wird im Rahmen der vorliegenden Erfindung ein einzelner Verstärkungsschritt der zyklischen Amplifikationsreaktion bezeichnet. Ein Verstärkungsschritt der PCR wird auch als PCR-Zyklus bezeichnet.

Als Amplifikationsprodukt wird im Rahmen der vorliegenden Erfindung ein Produkt aus der Verstärkung bzw. Vervielfältigung bzw. Amplifikation der zu amplifizierenden Nukleinsäuremoleküle durch die zyklische Amplifikationsreaktion, vorzugsweise durch die PCR bezeichnet. Ein durch PCR vervielfältigtes Nukleinsäuremolekül wird auch als PCR-Produkt bezeichnet.

Unter der Denaturierungstemperatur wird im Rahmen der vorliegenden Erfindung die Temperatur verstanden, bei der die doppelsträngige DNA im Amplifikationszyklus aufgetrennt wird. Die Denaturierungstemperatur beträgt, insbesondere bei einer PCR, üblicherweise mehr als 90°C, vorzugsweise etwa 95°C.

Unter der Annealing-Temperatur wird im Rahmen der vorliegenden Erfindung die Temperatur verstanden, bei der die Primer an die nachzuweisende Nukleinsäure hybridisieren. Die Annealing-Temperatur liegt, insbesondere bei einer PCR, üblicherweise im Bereich von 50°C bis 65°C und beträgt vorzugsweise etwa 60°C.

Unter der Kettenverlängerungs- bzw. Extensions-Temperatur wird im Rahmen der vorliegenden Erfindung die Temperatur verstanden, bei der die Nukleinsäure durch Einbau der Monomerbausteine synthetisiert wird. Die Extensionstemperatur liegt, insbesondere bei einer PCR, üblicherweise im Bereich von etwa 68°C bis etwa 75°C und beträgt vorzugsweise etwa 72°C.

Als Oligonukleotid-Primer bzw. Primer wird im Rahmen der vorliegenden Erfindung ein Oligonukleotid bezeichnet, das an die nachzuweisende DNA, auch Target-DNA genannt, bindet bzw. hybridisiert, wobei von der Bindungsstelle die Synthese des Gegenstrangs der nachzuweisenden DNA bei der zyklischen Amplifikationsreaktion startet. Insbesondere wird als Primer üblicherweise ein kurzes DNA- oder RNA-Oligonukleotid mit vorzugsweise etwa 12 bis 30 Basen bezeichnet, das komplementär zu einem Abschnitt eines größeren DNA- oder RNA-Moleküls ist und über eine freie 3'-OH-Gruppe an seinem 3'-Ende verfügt. Aufgrund dieser freien 3'-OH-Gruppe kann der Primer als Substrat für beliebige DNA- oder RNA-Polymerasen dienen, die in 5'-3'-Richtung Nukleotide an den Primer synthetisieren. Die Sequenz der neu synthetisierten Nukleotide ist dabei durch die Sequenz des mit dem Primer hybridisierten Templates vorgegeben, die jenseits der freien 3'-OH-Gruppe des Primers liegt. Primer üblicher Länge umfassen zwischen 12 bis 50 Nukleotide, bevorzugt zwischen 15 und 30 Nukleotide.

Als Template oder Template-Strang werden üblicherweise ein doppelsträngiges Nukleinsäuremolekül oder ein Nukleinsäurestrang bezeichnet, der als Vorlage zur Synthese von komplementären Nukleinsäuresträngen dient.

Unter einer molekularen Wechselwirkung bzw. einer Wechselwirkung wird im Rahmen der vorliegenden Erfindung insbesondere eine spezifische, kovalente oder nicht-kovalente Bindung zwischen einem Targetmolekül und einem immobilisierten Sondenmolekül verstanden. Bei einer bevorzugten Ausführungsform der vorliegenden Erfindung ist die Wechselwirkung zwischen Sonden- und Targetmolekülen eine Hybridisierung.

Als Hybridisierung wird die Bildung von doppelsträngigen Nukleinsäuremolekülen oder Duplexmolekülen aus komplementären einzelsträngigen Nukleinsäuremolekülen bezeichnet. Dabei findet die Assoziation vorzugsweise immer zu Paaren von A und T bzw. G und C statt. Im Rahmen einer Hybridisierung können z.B. DNA-DNA-Duplexes, DNA-RNA- oder RNA-RNA-Duplexes gebildet werden. Durch eine Hybridisierung können auch Duplexes mit Nukleinsäureanaloga gebildet werden, wie z.B. DNA-PNA-Duplexes, RNA-PNA-Duplexes, DNA-LNA-Duplexes und RNA-LNA-Duplexes. Hybridisierungsexperimente werden üblicherweise benutzt, um die Sequenzkomplementarität und damit die Identität zwischen zwei verschiedenen Nukleinsäuremolekülen nachzuweisen.

Unter Prozessierung werden im Rahmen der vorliegenden Erfindung insbesondere Aufreinigungs-, Aufkonzentrierungs-, Markierungs-, Amplifikations-, Wechselwirkungs- bzw. Hybridisierungs- und/oder Wasch- und Spülschritte, sowie weitere zum Nachweis bzw. zur Detektion von Targets mit Hilfe von Substanzbibliotheken durchgeführte Verfahrensschritte verstanden. Die Detektion selbst fällt nicht unter den Begriff Prozessierung.

Als Probe bzw. Probenlösung bzw. Analyt bzw. Lösung wird im Rahmen der vorliegenden Erfindung eine zu analysierende Flüssigkeit bezeichnet, die insbesondere die nachzuweisenden und ggf. zu amplifizierenden Targetmoleküle enthält. Eine derartige Lösung kann ferner u.a. neben üblichen Zusatzstoffen wie beispielsweise Puffern auch für die Durchführung von Amplifikationsreaktionen erforderliche Substanzen wie Primer enthalten.

Unter einem Austauschen von Lösungen in der Reaktionskammer aus der Reaktionskammer werden im Rahmen der vorliegenden Erfindung insbesondere Spül- bzw. Waschschritte verstanden. Das Austauschen von Lösungen dient z.B. zur Beseitigung von mit detektierbaren Markern versehenen Molekülen, welche nicht spezifisch mit Sonden auf dem Mikroarray wechselwirken, in dem nach erfolgter Wechselwirkung die Probenlösung gegen eine nicht markierte Lösung ausgetauscht wird. Moleküle, welche nicht spezifisch mit Sonden auf dem Mikroarray wechselwirken, sind z.B. mit einem detektierbaren Marker versehene Primern, die nicht während der Amplifikationsreaktion umgesetzt worden sind, oder mit einem detektierbaren Marker versehene Targetmoleküle, zu denen keine komplementäre Sonde auf dem Array vorliegt, die spezifisch mit diesem Targetmolekül wechselwirkt.

Unter einem Entfernen von Lösungen aus der Reaktionskammer werden im Rahmen der vorliegenden Erfindung Schritte verstanden, mit denen mit detektierbaren Markern versehene Moleküle, welche nicht spezifisch mit Sonden auf dem Mikroarray wechselwirken, aus der Reaktionskammer entfernt werden. Moleküle, welche nicht spezifisch mit Sonden auf dem Mikroarray wechselwirken, sind z.B. mit einem detektierbaren Marker versehene Primern, die nicht während der Amplifikationsreaktion umgesetzt worden sind, oder mit einem detektierbaren Marker versehene Targetmoleküle, zu denen keine komplementäre Sonde auf dem Array vorliegt, die spezifisch mit diesem Targetmolekül wechselwirkt.

Wenn im Rahmen der vorliegenden Erfindung zwischen dem Einbringen der Probe enthaltend Targetmoleküle in eine Reaktionskammer und dem Detektieren der Wechselwirkung kein Austauschen von Lösungen in der Reaktionskammer und/oder Entfernen von Lösungen aus der Reaktionskammer erfolgt, ist es jedoch denkbar, dass in diesem Zeitraum Lösungen zusätzlich in die Reaktionskammer eingebracht werden können, ohne dass ein Austausch bzw. ein Entfernen von bereits in der Reaktionskammer vorliegenden Lösungen erfolgt.

Ein erster Gegenstand der vorliegenden Erfindung umfasst somit ein Verfahren zum qualitativen und/oder quantitativen Nachweis von molekularen Wechselwirkungen zwischen Sonden- und Targetmolekülen, umfassend insbesondere die folgenden Schritte:

  a) Einbringen einer Probe enthaltend Targetmoleküle in eine Reaktionskammer, die einen Mikroarray aufweist, wobei der Mikroarray ein Substrat mit darauf auf Array-Elementen immobilisierten Sondenmoleküle umfasst;
  b) Detektieren einer Wechselwirkung zwischen den Targetmolekülen und den auf dem Substrat immobilisierten Sondenmolekülen,

wobei nach dem Einbringen der Probe enthaltend Targetmoleküle und vor bzw. während dem Detektieren kein Austauschen von Lösungen in der Reaktionskammer und/oder Entfernen von Lösungen aus der Reaktionskammer erfolgt.

Ein wesentliches Merkmal des erfindungsgemäßen Verfahrens bei diesem Aspekt der vorliegenden Erfindung ist, dass die Detektion einer Wechselwirkung zwischen den nachzuweisenden Targetmolekülen und den auf dem Substrat des Mikroarrays immobilisierten Sondenmolekülen erfolgt, ohne dass ein Austauschen von Lösungen in der Reaktionskammer bzw. ein Entfernen von Lösungen aus der Reaktionskammer erfolgt. D.h., das Detektieren der Wechselwirkung zwischen Targets und Sonden kann erfolgen, ohne dass im Anschluss an die Wechselwirkungsreaktion Spül- bzw. Waschschritte erforderlich sind und/oder ohne dass im Anschluss an die Wechselwirkungsreaktion Moleküle aus der Reaktionskammer entfernt werden, die nicht spezifisch mit Sonden auf dem Mikroarray wechselwirken.

Dies kann insbesondere durch fokiselektive Detektionsmethoden bei dem erfindungsgemäßen Verfahren gewährleistet werden, wie z.B. durch konfokale Techniken oder durch auf der Anwendung einer tiefenselektiven Beleuchtung aufgrund der z.B. auf totaler Reflexion beruhenden evaneszenten Auskopplung von Anregungslicht (TIRF) im Probensubstrat oder der Verwendung von Wellenleitern beruhende Methoden. Derartige fokiselektive Verfahren sind insbesondere dann zu bevorzugen, wenn eine weitere Ausgrenzung der durch die in der Flüssigkeit vorhandenen, d.h. nicht hybridsierten Fluoreszenzmoleküle verursachten Hintergrundsignale erforderlich ist, um die Sensitivität zu erhöhen. Bei Verwendung von fluoreszenzmarkierten Targetmolekülen können somit die spezifischen Wechselwirkungssignale von der Hintergrundfluoreszenz durch Einsatz von Verfahren wie Total Internal Reflection-Fluoreszenzmikroskopie (TIRF) oder konfokaler Fluoreszenzmikroskopie diskriminiert werden.

Beispiele hierfür sind CCD-basierte Detektoren, die zur Diskriminierung von optischen Effekten wie Streuung und Reflexionen die Anregung der Fluorophore im Dunkelfeld durch Auflicht oder Durchlicht realisieren (siehe z.B. C. E. Hooper et al., Quantitative Photone Imaging in the Life Sciences Using Intensified CCD Cameras, Journal of Bioluminescence and Chemoluminescence (1990), 337-344). Weitere Alternativen für Fluoreszenzdetektionssysteme, die bei dem erfindungsgemäßen Verfahren eingesetzt werden können, sind Weißlichtaufbauten wie sie z.B. in WO 00/12759, WO 00/25113 und WO 96/27025 beschrieben sind; konfokale Systeme, wie sie z.B. in US 5,324,633, US 6,027,880, US 5,585,639 und WO 00/12759 beschrieben sind; auf Nipkow-Scheiben basierende konfokale Anregungssysteme bei bildgebender konfokaler Abbildung, wie z.B. in US 5,760,950 beschrieben sind; auf strukturierter Anregungsverteilung basierende Systeme, wie sie z.B. in WO 98/57151 beschrieben sind; Fluoreszenzdetektionssysteme in hoher Integration unter Verwendung von Mikrooptiken, wie sie z.B. in WO 99/27140 beschrieben sind; und Laserscanningsysteme, wie sie z.B. in WO 00/12759 beschrieben sind. Ein allgemeiner Ablauf von Fluoreszenzdetektionsverfahren unter Verwendung derartiger herkömmlicher Fluoreszenzdetektionssysteme ist z.B. in US 5,324,633 beschrieben.

Für die Durchführung eines erfindungsgemäßen Nachweisverfahren ohne Austauschen von Lösungen in der Reaktionskammer und/oder Entfernen von Lösungen aus der Reaktionskammer vor der Detektion sind insbesondere die in WO 2004/087951 beschriebenen Vorrichtungen geeignet, in denen die Reaktionskammer durch einen Kapillarspalt gebildet wird. Auf den diesbezüglichen Inhalt von WO 2004/087951 wird hiermit ausdrücklich Bezug genommen.

Bei einer weiteren Ausführungsform dieses Aspekts der vorliegenden Erfindung wird das Austauschen und/oder Entfernen von Lösungen aus der Reaktionskammer dadurch vermieden, dass der Nachweis durch Detektion der Massenänderung auf der Arrayoberfläche, wie z.B. in WO 03/004699 beschrieben, erfolgt. Auf den diesbezüglichen Inhalt von WO 03/004699 wird hiermit ausdrücklich Bezug genommen.

Bei einer weiteren Ausführungsform dieses Aspekts der vorliegenden Erfindung wird das Austauschen und/oder Entfernen von Lösungen aus der Reaktionskammer dadurch vermieden, dass der Nachweis durch Detektion von akustischen Oberflächenwellen, wie beispielsweise in Z. Guttenberg et al., Lab Chip. 2005; 5(3):308-17 beschrieben, erfolgt.

Bei einer weiteren Ausführungsform dieses Aspekts der vorliegenden Erfindung wird das Austauschen und/oder Entfernen von Lösungen aus der Reaktionskammer dadurch vermieden, dass der Nachweis durch elektrochemische Detektion mittels Elektroden auf der Oberfläche des Arrays erfolgt, wie beispielsweise durch Messung der Änderung von Redoxpotentialen (siehe z.B. X. Zhu et al., Lab Chip. 2004; 4(6):581-7) oder zyklische Voltometrie (siehe z.B. J. Liu et al., Anal Chem. 2005; 77(9):2756-2761; J. Wang, Anal Chem. 2003; 75(15):3941-5) erfolgt.

Bei einer weiteren Ausführungsform dieses Aspekts der vorliegenden Erfindung wird das Austauschen und/oder Entfernen von Lösungen aus der Reaktionskammer dadurch vermieden, dass der Nachweis durch elektrische Detektion mittels Elektroden auf der Oberfläche des Arrays erfolgt, wie beispielsweise durch Impedanzmessung (siehe u.a. S.M. Radke et al., Biosens Bioelectron. 2005; 20(8):1662-7).

Bei einer weiteren Ausführungsform dieses Aspekts der vorliegenden Erfindung wird das Austauschen und/oder Entfernen von Lösungen aus der Reaktionskammer dadurch vermieden, dass ein Mikroarray mit FRET-Sonden (FRET, *fluorescence resonance energy transfer*) eingesetzt wird. Die Verwendung derartiger FRET-Sonden basiert auf der Bildung von Fluoreszenz-Quencher-Pärchen, so dass nur dann ein Fluoreszenzsignal entsteht, wenn ein Targetmolekül an die komplentäre Sonde auf der Oberfläche gebunden hat. Die Verwendung von FRET-Sonden ist beispielsweise beschrieben in B. Liu et al., PNAS 2005, 102, 3, 589-593; K. Usui et al., Mol Divers. 2004; 8(3):209-18; J.A. Cruz-Aguado et al., Anal Chem. 2004; 76(14):4182-8 und J. Szollosi et al., J Biotechnol. 2002;82(3):251-66.

Bei einer weiteren besonders bevorzugten Ausführungsform dieses Aspekts der vorliegenden Erfindung wird das Austauschen und/oder Entfernen von Lösungen aus der Reaktionskammer dadurch vermieden, dass eine wie nachstehend im Detail beschriebene erfindungsgemäße Vorrichtung zum qualitativen und/oder quantitativen Nachweis von molekularen Wechselwirkungen zwischen Sonden- und Targetmolekülen eingesetzt wird, wobei die Vorrichtung umfasst:

a) einen Mikroarray mit einem Substrat, auf dem auf Array-Elementen Sondenmoleküle immobilisiert sind, wobei der Mikroarray auf einer ersten Fläche der Vorrichtung angeordnet ist; und
b) eine Reaktionskammer, die zwischen der ersten Fläche mit dem darauf angeordneten Mikroarray und einer zweiten Fläche gebildet ist,

und wobei der Abstand zwischen dem Mikroarray und der zweiten Fläche veränderbar ist.

Ein weiterer Gegenstand der vorliegenden Erfindung betrifft die Verwendung von FRET-Sondenmolekülen wie vorstehend beschrieben und/oder Detektionsverfahren ausgewählt aus der Gruppe bestehend aus Total Internal Reflection-Fluoreszenzmikroskopie (TIRF) wie vorstehend beschrieben, konfokaler Fluoreszenzmikroskopie wie vorstehend beschrieben, Verfahren zur Detektion der Massenänderung wie vorstehend beschrieben, Verfahren zur Detektion von akustischen Oberflächenwellen wie vorstehend beschrieben, Verfahren zur elektrochemischen und/oder elektrischen Detektion wie vorstehend beschrieben, zur Vermeidung eines Austauschens von Lösungen in einer Reaktionskammer und/oder Entfernens von Lösungen aus einer Reaktionskammer zwischen bzw. nach dem Einbringen einer Probe enthaltend Targetmoleküle in die Reaktionskammer und vor bzw. während der Detektion bei einem Verfahren zum qualitativen und/oder quantitativen Nachweis von molekularen Wechselwirkungen zwischen Sonden- und Targetmolekülen, das insbesondere die folgenden Schritte umfasst:

a) Einbringen einer Probe enthaltend Targetmoleküle in eine Reaktionskammer, die einen Mikroarray aufweist, wobei der Mikroarray ein Substrat mit darauf auf Array-Elementen immobilisierten Sondenmoleküle umfasst;
b) Detektieren einer Wechselwirkung zwischen den Targetmolekülen und den auf dem Substrat immobilisierten Sondenmolekülen.

Ein weiterer Gegenstand der vorliegenden Erfindung ist insbesondere eine Vorrichtung zum qualitativen und/oder quantitativen Nachweis von molekularen Wechselwirkungen zwischen Sonden- und Targetmolekülen, umfassend:

a) einen Mikroarray mit einem Substrat, auf dem auf Array-Elementen Sondenmoleküle immobilisiert sind, wobei der Mikroarray auf einer ersten Fläche der Vorrichtung angeordnet ist; und
b) eine Reaktionskammer, die zwischen der ersten Fläche mit dem darauf angeordneten Mikroarray und einer zweiten Fläche gebildet ist,

wobei der Abstand zwischen dem Mikroarray und der zweiten Fläche veränderbar ist.

Nach erfolgter Wechselwirkung zwischen Sondenmolekülen und Targetmolekülen wird durch die in der Probenlösung vorliegenden markierten Moleküle, die nicht mit den Sondenmolekülen wechselwirken, ein unerwünschter Hintergrund verursacht. Handelt es sich bei den Sonden- und/oder Targetmolekülen um Nukleinsäuren und/oder Nukleinsäureanaloga, so wird dieser Hintergrund insbesondere durch die in der Probenlösung vorliegenden markierten Primer und/oder markierten Nukleinsäuren verursacht, die nicht mit den Sondenmolekülen hybridisiert sind.

Eine bekannte Möglichkeit zur Entfernung störender Hintergrundsignale ist der Austausch der Probenlösung nach erfolgter Wechselwirkung gegen eine nicht markierte, beispielsweise nicht fluoreszierende Lösung. Diese Variante ist jedoch im Allgemeinen bedingt durch Korrosion, Alterung der Lösungen und Dichtigkeitsprobleme, aufwendig und störanfällig.

Wesentliches Merkmal der erfindungsgemäßen Vorrichtung ist, dass der Abstand zwischen dem Mikroarray und der zweiten Fläche veränderbar ist. Ein variabler Abstand zwischen Mikroarray und zweiter Fläche bedeutet, dass die Reaktionskammer der erfindungsgemäßen Vorrichtung komprimierbar ist. Insbesondere ist der Abstand zwischen Mikroarray und zweiter Fläche derart variabel, dass der Mikroarray bündig und/oder reversibel mit seiner aktiven Seite, d.h. der Seite, auf der die Nukleinsäuresonden immobilisiert sind, an der zweiten Fläche anliegen kann bzw. auf diese gedrückt werden kann.

Eine komprimierbare Reaktionskammer ermöglicht somit durch Verringerung des Abstands zwischen Mikroarray und Detektionsebene vor Durchführung der Detektion die Verdrängung von Probenlösung, die markierte Moleküle enthält, die nicht mit den Sondenmolekülen wechselwirken und somit einen unerwünschten Hintergrund darstellen. Auf diese Weise ist eine Detektion von Wechselwirkungen zwischen Sonden- und Targetmolekülen mit beliebigen optischen Detektionssystemen möglich, ohne die Probenlösung vor der Detektion gegen eine nicht markierte Lösung auszutauschen. Beispielsweise ist eine einfache fluoreszenzmikroskopische Abbildung des DNA-Chips zur Detektion der Wechselwirkungssignale mit der erfindungsgemäßen Vorrichtung ohne Austausch der Probenlösung gegen eine nicht markierte, insbesondere fluoreszenzarme Flüssigkeit möglich.

Schließlich wird insbesondere durch die im Folgenden beschriebene Ausführungsformen der erfindungsgemäßen Vorrichtung gewährleistet, dass eine Fokussierung von optischen Detektionssystemen nicht mehr erforderlich ist. Die erfindungsgemäße Vorrichtung ermöglicht somit beispielsweise, dass im Gegensatz zu den bislang verwendeten fluoreszenzoptischen Detektionssystemen zum Nachweis von Nukleinsäuren ein einfaches Fluoreszenzmikroskopgerät ohne Autofokus-Funktion als Auslesegerät für die Detektion der Hybridisierung zwischen Targets und Sonden verwendet werden kann, ohne dass flüssigkeitshandhabende Schritte wie insbesondere Waschschritte zur Entfernung nicht an den Array gebundener Targetmoleküle, wie z.B. nicht hybridisierter Target-Nukleinsäuren, erforderlich sind.

Dadurch wird trotz der mit der erfindungsgemäßen Vorrichtung durchführbaren multifunktionalen Probenbehandlung und Analyse ein äußerst kostengünstiges System zum Nachweis und ggf. Amplifizieren von Targetmolekülen in einer Probe bereitgestellt. Die erfindungsgemäßen Vorrichtungen, insbesondere in Verbindung mit einem optischen Detektionssystem, sind ferner derart robust, dass sie auch für den mobilen Einsatz verwendet werden können.

Durch die geeignete Wahl von Chip, Verarbeitungsprotokollen und Analysechemikalien ist die erfindungsgemäße Vorrichtung für unterschiedlichste Arten von Genanalysen wie z.B. Prädispositionsdiagnostik, Erregerdiagnostik und Typisierung einsetzbar. In der erfindungsgemäßen Vorrichtung, die auch als Einweg-Kartusche ausgeführt sein kann, ist somit eine vollständige genetische Analyse mit geringem apparativem Aufwand durchführbar. Die erfindungsgemäße Vorrichtung ermöglicht damit die Durchführung von Nachweisverfahren am Ort des Geschehens, z.B. bei einer Blutspende. Ein Messergebnis kann innerhalb kurzer Zeit, vorzugsweise innerhalb ½ h bis 2 h, vorliegen. Sämtliche mit der erfindungsgemäßen Vorrichtung durchführbaren Schritte wie Aufreinigung, Aufarbeitung, Amplifikation von Nukleinsäuren und die eigentliche Hybridisierung sind automatisch durchführbar. Der Operator muss lediglich mit der Probenentnahme, der Aufgabe der Probe in die erfindungsgemäße Vorrichtung sowie der Kenntnisnahme der Analysenergebnisse vertraut sein.

Vorzugsweise ist der Abstand zwischen dem Mikroarray und der zweiten Fläche in einem Bereich von etwa 0 bis etwa 1 mm veränderbar. Weitere bevorzugte untere Grenzen für den Abstand zwischen Mikroarray und zweiter Fläche sind etwa 0,1 $\mu$m, etwa 1 $\mu$m und etwa 10 $\mu$m. Weitere bevorzugte obere Grenzen für den Abstand zwischen Mikroarray und zweiter Fläche sind etwa 0,01 mm, etwa 0,5 mm, etwa 1 mm und am meisten bevorzugt etwa 0,3 mm. Überraschenderweise wird die Wechselwirkung zwischen Sonden und Targets auf der Array-Oberfläche auch dann nicht beeinträchtigt, wenn der Abstand zwischen Substrat-Oberfläche und zweiter Fläche nahezu null bzw. etwa null ist.

Vorzugsweise umfasst die erfindungsgemäße Vorrichtung ferner ein Detektionssystem. Dabei ist es bevorzugt, dass das Detektionssystem ein optisches System ist. Beispiele für im Rahmen der vorliegenden Erfindung geeignete optische Systeme sind Detektionssysteme basierend auf Fluoreszenz, optischer Absorption, Resonanztransfer u. dgl.. Vorzugsweise ist das optische Detektionssystem ein fluoreszenzoptisches System. Besonders bevorzugt ist das fluoreszenzoptische System ein Fluoreszenzmikroskop ohne Autofokus, z.B. ein Fluoreszenzmikroskop mit Fixfokus.

In einer weiteren Ausführungsform ist das Detektionssystem mit mindestens einem Abstandshalter verbunden, der bei Auflage auf der zweiten Fläche einen Abstand zwischen dem Detektionssystem und der zweiten Fläche einstellt. Ist der Abstand zwischen Mikroarray und zweiter Fläche in etwa null, so legt der Abstandshalter auch den Abstand zwischen der Oberfläche des Chips und dem optischen System des Detektionsgerätes fest. Dies ermöglicht es, die Varianz des Abstands zwischen optischer Detektionsvorrichtung und Mikroarray-Oberfläche sehr gering zu halten. Die Varianz umfasst lediglich die Dickenvarianz der zweiten Fläche, im Allgemeinen eine Glasfläche, die Durchbiegung der zweiten Fläche sowie die Dicke einer durch etwaige Verunreinigungen an den Anpressflächen zwischen Chip und Detektionsebene bzw. zwischen Abstandshalter und Detektionsebene verursachten Schicht. Dadurch wird eine Nachfokussierung zur Scharfstellung der optischen Systems überflüssig, was die Handhabung des Gerätes erheblich vereinfacht und/oder eine kostspielige Autofokuseinrichtung überflüssig macht.

In einer weiteren Ausführungsform sind für den zwischen der ersten und zweiten Fläche gebildeten Reaktionsraum seitlich begrenzende Ausgleichsbereiche vorgesehen, die bei Verringerung des Abstands zwischen Mikroarray und zweiter Fläche das Volumen in der Reaktionskammer in Wesentlichen konstant halten.

Vorzugsweise ist ferner der zwischen der ersten und zweiten Fläche gebildete Reaktionsraum seitlich durch elastische Dichtungen begrenzt. Bei den elastischen Dichtungen handelt es sich besonders bevorzugt um Silikonkautschuk.

Um die Detektion von Wechselwirkungen zwischen Sonden- und Targetmolekülen zu gewährleisten, ist die zweite Fläche insbesondere aus einem optisch durchlässigen Material, vorzugsweise Glas ausgestaltet.

In einer weiteren Ausführungsform der erfindungsgemäßen Vorrichtung ist die erste Fläche zumindest im Bereich unterhalb des Mikroarrays derart ausgestaltet, dass der Mikroarray relativ zur zweiten Fläche so führbar ist, dass der Abstand zwischen dem Mikroarray und der zweiten Fläche veränderbar ist.

Dabei kann die erste Fläche zumindest im Bereich unterhalb des Mikroarrays derart ausgestaltet sein, dass der Mikroarray in Richtung der zweiten Fläche so führbar ist, dass der Abstand zwischen dem Mikroarray und der zweiten Fläche verringerbar ist und/oder dass der Mikroarray in einer Richtung entgegengesetzt zur zweiten Fläche so führbar ist, dass der Abstand zwischen dem Mikroarray und der zweiten Fläche vergrößerbar ist.

Bei dieser Ausführungsform ist es bevorzugt, dass die erste Fläche zumindest im Bereich unterhalb des Mikroarrays elastisch verformbar ist. Besonders bevorzugt ist die erste Fläche aus einem elastischen Kunststoff, z.B. einer elastischen Membran, ausgestaltet.

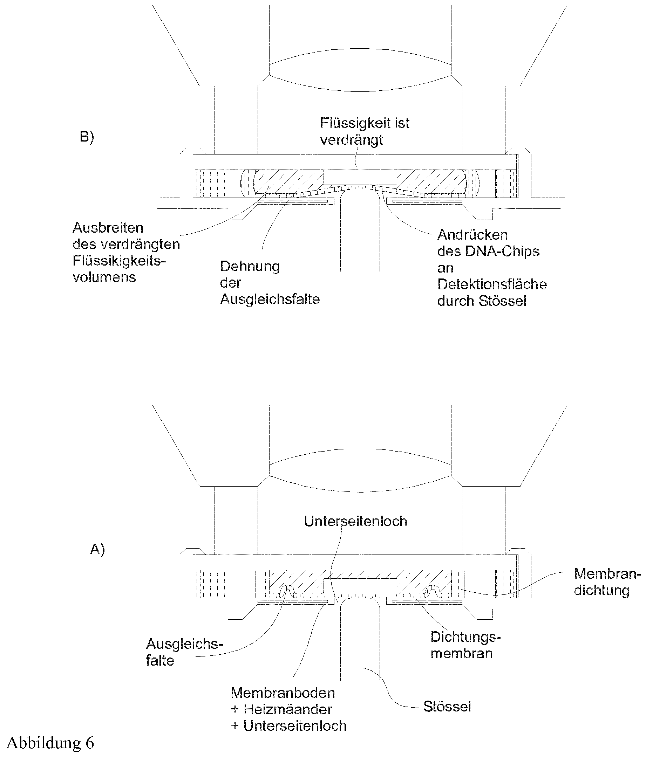

Ferner kann es bevorzugt sein, dass die erste Fläche mittels zweier übereinander liegender Schichten ausgestaltet ist, wobei eine äußere Schicht der beiden übereinander liegenden Schichten zumindest im Bereich unterhalb des Mikroarrays eine Aussparung aufweist. Bei dieser Ausgestaltung ist es bevorzugt, dass eine innere der beiden übereinander liegenden Schichten aus einer elastischen Dichtung bzw. einer Dichtungsmembran gebildet ist, die üblicherweise auch den Reaktionsraum seitlich begrenzt (siehe Abbildung 6). Die Dichtungsmembran ist in Richtung der zweiten Fläche führbar. Durch die Dichtungsmembran wird eine Aussparung der äußeren Schicht, die üblicherweise der Unterseite des Kammerkörpers entspricht, verschlossen. Bei Durchführung einer PCR in der Reaktionskammer entsteht durch die bei einer PCR vorliegenden höheren Temperaturen ein Innendruck, der dazu führt, dass die Reaktionskammer trotz der relativ labilen Dichtungsmembran druckfest ist. Diese Ausgestaltung entspricht somit einem selbst schließenden Ventil. Um die Elastizität der Dichtungsmembran zu gewährleisten, ist die Membran vorzugsweise mit einer Ausgleichsfalte versehen (siehe Abbildung 6).

Ferner kann es vorgesehen sein, dass die Vorrichtung mindestens ein Mittel umfasst, mit dem der Mikroarray relativ zur zweiten Fläche führbar ist. Dieses Mittel wird im Folgenden auch als Mittel zur Führung der ersten Fläche bezeichnet. Das Mittel zur Führung der ersten Fläche wird vorzugsweise ausgewählt aus der Gruppe bestehend aus einem Stab, einem Stift, einem Stößel und einer Schraube.

Dabei kann die Vorrichtung mindestens ein Mittel zur Führung der ersten Fläche umfassen, mit dem der Mikroarray in Richtung zur zweiten Fläche so führbar ist, dass der Abstand zwischen dem Mikroarray und der zweiten Fläche verringerbar ist, und/oder mit dem der Mikroarray in einer Richtung entgegengesetzt zur zweiten Fläche so führbar ist, dass der Abstand zwischen dem Mikroarray und der zweiten Fläche vergrößerbar ist.

Besonders bevorzugt ist der Mikroarray durch Druck und/oder Zug des Mittels auf die erste Fläche relativ zur zweiten Fläche führbar.

Dabei können die vorstehend genannten auf der zweiten Fläche aufliegenden Abstandshalter als Widerlager für das Mittel zur Führung der ersten Fläche dienen.

Ferner kann es bevorzugt sein, dass die erste Fläche durch das Mittel zur Führung der ersten Fläche in Vibration, insbesondere in eine Vibration mit einer Frequenz von 10 bis 30 Hz, besonders bevorzugt von etwa 20 Hz versetzbar ist. Auf diese Weise können oberhalb des Chips vorliegende Blasen, die eine Detektion behindern würden, entfernt werden und/oder die Wechselwirkungsgeschwindigkeit, z.B. die Hybridisierungsgeschwindigkeit, durch eine durch die Vibration des Mittels zur Führung der ersten Fläche bedingte Durchmischung erhöht werden.

Ebenso kann es bevorzugt sein, dass die zweite Fläche relativ zur ersten Fläche so führbar ist, dass der Abstand zwischen dem Mikroarray und der zweiten Fläche veränderbar ist.

Dabei kann die zweite Fläche relativ zur ersten Fläche so führbar sein, dass der Abstand zwischen dem Mikroarray und der zweiten Fläche verringerbar ist und/oder dass der Abstand zwischen dem Mikroarray und der zweiten Fläche vergrößerbar ist.

Dies kann insbesondere dadurch gewährleistet werden, indem die zweite Fläche durch Druck und/oder Zug des Abstandshalters auf die zweite Fläche relativ zur ersten Fläche so führbar ist, dass der Abstand zwischen dem Mikroarray und der zweiten Fläche veränderbar ist.

Bei einer weiteren bevorzugten Ausführungsform der erfindungsgemäßen Vorrichtung ist sowohl die erste Fläche als auch die zweite Fläche so führbar, dass der Abstand zwischen dem Mikroarray und der zweiten Fläche veränderbar ist.

Bei einer weiteren Ausführungsform ist die erfindungsgemäße Vorrichtung so ausgestaltet, dass der auf der ersten Fläche angebrachte Mikroarray bereits im ursprünglichen Zustand auf der die Detektionsebene bildenden zweiten Fläche aufliegt, vorzugsweise bündig aufliegt. Die erste Fläche ist so führbar, dass der der Abstand zwischen dem Mikroarray und der zweiten Fläche vergrößerbar ist. Vorzugsweise ist die erste Fläche dabei aus einem elastischen Material gebildet.

Bei einer weiteren Ausführungsform der erfindungsgemäßen Vorrichtung ist die erste Fläche um eine Drehachse schwenkbar ausgestaltet. Die Drehachse unterteilt die erste Fläche in zwei Schenkelabschnitte. Der Mikroarray ist bei dieser Ausgestaltung auf einem ersten Schenkelabschnitt der ersten Fläche angeordnet. Die Drehachse für die Schwenkbewegung verläuft vorzugsweise in der Mitte der ersten Fläche, d.h. die beiden Schenkelabschnitte sind vorzugsweise gleich groß. Die erste Fläche ist vorzugsweise aus einem elastischen Material ausgestaltet.

In einer ersten Position der schwenkbaren ersten Fläche ist die erste Fläche im Wesentlichen parallel zur zweiten Fläche angeordnet. Die Oberfläche des Mikroarrays liegt in der ersten Position im Wesentlichen bündig an der zweiten Fläche an, d.h. die Substratoberfläche mit den darauf immobilisierten Sondenmoleküle ist im Wesentlichen nicht von der Probenlösung benetzt. Zwischen dem zweiten Schenkelabschnitt der ersten Fläche und der zweiten Fläche ist in dieser ersten Position ein Raum gebildet, der im Folgenden auch als Prozessierungskammer bezeichnet ist. Diese Prozessierungskammer kann als Kammer für die Prozessierung der Probenlösung dienen.

In einer zweiten Position der schwenkbaren ersten Fläche ist die erste Fläche in einem Winkel verschieden von 180° zu der zweiten Fläche angeordnet. Die Oberfläche des Mikroarrays liegt bei dieser zweiten Position nicht an der zweiten Fläche an, d.h. die auf dem Substrat des Mikroarrays immobilisierten Sondenmoleküle sind für die in der Probenlösung vorliegenden Targetmoleküle frei zugänglich und können so mit diesen wechselwirken. Die Prozessierungskammer ist in der zweiten Position komprimiert.

Die schwenkbare erste Fläche ist vorzugsweise durch Zug auf den ersten Schenkelabschnitt der ersten Fläche und/oder durch Druck auf den zweiten Schenkelabschnitt der ersten Fläche schwenkbar. Der Zug und/oder Druck kann durch ein wie vorstehend beschriebenes Mittel zur Führung der ersten Fläche ausgeübt werden.

Der Chip bzw. das Substrat bzw. die erste Fläche kann vorzugsweise aus Silizium, keramische Materialien wie Aluminiumoxid-Keramiken, Borofloat-Gläsern, Quarzglas, einkristallinem $CaF_2$, Saphir-Scheiben, Topas, PMMA, Polycarbonat und/oder Polystyrol bestehen. Die Wahl der Materialien ist ebenfalls am späteren Verwendungszweck der Vorrichtung bzw. des Chips auszurichten. Wird der Chip zum Beispiel für die Charakterisierung von PCR-Produkten verwendet, dürfen nur solche Materialien eingesetzt werden, die einer Temperatur von 95°C standhalten können.

Die Chips sind bevorzugt durch Nukleinsäuremoleküle, insbesondere durch DNA- oder RNA-Moleküle funktionalisiert. Sie können aber auch durch Peptide und/oder Proteine, wie zum Beispiel Antikörper, Rezeptormoleküle, pharmazeutisch aktive Peptide und/oder Hormone, Kohlenhydrate und/oder Mischpolymere dieser Biopolymere funktionalisiert sein.

Bei einer weiteren bevorzugten Ausführungsform sind die molekularen Sonden über einen Polymer-Linker, beispielsweise eine modifizierte Silanschicht, auf der Substratoberfläche immobilisiert. Ein derartiger polymerer Linker kann der Derivatisierung der Substratoberfläche und somit der Immobilisierung der molekularen Sonden dienen. Im Falle einer kovalenten Anbindung der Sonden finden Polymere, z.B. Silane, Verwendung, die mit reaktiven Funktionalitäten wie Epoxiden oder Aldehyden funktionalisiert bzw. modifiziert sind. Des Weiteren ist dem Fachmann auch die Aktivierung einer Oberfläche durch Isothiocyanat, Succinimidester und Imidoester bekannt. Hierfür werden häufig aminofunktionalisierte Oberflächen entsprechend derivatisiert. Ferner können durch die Zugabe von Kupplungsreagenzien, wie z.B. Dicyclohexylcarbodiimid, entsprechende Immobilisierungen der molekularen Sonden gewährleistet werden.

Der Kammerkörper der Reaktionskammer besteht vorzugsweise aus Materialien wie Glas, Kunststoff und/oder Metallen wie Edelstahl, Aluminium und Messing. Zu seiner Herstellung können beispielsweise Spritzguss-geeignete Kunststoffe verwendet werden. Unter anderem sind Kunststoffe wie Makrolon, Nylon, PMMA und Teflon denkbar. Bei speziellen Ausgestaltungen sind elektrisch leitfähige Kunststoffe wie Polyamid mit 5-30% Kohlefasern, Polycarbonat mit 5-30% Kohlefasern, Polyamid mit 2-20% rostfreien Stahlfasern und PPS mit 5-40% Kohlenstofffaser und insbesondere 20-30% Kohlenstofffaser bevorzugt. Alternativ und/oder zusätzlich kann der Reaktionsraum zwischen erster und zweiter Fläche aber auch durch Septen abgeschlossen sein, die beispielsweise ein Befüllen des Reaktionsraums mittels Spritzen ermöglichen. Bei einer bevorzugten Ausführungsform besteht der Kammerkörper aus optisch durchlässigen Materialien wie Glas, PMMA, Polycarbonat, Polystyrol und/oder Topas. Dabei ist die Wahl der Materialien dem Verwendungszweck der Vorrichtung anzupassen. Zum Beispiel sind die Temperaturen, denen die Vorrichtung ausgesetzt sein wird, bei der Wahl der Materialien zu beachten. Soll die Vorrichtung zum Beispiel für die Durchführung einer PCR verwendet werden, dürfen z.B. nur solche Kunststoffe eingesetzt werden, die über längere Zeiträume bei Temperaturen wie 95°C stabil sind.

Der Kammerkörper ist insbesondere so ausgestaltet, dass der Mikroarray mit seiner aktiven Seite, d.h. der Seite des Arrays, auf der die Nukleinsäuresonden immobilisiert sind, bündig und/oder reversibel an die zweite Fläche gedrückt werden kann.

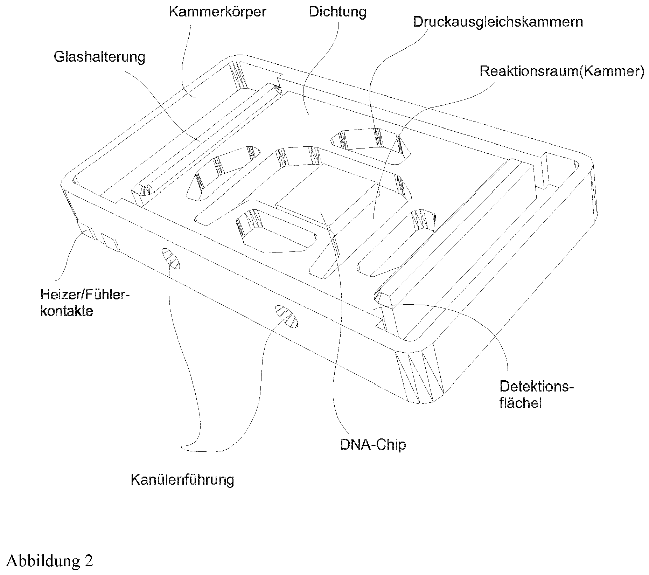

Bei einer speziellen Ausführungsform umfasst die erfindungsgemäße Vorrichtung Module ausgewählt aus der Gruppe bestehend aus einem Kammerkörper, vorzugsweise aus Kunststoff; einem die Reaktionskammer abdichtenden Septum bzw. einer Dichtung; einem DNA-Chip und/oder einer zweiten optisch transparenten Fläche, vorzugsweise einer Glasplatte, wobei die zweite Fläche ggf. auch gleichzeitig als Chip dienen kann (siehe Abbildung 2 und Abbildung 3). Kammerkörper und Dichtung sind bei dieser Ausführungsform elastisch ausgeführt, so dass der DNA-Chip bündig und reversibel mit seiner aktiven Seite an den Glasdeckel gedrückt werden kann. Dadurch wird die zwischen DNA-Chip und Detektionsfläche befindliche markierte Analyseflüssigkeit komplett verdrängt (siehe Abbildung 5 und Abbildung 6). Auf diese Weise kann eine hochempfindliche Fluoreszenzdetektion, z.B. eine computerbildgebende Fluoreszenzmikroskopie durchgeführt werden, die nicht durch eine Hintergrundfluoreszenz der Probenlösung beeinträchtigt wird.

Die zweite Fläche des Kammerkörpers besteht vorzugsweise aus transparenten Materialien wie Glas und/oder optisch durchlässigen Kunststoffen, z.B. PMMA, Polycarbonat, Polystyrol oder Acryl.

Vorzugsweise ist die Reaktionskammer als Kapillarspalt mit variabler Dicke zwischen der zweiten Fläche und dem Mikroarray ausgestaltet. Durch die Ausbildung eines Kapillarspalts zwischen Chip und Detektionsebene können Kapillarkräfte zur sicheren Befüllung der Reaktionkammer genutzt werden. Diese Kapillarkräfte liegen bereits im nicht komprimierten Zustand der Reaktionskammer vor, können jedoch durch Zusammendrücken der Reaktionskammer erhöht werden. Besonders bevorzugt weist der Kapillarspalt eine Dicke im Bereich von etwa 0 $\mu$m bis etwa 100 $\mu$m auf.

Durch die Möglichkeit, den Reaktionsraum komprimieren zu können und damit die Spaltbreite zwischen Mikroarray und Detetektionsebene verringern zu können, ergeben sich weitere Möglichkeiten für die Handhabung der Flüssigkeit innerhalb der Reaktionskammer. So sind bei einer weiteren Ausführungsform der vorliegenden Erfindung anstelle einer einzigen Kammer mehrere Unterkammern vorgesehen, wobei die Abtrennungen zwischen den Unterkammern nicht bis zur zweiten Fläche hochgezogen sind, so dass zwischen den Unterkammern im nicht komprimierten Zustand der Reaktionskammer eine fluidische Verbindung besteht. Durch Komprimierung der Reaktionskammer können die Kammern voneinander abgetrennt werden. Damit lassen sich die Zwischenwände zwischen den Kammern durch Zusammenpressen wie Ventile handhaben.

Eine spezielle Ausgestaltung dieser durch Ventile getrennten Unterkammern ist die Unterteilung des Reaktionsraums der erfindungsgemäßen Vorrichtung in verschiedene PCR-Kammern. In jede Kammer werden individuelle Primer vorgelegt. Die Unterkammern werden zu Beginn mit dem Analyten gleichzeitig befüllt. Anschließend wird der Reaktionsraum komprimiert. Danach durchläuft der Reaktionsraum den Temperaturzyklus für die PCR. Da jede Unterkammer mit unterschiedlichen Primern befüllt ist, findet in jeder Kammer eine andere Amplifikationsreaktion statt. Ein Austausch zwischen den Kammern findet nicht statt.

Nachdem die PCR durchgeführt wurde, findet die Hybridisierung statt. Dabei kann jede Unterkammer einen individuellen Chipbereich oder einen individuellen Chip beinhalten. Es ist aber auch möglich, durch Vergrößerung des Abstands zwischen Mikroarray und zweiter Fläche eine fluidische Verbindung zwischen den Unterkammern zu ermöglichen, so dass sich die verschiedenen Amplifikate miteinander vermischen und auf diese Weise an einer Chipoberfläche hybridisieren.

Der Vorteil dieser Ausführungsform mit durch Ventile getrennten Unterkammern besteht in der Erhöhung der Multiplexität der PCR, also der Anzahl von einander unabhängigen PCR's mit einer Probe, die bei einer Eintopfreaktion aus biochemischen Gründen limitiert ist. So ist es möglich, die Zahl der PCR's der möglichen Anzahl an Sonden auf der Chipoberfläche anzupassen.

In einer weiteren Ausführungsform der vorliegenden Erfindung umfasst die Reaktionskammer somit mindestens zwei Unterkammern, wobei in einem ersten nicht komprimierten Zustand die Unterkammern fluidisch miteinander verbunden sind und in einem zweiten komprimierten Zustand keine fluidische Verbindung zwischen den Unterkammern besteht.

Besonders bevorzugt ist jede Unterkammer einem definierten Bereich des Mikroarrays zugeordnet.

Die Unterkammern können insbesondere dadurch gebildet sein, indem der Mikroarray und/oder die zweite Fläche mit Kavitäten versehen sind, die als Wände zwischen den Unterkammern dienen.

Besonders bevorzugt sind die Wände zwischen den Unterkammern durch elastische Dichtungen gebildet.

Selbstverständlich kann diese Ausgestaltung der Prozesseinheit mit durch Ventile getrennten Unterkammern mit sämtlichen vorstehend beschriebenen Kompressionsprinzipen beliebig kombiniert werden.

Bei einer weiteren Ausführungsform der erfindungsgemäßen Vorrichtung ist die erste Fläche aus einem teilweise deformierbaren elastischen Material gebildet, z.B. aus einer elastischen Membran. Indem nur ein Teil des Reaktionsraums zusammengedrückt werden kann, können unter anderem Unterkammern, in denen der Chip in Richtung der zweiten Fläche geführt wird, Unterkammern, die sich voneinander trennen lassen, sowie Unterkammern, die sich nicht verändern können, erzeugt werden. Dadurch lassen sich im Reaktionsraum einfache Pumpensysteme realisieren, die z.B. dafür verwendet werden können, am Ende einer Vervielfältigungsreaktion Salze in die Hybridisierungskammer zu pumpen. Dies kann vorteilhaft sein, um z.B. die chemischen Hybridisierungsbedingungen des PCR-Puffers zu optimieren, wobei der PCR-Puffer nur für die Durchführung der PCR optimiert ist.

Bei Unterteilung der Reaktionskammer in mehrere Unterkammern ist es bevorzugt, mehrere Mittel zur Agitierung einzusetzen. Üblicherweise sind die Mittel zur Agitierung identisch mit den Mitteln zur Führung der ersten Fläche. Dadurch lassen sich einzelne Kammern gezielt agitieren. Dies kann z.B. sinnvoll sein, um getrennte Vervielfältigungsräume und/oder Hybridisierungsräume zu realisieren.

Selbstverständlich kann auch diese Ausführungsform der erfindungsgemäßen Vorrichtung mit mehreren Agitationsmitteln mit sämtlichen vorstehend beschriebenen Kompressionsprinzipen beliebig kombiniert werden.

Die vorstehend beschriebenen Bauteile bzw. Module der erfindungsgemäßen Vorrichtung ausgewählt aus der Gruppe bestehend aus einem Kammerkörper, den Reaktionsraum seitlich begrenzenden Dichtungen, Mikroarray und Detektionsebene, bilden die sog. Prozesseinheit der erfindungsgemäßen Vorrichtung. In der Prozesseinheit sind PCR, Hybridisierungsreaktionen, Detektion und/oder Auswertung durchführbar.

Die Prozesseinheit der erfindungsgemäßen Vorrichtung ist vorzugsweise modular aufgebaut. Das heißt, die Prozesseinheit kann eine beliebige Kombination der Module umfassen. Die Module können auch während der Analyse ausgetauscht werden.

Bei einer weiteren bevorzugten Ausführungsform umfasst die erfindungsgemäße Vorrichtung zusätzlich eine Temperatursteuerungs- und/oder -regeleinheit zur Steuerung und/oder Regelung der Temperatur in der Reaktionskammer. Eine derartige Temperatursteuerungs- und/oder -regeleinheit zur Steuerung und/oder Regelung der Temperatur in der Reaktionskammer umfasst insbesondere Heiz- und/oder Kühlelemente bzw. Temperaturblöcke. Die Heiz- und/oder Kühlelemente bzw. die Temperaturblöcke können dabei so angeordnet sein, dass sie die erste Fläche und/oder die zweite Fläche kontaktieren. Durch einen Kontaktierung sowohl der ersten als auch der zweiten Fläche wird eine besonders effektive Temperatursteuerung und- regelung gewährleistet.

Bei dieser Ausführungsform ist das Substrat des Mikroarrays bzw. die erste Fläche und/oder die zweite Fläche mit Heiz- und/oder Kühlelementen und/oder Temperaturblöcken verbunden und sollte dann bevorzugt aus Materialien bestehen, die gut wärmeleitend sind. Derartige wärmeleitfähige Materialien bieten den wesentlichen Vorteil, dass sie ein homogenes Temperaturprofil über die gesamte Fläche des Reaktionsraums gewährleisten und somit temperaturabhängige Reaktionen wie beispielsweise eine PCR in der gesamten Reaktionskammer homogen, mit hoher Ausbeute und mit hoher Genauigkeit steuerbar bzw. regelbar durchführbar sind.

Somit bestehen das Substrat des Mikroarrays bzw. die erste Fläche bzw. die zweite Fläche bei einer bevorzugten Ausführungsform aus Materialien mit einer hohen Wärmeleitfähigkeit, vorzugsweise mit einer Wärmeleitfähigkeit im Bereich von 15 bis 500 $Wm^{-1}K^{-1}$, besonders bevorzugt im Bereich von 50 bis 300 $Wm^{-1}K^{-1}$ und am meisten bevorzugt im Bereich von 100 bis 200 $Wm^{-1}K^{-1}$, wobei die Materialien üblicherweise nicht optisch transparent sind. Beispiele für geeignete wärmeleitfähige Materialien sind Silizium, keramische Materialien wie Aluminiumoxid-Keramiken und/oder Metalle wie Edelstahl, Aluminium, Kupfer oder Messing.

Besteht das Substrat des Mikroarrays bzw. die erste Fläche bzw. die zweite Fläche der erfindungsgemäßen Vorrichtung im Wesentlichen aus keramischen Materialien, so werden vorzugsweise Aluminiumoxid-Keramiken eingesetzt. Beispiele für derartige Aluminiumoxid-Keramiken sind die Keramiken A-473, A-476 sowie A-493 der Firma Kyocera (Neuss, Deutschland).

Vorzugsweise ist das Substrat des Mikroarrays bzw. die erste Fläche bzw. die zweite Fläche auf der Rückseite, d.h. der der Reaktionskammer abgewandten Seite mit ggf. miniaturisierten Temperaturfühlern und/oder Elektroden versehen bzw. weist dort Heizerstrukturen auf, so dass ein Temperieren der Probenflüssigkeit sowie eine Durchmischung der Probenflüssigkeit durch einen induzierten elektroosmotischen Fluss möglich ist.

Die Temperaturfühler können beispielsweise als Nickel-Chrom-Dünnfilm-Widerstandstemperaturfühler ausgeführt sein.

Die Elektroden können beispielsweise als Gold-Titan-Elektroden und insbesondere als Quadrupol ausgeführt sein.

Die Heiz- und/oder Kühlelemente können vorzugsweise so gewählt werden, dass ein schnelles Erhitzen und Abkühlen der Flüssigkeit in der Reaktionskammer möglich ist. Unter schnellem Erhitzen und Abkühlen wird dabei verstanden, dass durch die Heiz- und/oder Kühlelemente Temperaturänderungen in einem Bereich von 0,2 K/s bis 30 K/s, vorzugsweise von 0,5 K/s bis 15 K/s, besonders bevorzugt von 2 K/s bis 15 K/s und am meisten bevorzugt von 8 K/s bis 12 K/s oder etwa 10 K/s vermittelt werden können. Vorzugsweise können durch die Heiz- und/oder Kühlelemente auch Temperaturänderungen von 1 K/s bis 10 K/s vermittelt werden.

Die Heiz- und/oder Kühlelemente, z.B. Widerstandsheizer, können beispielsweise als Nickel-Chrom-Dünnfilm-Widerstandsheizer ausgeführt sein.

Für weitere Einzelheiten über die Spezifikation und Dimension der Temperaturfühler, Heiz- und/oder Kühlelemente bzw. Mittel zur Temperaturbeaufschlagung und der Elektroden wird auf den Inhalt der internationalen Patentanmeldung WO 01/02094 verwiesen.

Bei einer bevorzugten Ausführungsform wird die Temperierung der Reaktionskammer dadurch gewährleistet, dass ein Kammerkörper aus elektrisch leitfähigem Material eingesetzt wird. Ein derartig elektrisch leitfähiges Material ist vorzugsweise ein elektrisch leitfähiger Kunststoff, wie z.B. Polyamid, ggf. mit 5-30% Kohlefasern, Polycarbonat, ggf. mit 5-30% Kohlefasern und/oder Polyamid, ggf. mit 2-20% rostfreien Stahlfasern. Vorzugsweise wird als elektrisch leitfähiger Kunststoff PPS (Polyphenylensulfid) mit 5-40% Kohlenstofffaser, besonders bevorzugt 20-30% Kohlenstofffaser eingesetzt. Ferner ist es bevorzugt, dass der Kammerkörper so ausgestaltetet ist, dass er Verdickungen bzw. Verjüngungen aufweist. Derartige Verdickungen bzw. Verjüngungen im Kammerkörper ermöglichen eine gezielte Beheizung der Reaktionskammer bzw. der entsprechenden Flächen. Die Verwendung derartiger Volumenleiter hat ferner den Vorteil, dass auch bei ggf. geringerer Wärmeleitfähigkeit des eingesetzten Materials eine homogene Temperierung der Kammer bzw. der entsprechenden Flächen gewährleistet ist, da in jedem Volumenelement Wärme freigesetzt wird.

Die Einkopplung und Abführung der Wärme in den Reaktionsraum kann auf unterschiedliche Arten erfolgen. Es ist unter anderem vorgesehen, die Wärme über externe Mikrowellenstrahlung, interne oder externe Widerstandsheizung, interne Induktionsschleifen oder -flächen, durch Wasserkühlung und -heizung, durch Reibung, durch Bestrahlung mit Licht, insbesondere IR-Licht, durch Luftkühlung und/oder -heizung, durch Reibung, durch Temperaturstrahler sowie durch Peltierelemente einzubringen.

Die Temperaturmessung im Reaktionsraum kann auf unterschiedlichen Arten erfolgen, beispielsweise durch integrierte Widerstandssensoren, Halbleitersensoren, Lichtwellenleitersensoren, pyrochrome Farbstoffe, pyrochrome Flüssigkristalle, externe Pyrometer wie IR-Strahlung und/oder im Mittel zur Führung des Mikroarrays integrierte Temperatursensoren aller Art.

Die Messung der Temperatur in der Reaktionskammer kann ferner vorgenommen werden durch Integration eines Temperatursensors in den Kammerkörper, z.B. durch Einspritzen im Laufe des Herstellungsprozesses der Kammerkörpers, durch berührungsfreie Messung mit Hilfe eines Pyrometers, eines IR-Sensors und/oder Thermopiles, durch berührende Messung, z.B. durch einen in der Vorrichtung integrierten und eine geeignete Fläche oder ein geeignetes Volumen des Kammerkörpers oder der Kammer kontaktierenden Temperatursensor, durch Messung der temperaturabhängigen Änderung des Brechungsindex an der Detektionsfläche, durch Messung der temperaturabhängigen Änderung der Farbe von speziellen Molekülen z.B. in der Lösung, auf dem Sondenarray bzw. in der Kammerdichtung und/oder durch Messung der temperaturabhängigen Änderung des pH-Wertes der verwendeten Lösung durch Messung der Farbänderung eines pHsensitiven Indikators bspw. durch Messung von dessen Absorption

Außerdem kann eine selbsttätige Begrenzung der Temperatur durch einen sprunghaften Anstieg des Widerstandes des Heizers erfolgen, wobei die entsprechende Sprungtemperatur vorzugsweise in einem Bereich von 95°C bis 110°C liegt. Bei der Sprungtemperatur ändert sich der Widerstand des Heizers sprungartig nach oben, wodurch nahezu kein Strom mehr fliest und demzufolge kaum mehr Wärme frei wird. Als Material für derartige Heizer können insbesondere Polymere wie elektrisch leitfähige Polyamide eingesetzt werden, deren Widerstand sich bei der Sprungtemperatur aufgrund der Veränderung der Matrix des Polymers oder einer Phasenänderung erhöht.

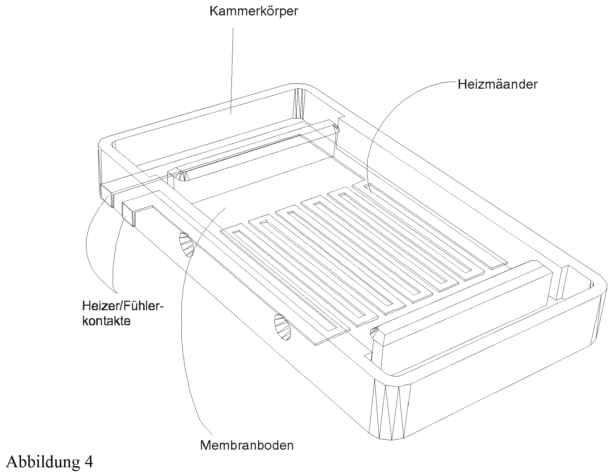

Die Temperatursteuerungs- und -regeleinheit kann bei einer Ausgestaltung in die erste Fläche und/oder zweite Fläche integriert sein. Bei dieser Ausführungsform ist die Prozesseinheit insbesondere mit einem Heizer ausgestattet (siehe Abbildung 4), der zur Realisierung der Temperaturwechsel bei PCR und Hybridisierung dient.

Die Prozesseinheit weist vorzugsweise eine geringe Wärmekapazität auf, so dass bei geringem Energiebedarf maximale Temperaturwechselgeschwindigkeiten von z.B. mindestens 5 K/s realisierbar sind. Um eine schnelle Abkühlung der Prozesseinheit zu gewährleisten, ist bei einer weiteren bevorzugten Ausführungsform die Bereitstellung einer Kühlung, z.B. einer Luftkühlung, vorgesehen.

Die Abkühlung der Prozesseinheit kann bevorzugt auch dadurch erreicht werden, dass der die Prozesseinheit umgebende Raum permanent auf einer erniedrigten Temperatur temperiert und die Kartusche dadurch passiv gekühlt wird. Dadurch wird eine aktive Kühlung der Reaktionskartusche unnötig.

Bei einer weiteren Ausgestaltung kann die Temperatursteuerungs- und -regeleinheit Temperaturblöcke umfassen, die jeweils auf eine definierte Temperatur vorgeheizt sind. Insbesondere weist die Prozesseinheit bei dieser Ausführungsform keinen integrierten Heizer auf. Durch den Wegfall einer integrierten Heizeinrichtung kann die Bereitstellung der Prozesseinheit noch kostengünstiger durchgeführt werden.

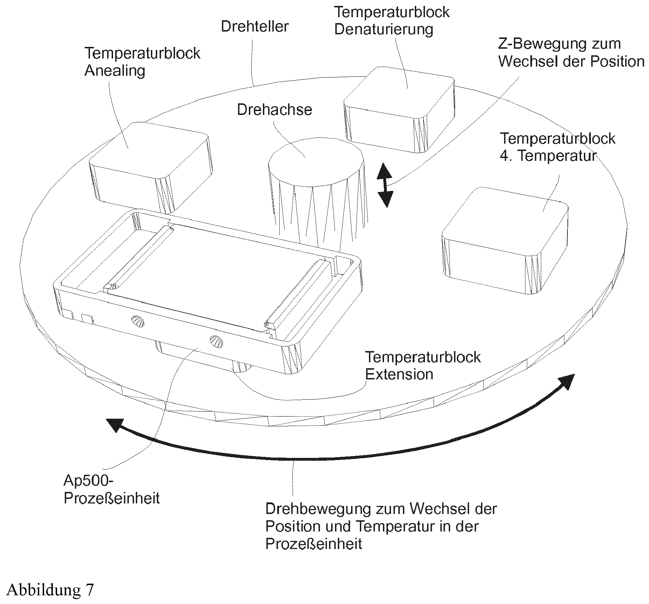

Die Wärmeübertragung zwischen den Temperaturblöcken der Temperatursteuerungs- und -regeleinheit wird vorzugsweise dadurch gewährleistet, dass die Temperaturblöcke die erste Fläche und/oder zweite Fläche der erfindungsgemäßen Vorrichtung kontaktieren. Die Temperaturblöcke können vorzugsweise linear oder auf einem Drehteller angeordnet sein und so beispielsweise in der Detektionsvorrichtung integriert sein. Abbildung 7 zeigt einen Drehteller, der mehrere Temperaturblöcke aufweist, die jeweils auf eine definierte Temperatur eingestellt sind. Durch Wechsel der Temperaturblöcke unter der Prozesseinheit wird die Prozesseinheit auf eine jeweilige durch den Temperaturblock definierte Temperatur gebracht. Die Temperaturblöcke sind vorzugsweise so gefertigt, dass sie eine deutlich höhere Wärmekapazität als die Prozesseinheit aufweisen, so dass auch bei dieser Ausführungsform maximale Temperaturwechselgeschwindigkeiten von z.B. mindestens 5 K/s realisierbar sind. Vorzugsweise werden die Temperaturblöcke lediglich thermostatisiert und nicht geheizt oder gekühlt, so dass auch hier der Energiebedarf minimal ist. Auf eine Kühlung der Prozesseinheit kann bei dieser Ausführungsform verzichtet werden.

Bei einer weiteren Ausgestaltung ist die Temperatursteuerungs- und regeleinheit in das bzw. die Mittel zur Führung der ersten Fläche und/oder das bzw. die Mittel zur Agitierung und/oder den Abstandshalter integriert. Die Wärmeübertragung erfolgt bei dieser Ausgestaltung durch Kontaktierung des Mittels und/oder des Abstandshalters mit der ersten Fläche und/oder der zweiten Fläche.

Vorzugsweise umfasst die Vorrichtung zusätzlich eine Aufarbeitungseinheit zur Reinigung und/oder Aufkonzentration der Probenlösung und/oder Steuerung des Be- und/oder Entladens der Reaktionskammer mit Fluiden. Unter Fluiden werden im Rahmen der vorliegenden Erfindung Flüssigkeiten oder Gase verstanden. Darüber hinaus kann die Analysenlösung in der Aufarbeitungseinheit umgepuffert werden. Die Aufarbeitungseinheit kann schließlich auch zur Bereitstellung mit den notwendigen Analysenchemikalien genutzt werden. Die Verbindung der Fluidbehälter mit der Reaktionskammer kann beispielsweise wie in der internationalen Patentanmeldung WO 01/02094 ausgeführt sein.

Besonders bevorzugt sind bei dieser Ausführungsform die Reaktionskammer und die Aufarbeitungseinheit über zwei Kanülen miteinander verbunden, wobei die Kanülen so angeordnet sind, dass eine erste Kanüle die Zuführung von Fluiden aus der Aufarbeitungseinheit in die Reaktionskammer gewährleistet und eine zweite Kanüle das Entweichen der durch die zugeführten Fluide aus der Reaktionskammer verdrängten Luft gewährleistet. Eine in die Aufarbeitungseinheit gegebene Probe kann so über die Kanülen in die Reaktionskammer der Prozesseinheit gelangen. Zu diesem Zweck sind die Kanülen derart angeordnet, dass sie durch die Kanülenführung in die Reaktionskammer reichen.

Die Aufarbeitungseinheit kann so ausgestaltet sein, dass sie von der Prozesseinheit abtrennbar ist. Nach Befüllung der Reaktionskammer mit der Probenlösung und ggf. weiteren Reaktionsflüssigkeiten kann so die Aufarbeitungseinheit von der Prozesseinheit getrennt, vorzugsweise abgezogen, und ggf. entsorgt werden.

Im Folgenden werden Ausführungsformen von integrierten oder nicht integrierten Einheiten zur Befüllung der Reaktionskammer beschrieben, die im Folgenden auch als Befülleinheit oder Aufarbeitungseinheit bezeichnet wird.

Üblicherweise wird die Reaktionslösung mit einem geeigneten Werkzeug, beispielsweise mit einer Pipette in eine bestimmte Öffnung der Befülleinheit eingebracht. Die Beförderung der Flüssigkeiten in die Vorrichtung erfolgt über den Druck der Pipette, oder durch ein weiteres druckerzeugendes Werkzeug wie z.B. eine Spritze oder einer automatisierten Einheit, die beispielsweise funktionaler Bestandteil eines Prozessierungsautomaten ist.

Die Befülleinheit ist vorzugsweise in ergonomisch sinnvoller Weise für die manuelle Bedienbarkeit ausgestaltet. Ferner weist sie vorzugsweise leicht zugängliche Öffnungen an den Außenseiten zur Einbringung der reaktiven Substanzen auf.

Eine Befülleinheit umfasst ferner vorzugsweise ein geeignetes fluidisches Interface zum Durchdringen der Dichtung des Kammerkörpers. Hierzu werden insbesondere Kanülen verwendet, die beispielsweise aus Edelstahl oder Polymeren sind und üblicherweise Durchmesser von 0.05 mm bis 2 mm aufweisen. Vorzugsweise sind mindestens eine oder mehrere Kanülen angeordnet, besonders bevorzugt zwei, wobei eine zum Befüllen mit einer reaktiven Flüssigkeit und eine andere zum Entlüften des Reaktionsraumes und zur Aufnahme von Überschüssigen Flüssigkeiten Verwendung finden kann. Derartige Kanülen können fest oder austauschbar mit der Befülleinheit verbunden sein, wobei vorzugsweise eine vom Anwender nichtlösbare Verbindung zur Realisierung von Befüll-Einwegartikeln realisiert ist.

Die Befülleinheit kann ferner eine Einheit zur Abdeckung der Kanülen umfassen, so dass nach der Trennung der Systeme verhindert werden kann, dass sich der Anwender an den Kanülen verletzt oder die Umgebung kontaminiert wird.

Die Befülleinheit umfasst ferner vorzugsweise ein geeignetes mechanisches Interface zur passgenauen Kontaktierung der Reaktionskartusche. Dieses Interface kann z.B. durch spezielle Schnappverschlüsse ausgeführt sein. Auf diese Art und Weise kann eine Durchdringung der Dichtung der Kammerkörpers an bevorzugten Stellen gewährleistet werden.

Bei der Prozessierung der Reaktionskartusche in entsprechenden Prozessierungsautomaten sind geeignete mechanische Vorkehrungen zu treffen, die eine Justage und lagegenaue Positionierung in den Geräten zulassen. Dieses bezieht sich insbesondere auf die Positionierung für den Austausch und/oder die Zufuhr von Flüssigkeiten und die Positionierung der Reaktionskartusche zur Detektion der Signale nach der Durchführung der Reaktionen in der Reaktionskammer.

Die Vorrichtung bzw. die Befülleinheit kann ferner einen integrierten Abfallbehälter umfassen, der zur Aufnahme überschüssiger oder verdrängter gasförmiger oder flüssiger Medien wie z.B. Schutzgasfüllungen oder Puffer dient. Der Abfallbehälter kann beispielsweise mit einem weiteren gasförmigen, flüssigem oder festem Medium gefüllt sein, welches die flüssigen oder gasförmigen Substanzen reversibel oder irreversibel bindet, wie z.B. Zellulose, Filtermaterialien, Silicagele. Des Weiteren kann der Abfallbehälter über eine Entlüftungsöffnung verfügen oder zur Verbesserung des Befüllverhaltens der Gesamteinheit mit einem Unterdruck ausgestattet sein.

Der Abfallbehälter kann alternativ auch als getrenntes Modul ausgeführt sein. In diesem Falle ist die Befülleinheit mit entsprechenden nach außen führenden fluidischen Interfaces, die kommerziellen Standards wie z.B. LuerLock entsprechen können, ausgestattet. Solche Interfaces können einen Form- oder Kraftschluß zu weiterführenden Systemen aufweisen.

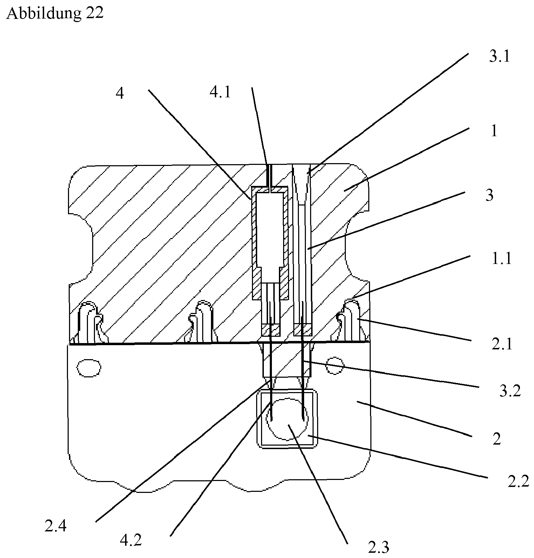

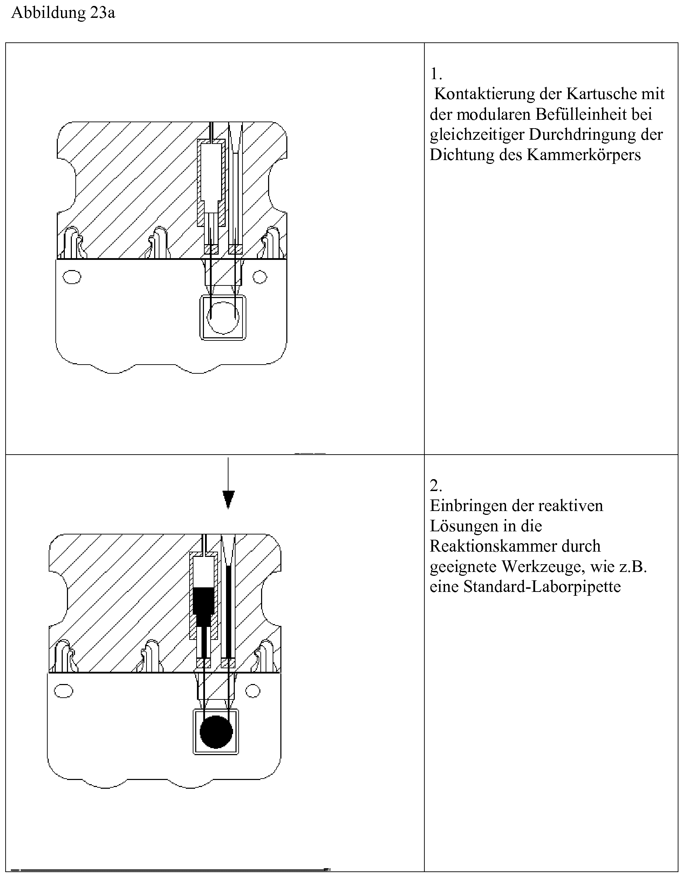

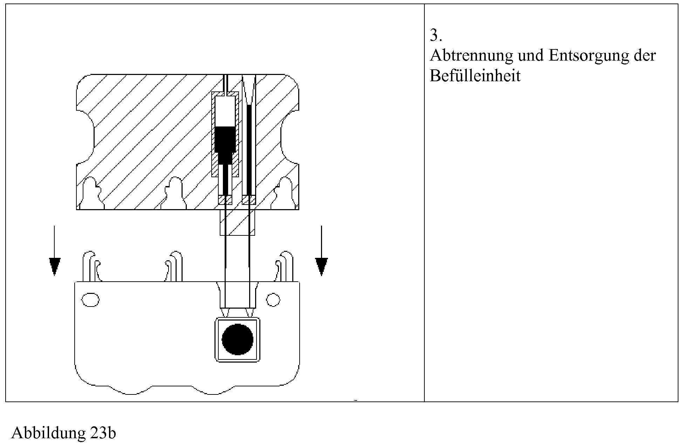

Bei einer ersten speziellen Ausführungsform erfolgt die Befüllung mit einer abnehmbaren Befülleinheit mit integriertem Abfallbehälter. Die Befülleinheit dient insbesondere zur einmaligen Befüllung der Reaktionskammer. Die Befülleinheit ist beispielsweise so ausgeführt, dass diese an die Kartusche gesteckt oder vorübergehend befestigt wird, die Proben in den Reaktionsraum eingebracht werden, und nach erfolgter Befüllung die Befülleinheit wieder der Kartusche getrennt und entsorgt wird. Bei dieser speziellen ersten Ausführungsform umfasst die Befülleinheit ferner einen integrierten Abfallbehälter, der wie vorstehend beschrieben ausgestaltet sein kann. Ein Beispiel für diese Ausführungsform ist in Abbildung 22 gezeigt. Der Ablauf zur Befüllung einer Reaktionskartusche mit einer modularen Befülleinheit ist in Abbildung 23 gezeigt.

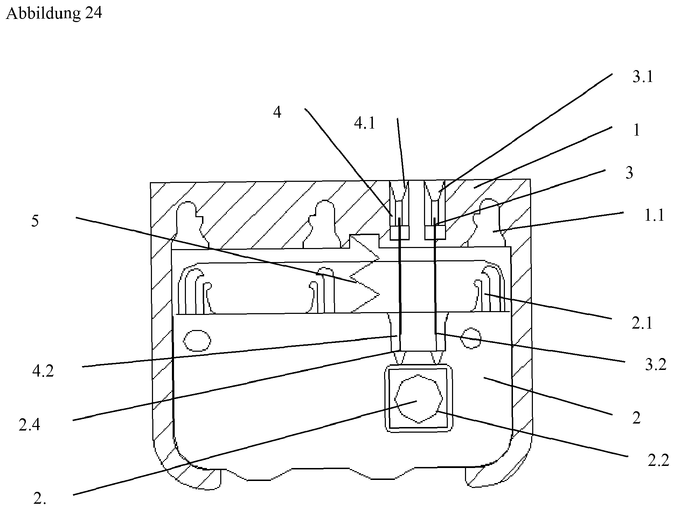

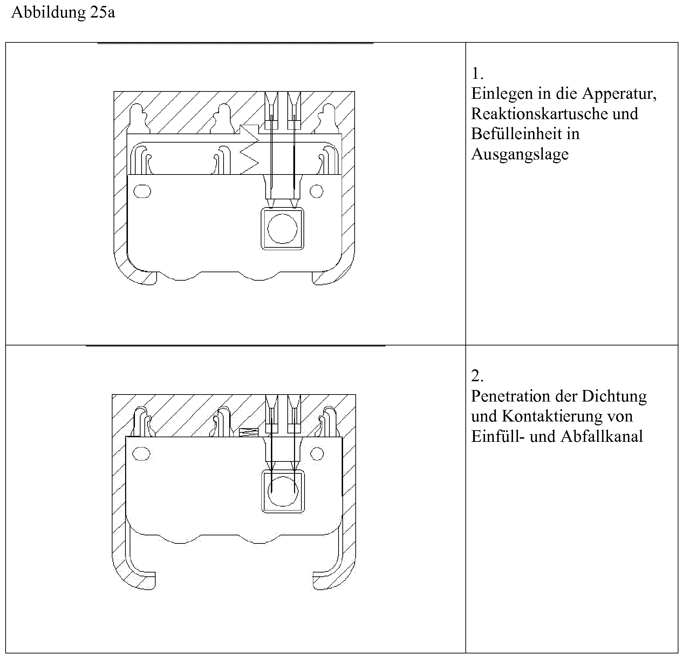

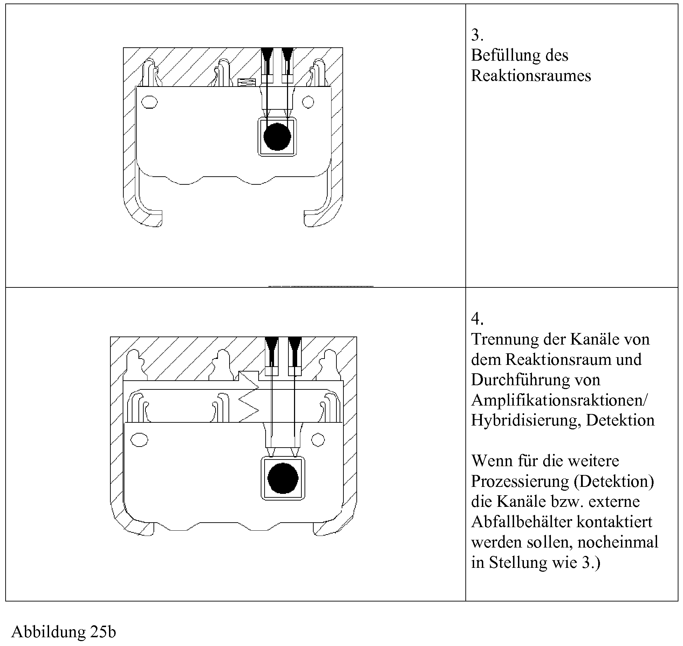

Bei einer zweiten speziellen Ausführungsform erfolgt die Befüllung mit einer integrierten Befülleinheit. Hier ist die Befülleinheit integrativer Bestandteil der Reaktionskartusche und wird demzufolge nicht von dieser getrennt, die Entsorgung der Befülleinheit und der Kartusche erfolgt gleichzeitig. Die Befülleinheit wird dabei vorzugsweise zur einmaligen Befüllung der Reaktionskammer und möglicherweise für weitere prozessinterne Fluidschritte verwendet. Die Befülleinheit umfasst bei dieser Ausführungsform ferner vorzugsweise eine technische Einrichtung, die eine Vorzugsstellung der Kanülen im System realisiert, insbesondere um ein unbeabsichtigtes Einstechen der Kanülen in die Dichtung des Kammerkörpers zu verhindern. Es ist aber auch denkbar, dass in dieser Vorzugsstellung die Kanülen in die Dichtung des Kammerkörpers einstechen. Diese technische Einrichtung kann beispielsweise durch das Einbringen von Federn, elastischen Elementen oder bestimmten Vertiefungen und Erhöhungen zur Realisierung einer Rastung realisiert werden. Die Befülleinheit umfasst bei dieser Ausführungsform ferner einen Befüll- und Abfallkanal, welcher entsprechende nach außen führende fluidische Interfaces umfasst, die auch kommerziellen Standards wie z.B. LuerLock entsprechen können. Solche Interfaces können einen Form- oder Kraftschluß zu weiterführenden Systemen aufweisen und dienen der Zuführung und/oder Abführung gasförmiger und/oder flüssiger Medien. Ein Beispiel für diese Ausführungsform ist in Abbildung 24 gezeigt. Der Ablauf zur Befüllung einer Reaktionskartusche mit einer integrierten Befülleinheit ist in Abbildung 25 gezeigt.

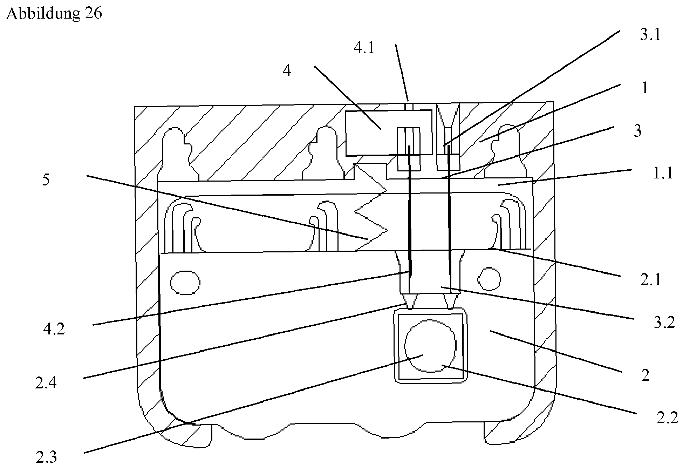

Bei einer dritten speziellen Ausführungsform erfolgt die Befüllung mit einer integrierten Befülleinheit mit integriertem Abfallbehälter. Die Befülleinheit ist bei dieser Ausführungsform integrativer Bestandteil der Reaktionskartusche und wird demzufolge nicht von dieser getrennt, die Entsorgung der Befülleinheit und der Kartusche erfolgt gleichzeitig. Die Befülleinheit wird dabei vorzugsweise zur einmaligen Befüllung der Reaktionskammer und möglicherweise für weitere prozessinterne Fluidschritte verwendet. Die Befülleinheit umfasst auch bei dieser Ausführungsform ferner vorzugsweise eine technische Einrichtung, die eine Vorzugsstellung der Kanülen im System realisiert, vorzugsweise um ein unbeabsichtigtes Einstechen der Kanülen in die Dichtung des Kammerkörpers zu verhindern. Es ist aber auch denkbar, dass in dieser Vorzugsstellung die Kanülen in die Dichtung des Kammerkörpers einstechen. Diese technische Einrichtung kann beispielsweise durch das Einbringen von Federn, elastischen Elementen oder bestimmten Vertiefungen und Erhöhungen zur Realisierung einer Rastung realisiert werden. Die Befülleinheit umfasst bei dieser Ausführungsform ferner einen integrierten Abfallbehälter, der wie vorstehend beschrieben ausgestaltet sein kann. Ein Beispiel für diese Ausführungsform ist in Abbildung 26 gezeigt. Der Ablauf zur Befüllung einer Reaktionskartusche mit einer integrierten Befülleinheit und integriertem Abfallbehälter kann beispielsweise durch Kombination der in den Abbildungen 23 und 25 beschriebenen Vorgehensweisen erfolgen.

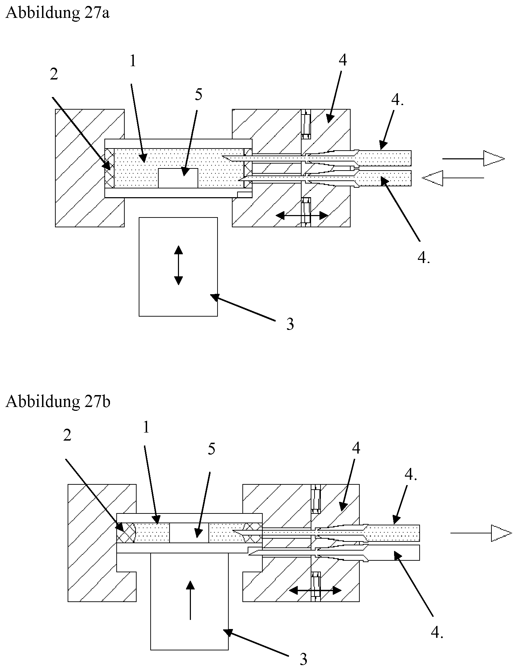

Im Folgenden wird eine spezielle Ausführungsform zur Anordnung von Kanülen zum Druckausgleich während des Quetschvorgangs beschrieben. Die Kanülen eines Befülltools für die Kartusche können beispielsweise derart angeordnet sein, dass sowohl eine Befüllung im entspannten Zustand als auch die Überführung der überschüssigen Reaktionslösungen bei Zusammenquetschen des Reaktionsraumes möglich ist. Erreicht werden kann dies vorzugsweise durch eine angepasste Konstruktion der Dichtung und der Kanülenanordnung, bei der die Kanülen bevorzugt in die Ausgleichsbereiche innerhalb der Reaktionskammer einstechen. Eine solche Anordnung ist insbesondere sinnvoll, wenn das überschüssige Volumen nicht durch eine spezielle Dichtungsgestaltung aufgenommen werden kann. Ein Beispiel für eine mögliche vertikale Kanülenanordnung bei unveränderter Dichtungsform ist in Abbildung 27 gezeigt.

Die erfindungsgemäße Vorrichtung kann ferner eine mit dem Detektionssystem verbundene Einheit zur Steuerung des Testablaufs und/oder zur Verarbeitung von durch das Detektionssystem aufgenommenen Signalen umfassen. Die Steuerungs- und/oder Verarbeitungseinheit kann ein Mikrocontroler oder Industrierechner sein. Diese Kopplung von Detektiereinheit und Verarbeitungseinheit, die die Umwandlung der Reaktionsergebnisse in das Analyseergebnis gewährleistet, erlaubt unter anderem den Einsatz der erfindungsgemäßen Vorrichtung als Handgerät beispielsweise in der medizinischen Diagnostik.

Ferner weist die erfindungsgemäße Vorrichtung vorzugsweise zusätzlich eine Schnittstelle für externe Rechner auf. Dies erlaubt unter anderem die Übertragung von Daten zur Speicherung außerhalb der Vorrichtung.

In einer weiteren bevorzugten Ausführungsform ist die Vorrichtung mit einer Codierung, vorzugsweise einer Datenmatrix und/oder einem Barcode, versehen, die Informationen über die Substanzbibliothek und/oder die Durchführung der Vervielfältigungs- und/oder Nachweisreaktion enthält. Durch eine derartige individuelle Identifikationsnummer kann das Auslese- bzw. Detektionsgerät automatisch erkennen, welcher Test durchgeführt wurde. Dazu wird bei der Herstellung der erfindungsgemäßen Vorrichtung ein Datensatz in einer Datenbank gespeichert, welcher Informationen über die Substanzbibliothek, die Durchführung der Nachweisreaktion und dergleichen enthält. So kann der Datensatz insbesondere Informationen über die Anordnung der Sonden auf dem Array sowie Informationen darüber enthalten, wie die Auswertung am vorteilhaftesten zu erfolgen hat. Der Datensatz bzw. die Datenmatrix kann ferner Informationen über das Temperatur-Zeit-Regime einer ggf. durchzuführenden PCR zur Vervielfältigung der Zielmoleküle enthalten. Der so erstellte Datensatz erhält vorzugsweise eine Nummer, die in Form der Datenmatrix auf der Halterung angebracht wird.

Über die in der Datenmatrix verzeichnete Nummer kann dann ggf. beim Auslesen der Substanzbibliothek der angelegte Datensatz aufgerufen werden. Schließlich kann die Datenmatrix von der Temperatursteuerungs- bzw. -regeleinheit und anderen Controllern wie z.B. einer Steuerung für die Be- und Entfüllung der Reaktionskammer über die Fluidbehälter ausgelesen werden und so eine automatische Durchführung von Vervielfältigungs- und Nachweisreaktion gewährleistet werden.

Die Codierung wie eine Datenmatrix muss nicht zwingend die komplette Information beinhalten. Sie kann auch einfach eine Identifikation bzw. Kennung enthalten, mittels der der dann aus einem Rechner oder von einem Datenträger die erforderlichen Daten zugeladen werden.

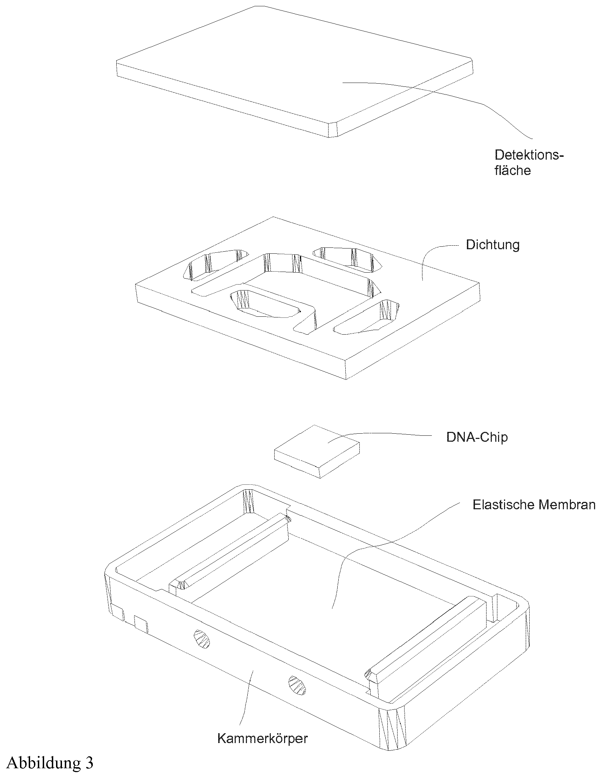

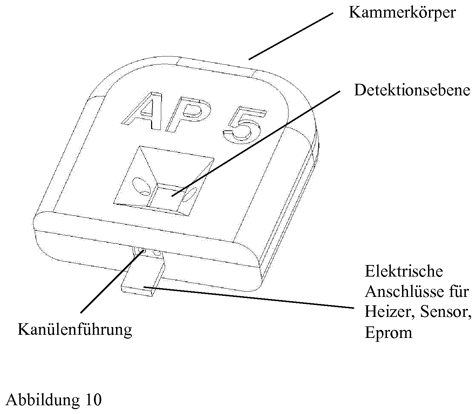

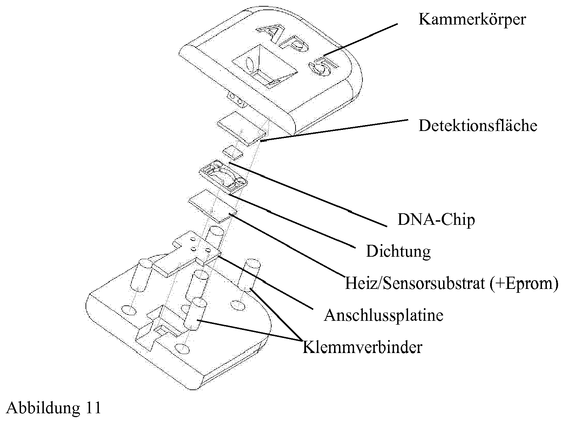

Die erfindungsgemäße Vorrichtung ist äußerst fertigungsfreundlich. In Abbildung 3 ist gezeigt, dass die Prozesseinheit aus nur vier Einzelbauteilen bestehen kann, die auf einfache Weise ineinander gelegt werden. In den Abbildungen 10 und 11 sind Ausführungsformen dargestellt, die aufgrund der erfindungsgemäßen Konstruktion ebenfalls fertigungsfreundlich sind, obwohl sie aus mehreren Teilen bestehen. Die geometrischen Toleranzen der Abmessungen der Einzelbauteile können mit z.B. 1/10 bis 2/10 mm sehr groß ausfallen, so dass z.B. die Spritzgussfertigung von Dichtung und Kammerkörper großtechnisch äußerst kostengünstig durchgeführt werden kann. Die geringen Toleranzen werden durch das Anpressen des Chips an die Detektionsebene ermöglicht, da dadurch der optische Weg zum Detektionsmikroskop kaum durch die Bauteile der Prozesseinheit beeinflusst wird. Die einzigen geometrischen Größen, die eine geringe Toleranz aufweisen, sind die x,y-Lage des Chips und die Dicke der Detektionsebene. Hingegen spielt die Varianz der z-Lage des Chips nur eine untergeordnete Rolle. Trotz dieser geringen technischen Anforderungen ist eine Fokussierungseinrichtung am optischen System, z.B. einem Fluoreszenzdetektionsmikroskop, nicht erforderlich. Diese Eigenschaften verdeutlichen die Eignung der erfindungsgemäßen Vorrichtung für den mobilen Vororteinsatz.

Bei einem weiteren Aspekt der vorliegenden Erfindung wird ein Verfahren zum qualitativen und/oder quantitativen Nachweis von molekularen Wechselwirkungen zwischen Sonden- und Targetmolekülen bereitgestellt, dass die folgenden Schritte umfasst:

a) Einbringen einer Probe, vorzugsweise einer Probenlösung umfassend Targetmoleküle in eine Reaktionskammer einer wie vorstehend beschriebenen erfindungsgemäßen Vorrichtung; und
b) Detektieren einer Wechselwirkung zwischen den Targetmolekülen und den auf dem Substrat immobilisierten Sondenmolekülen.

Das erfindungsgemäße Verfahren ermöglicht den qualitativen und/oder quantitativen Nachweis von molekularen Wechselwirkungen zwischen Sonden- und Targetmolekülen in einer Reaktionskammer, ohne dass nach der erfolgten Wechselwirkung und vor der Detektion ein Austausch der Proben- bzw. Reaktionsflüssigkeiten zur Entfernung eines störenden Hintergrunds erforderlich ist.

Der Nachweis einer Wechselwirkung zwischen der Sonde und dem Targetmolekül erfolgt im Rahmen der vorliegenden Erfindung üblicherweise folgendermaßen: Nach der Fixierung der Sonde bzw. der Sonden in vorgegebener Art und Weise an einer bestimmten Matrix in Form eines Mikroarrays bzw. nach dem Bereitstellen eines Mikroarrays werden die Targets in einer Lösung mit den Sonden in Kontakt gebracht und unter definierten Bedingungen inkubiert. Infolge der Inkubation findet zwischen Sonde und Target eine spezifische Wechselwirkung bzw. Hybridisierung statt. Die dabei auftretende Bindung ist deutlich stabiler als die Bindung von Targetmolekülen an Sonden, die für das Targetmolekül nicht spezifisch sind.

Der Nachweis bzw. die Detektion der spezifischen Wechselwirkung zwischen einem Target und seiner Sonde kann dann durch eine Vielzahl von Methoden erfolgen, die in der Regel von der Art des Markers abhängen, der vor, während oder nach der Wechselwirkung des Targetmoleküls mit dem Mikroarray in Targetmoleküle eingebracht worden ist. Typischerweise handelt es sich bei solchen Markern um fluoreszierende Gruppen, so dass spezifische Target-Sonden-Wechselwirkungen mit hoher Ortsauflösung und im Vergleich zu anderen herkömmlichen Nachweismethoden, vor allem massensensitiven Methoden, mit geringem Aufwand fluoreszenzoptisch ausgelesen werden können (siehe z.B. A. Marshall, J. Hodgson, DNA chips: An array of possibilities, Nature Biotechnology 1998, 16, 27-31; G. Ramsay, DNA Chips: State of the art, Nature Biotechnology 1998, 16, 40-44).

In Abhängigkeit von der auf dem Mikroarray immobilisierten Substanzbibliothek und der chemischen Natur der Targetmoleküle können anhand dieses Testprinzips Wechselwirkungen zwischen Nukleinsäuren und Nukleinsäuren, zwischen Proteinen und Proteinen sowie zwischen Nukleinsäuren und Proteinen untersucht werden (zur Übersicht siehe F. Lottspeich, H. Zorbas, 1998, Bioanalytik, Spektrum Akademischer Verlag, Heidelberg/Berlin).

Als Substanzbibliotheken, die auf Mikroarrays oder Chips immobilisiert werden können, kommen dabei insbesondere Antikörper-Bibliotheken, Rezeptor-Bibliotheken, Peptid-Bibliotheken und Nukleinsäure-Bibliotheken in Frage.

Die Nukleinsäure-Bibliotheken nehmen die mit Abstand wichtigste Rolle ein. Es handelt sich dabei um Mikroarrays, auf denen Desoxyribonukleinsäure- (DNA) Moleküle oder Ribonukleinsäure- (RNA) Moleküle immobilisiert sind.

Bei einer bevorzugten Ausführungsform des erfindungsgemäßen Verfahrens wird der Abstand zwischen Mikroarray und zweiter Fläche vor dem Detektieren in Schritt b) in einer Position gehalten, die das Prozessieren der Probenlösung und/oder die Wechselwirkung zwischen den Targetmolekülen und den auf dem Substrat immobilisierten Sondenmolekülen, beispielsweise das Amplifizieren von nachzuweisenden Nukleinsäuren und/oder die Hybridisierung zwischen nachzuweisenden Nukleinsäuren und den auf dem Substrat immobilisierten Nukleinsäuresonden, ermöglicht.

Ferner ist es bevorzugt, dass in Schritt b) der Abstand zwischen dem Mikroarray und der zweiten Fläche verändert, vorzugsweise verringert wird. D.h. die Detektion wird vorzugsweise bei einem verringerten Abstand zwischen Mikroarray und Detektionsebene durchgeführt. Besonders bevorzugt ist der Abstand zwischen Mikroarray und Detektionsebene bei der Detektion in etwa gleich null.

Bei einer Ausgestaltung wird zur Verringerung des Abstands zwischen Mikroarray und zweiter Fläche der Mikroarray in Richtung der zweiten Fläche geführt. Dies wird vorzugsweise durch Druck mindestens eines Mittels zur Führung der ersten Fläche, beispielsweise eines Stößels, eines Stabs, eines Stifts und/oder einer Schraube, auf die erste Fläche gewährleistet, wobei der Druckpunkt des Mittels insbesondere unterhalb des Mikroarrays liegt.

Das Herandrücken des Mikroarrays an die zweite Fläche bzw. die Detektionsebene kann dadurch ermöglicht sein, dass die erste Fläche zumindest im Bereich unterhalb des Mikroarrays elastisch verformbar ist. Alternativ kann die erste Fläche mittels zweier übereinander liegender Schichten ausgestaltet sein, wobei eine äußere Schicht der beiden übereinander liegenden Schichten zumindest im Bereich unterhalb des Mikroarrays eine Aussparung aufweist und eine innere der beiden übereinander liegenden Schichten aus einer elastischen Dichtung gebildet ist. Der Druck wird dann mit dem Mittel zur Führung der ersten Fläche auf die innere Schicht im Bereich der Aussparung ausgeübt.

Das Mittel zur Führung der ersten Fläche, z.B. ein Stift, ein Stab, ein Stößel und/oder eine Schraube, kann aber nicht nur zur Ausübung eines Drucks auf die erste Fläche dienen. Sollten auf dem DNA-Chip Blasen entstehen, die eine Detektion erschweren würden, so lassen sich diese durch Agitation durch das Mittel zur Führung der ersten Fläche, z.B. mit einer an der ersten Fläche, insbesondere in Form einer elastischen Membran, angelegten Vibrationsfrequenz von etwa 20 Hz, entfernen.

Ferner besteht häufig das Problem, dass die Wechselwirkung, z.B. die Hybridisierung, an der Chipoberfläche sehr lange dauert. Dies liegt unter anderem daran, dass die Wechselwirkungs- bzw. Hybridisierungsgeschwindigkeit diffusionsbestimmt ist. Vorzugsweise lässt sich Wechselwirkungs- bzw. Hybridisierungsgeschwindigkeit durch Agitation über das Mittel zur Führung der ersten Fläche, z.B. mit einer an der ersten Fläche, insbesondere in Form einer elastischen Membran, angelegten Vibrationsfrequenz von etwa 20 Hz, erhöhen, da die Agitation bzw. Vibration zu einer Durchmischung in der Reaktionskammer führt. Bei einer weiteren Ausgestaltung wird zur Verringerung des Abstands zwischen Mikroarray und zweiter Fläche die zweite Fläche in Richtung der ersten Fläche geführt. Dies kann insbesondere dadurch gewährleistet werden, dass die zweite Fläche durch Druck des Abstandshalters auf die zweite Fläche in Richtung der ersten Fläche geführt wird.

Bei einer weiteren Ausgestaltung wird zur Verringerung des Abstands zwischen Mikroarray und zweiter Fläche die erste Fläche in Richtung der zweiten Fläche und die zweite Fläche in Richtung der ersten Fläche geführt.

Im Folgenden werden weitere Ausführungsformen zum Führen der ersten Fläche relativ zur zweiten Fläche bzw. der zweiten Fläche relativ zur ersten Fläche beschrieben. Diese Ausführungsformen sind nicht nur zur Positionierung der ersten Fläche bzw. des Sondenarrays relativ zur zweiten Fläche bzw. der Detektionsfläche geeignet, sondern können insbesondere auch eingesetzt werden, um das Sondenarray relativ zur Detektionsfläche zu bewegen. Durch eine derartige Bewegung kann beispielsweise eine Agitation der Lösung in der Reaktionskammer erreicht werden.

In einer Ausführungsform wird das Sondenarray mit Hilfe eines Magnetfeldes gegen relativ zur Detektionsfläche geführt oder in der Kammer bewegt. Beispielsweise enthält das Sondenarray und/oder die zweite Fläche ein magnetisches Material bzw. enthält eine Komponente, der ein magnetisches Material beigemischt ist und/oder ist in einer Fassung aus einem ganz oder teilweise magnetischen Material eingefasst. Ferner kann es bevorzugt sein, dass das Sondenarray und/oder die zweite Fläche passiv bewegt werden, indem mittels eines magnetischen Feldes ein magnetischer Körper bewegt wird, der unter der jeweiligen Fläche angeordnet ist und beispielsweise mit dieser verbunden ist.

In einer weiteren Ausführungsform wird das Sondenarray durch Einwirken der Schwerkraft relativ zur Detektionsfläche bewegt und/oder positioniert.

In einer weiteren Ausführungsform wird das Sondenarray durch eine in der Reaktionskammer erzeugte Strömung relativ zur Detektionsfläche bewegt und/oder positioniert. Dazu kann die Vorrichtung beispielsweise so ausgestaltet sein, dass, wenn das Sondenarray von einer Flüssigkeit umströmt wird, auf einer Seite der Reaktionskammer ein Unterdruck und auf der gegenüberliegenden Seite ein Überdruck entsteht, wodurch eine Bewegung des Sondenarrays in der Reaktionskammer erfolgt. Eine solche Strömung kann z.B. durch eine Wärmeströmung, welche durch lokale Temperaturunterschiede in der Kammer hervorgerufen wird, realisiert werden.

In einer weiteren Ausführungsform wird das Sondenarray durch Einwirken eines elektrischen Feldes relativ zur Detektionsfläche bewegt und/oder positioniert.

In einer weiteren Ausführungsform wird durch lokale Überhitzung unter dem Sondenarray eine Gasblase erzeugt, welche dazu führt, dass der Chip in der Kammer bewegt bzw. gegen die Detektionsfläche geführt wird.

Durch die Verringerung des Abstands zwischen Mikroarray und zweiter Fläche vor der Detektion wird die Probenlösung aus dem Bereich zwischen Mikroarray und Detektionsebene vorzugsweise im Wesentlichen vollständig entfernt. Dadurch werden die Hintergrundsignale, die durch nicht an die Arrayoberfläche gebundene markierte Moleküle, z.B. durch markierte Primer und/oder nicht an die Arrayoberfläche gebundene markierte Targetnukleinsäuren, verursacht werden, vermindert.

Besonders bevorzugt wird somit bei der Detektion in Schritt b) der Abstand zwischen dem Mikroarray und der zweiten Fläche so verändert, dass die Probenlösung zwischen dem Mikroarray und der zweiten Fläche im Wesentlichen entfernt ist. Der Mikroarray liegt dann im Wesentlichen in der Detektionsebene und ein störender Hintergrund wird nahezu vollständig vermieden.

Bei einer weiteren alternativen Ausführungsform liegt der Mikroarray bereits im ursprünglichen Zustand der Vorrichtung bündig an der die Detektionsebene bildenden zweiten Fläche an und wird nicht erst durch Führen der ersten Fläche in Richtung der zweiten Fläche und/oder Führen der zweiten Fläche in Richtung der ersten Fläche in die Detektionsebene gebracht. Während der Prozessierungsschritte ist der Mikroarray bei dieser Ausgestaltung nicht von der Probenlösung benetzt. Zur Durchführung der Wechselwirkungsreaktion, z.B. einer Hybridisierung, wird die erste Fläche, die vorzugsweise aus einem elastischen Material, z.B. einer elastischen Membran, gebildet ist, von der Detektionsfläche weggeführt. Dadurch wird die Chipoberfläche von der Detektionsfläche entfernt und von der Probenlösung benetzt. Die Wechselwirkung, z.B. eine Hybridisierung, kann stattfinden. Zur Durchführung der Detektion und der weiteren Prozessierung wird die erste Fläche, z.B. in Form einer elastischen Membran, wieder freigelassen, wodurch sie in ihre ursprünglich eingestellte Position zurückschnellt, was durch Druck mit einem Mittel zur Führung der ersten Fläche, z.B. einem Stift, einem Stab, einer Schraube und/oder einem Stößel, beschleunigt werden kann. Dadurch wird der Mikroarray wieder an die Detektionsebene gedrückt und die Detektion kann hintergrundfrei durchgeführt werden.

Bei einer weiteren Ausführungsform des erfindungsgemäßen Verfahrens wird eine wie vorstehend beschriebene erfindungsgemäße Vorrichtung eingesetzt, deren erste Fläche um eine Drehachse schwenkbar ausgestaltet ist.

In einer ersten Position, die auch als Ausgangstellung bezeichnet wird, liegt die Oberfläche des auf dem ersten Schenkelabschnitt angeordneten Mikroarrays im Wesentlichen bündig an der zweiten Fläche an, d.h. die Substratoberfläche mit den darauf immobilisierten Sondenmoleküle ist im Wesentlichen nicht von der Probenlösung benetzt. In dem in der ersten Position zwischen dem zweiten Schenkelabschnitt der ersten Fläche und der zweiten Fläche gebildeten Raum, der Prozessierungskammer, erfolgt vorzugsweise in dieser ersten Position die Prozessierung der Reaktionslösung, d.h. insbesondere Aufreinigungs-, Aufkonzentrierungs-, Wasch- und Spül- und/oder Amplifikationsschritte.

Anschließend wird die schwenkbare erste Fläche in eine zweite Position gebracht, in der die erste Fläche in einem Winkel verschieden von 180°, vorzugsweise in einem Winkel von 45°, zu der zweiten Fläche angeordnet ist. Dies erfolgt vorzugsweise durch Zug auf den ersten Schenkelabschnitt der ersten Fläche und/oder durch Druck auf den zweiten Schenkelabschnitt der ersten Fläche durch ein wie vorstehend beschriebenes Mittel zur Führung der ersten Fläche. Durch Führen der ersten Fläche in die zweite Position wird der Mikroarray von der zweiten Fläche weggeführt und die Probenlösung dringt in den entstehenden Hohlraum zwischen Mikroarray und zweiter Fläche ein. Die auf dem Substrat des Mikroarrays immobilisierten Sondenmoleküle sind für die in der Probenlösung vorliegenden Targetmoleküle frei zugänglich, so dass eine Wechselwirkungsreaktion zwischen Sonden- und Targetmolekülen stattfinden kann. Die Ausübung eines Drucks und/oder Zugs auf die erste Fläche hat bei dieser Ausführungsform des erfindungsgemäßen Verfahrens den Vorteil, dass auf diese Weise die Probenlösung bewegt wird und so die Wechselwirkungsreaktion beschleunigt werden kann.

Zur Durchführung der Detektion und ggf. einer weiteren Prozessierung wird die schwenkbare erste Fläche wieder in die erste Position geführt, beispielsweise durch Druck auf den ersten Schenkelabschnitt der ersten Fläche und/oder durch Zug an dem zweiten Schenkelabschnitt der ersten Fläche bzw. bei elastischer Ausgestaltung der ersten Fläche durch Freilassen des ersten Schenkelabschnitts. Das Mikroarray liegt nun wiederum im Wesentlichen bündig an der zweiten Fläche an, so dass die Probenlösung zwischen der zweiten Fläche und dem Mikroarray in dieser Position im Wesentlichen verdrängt wird und eine im Wesentlichen hintergrundsfreie Detektion erfolgen kann.

Die zu untersuchenden Targets können in jeder Art von Probe, vorzugsweise in einer biologischen Probe vorliegen.

Vorzugsweise werden die Targets vor ihrer Detektion und Quantifizierung durch das erfindungsgemäße Verfahren isoliert, gereinigt, kopiert und/oder amplifiziert.

Das erfindungsgemäße Verfahren ermöglicht ferner die Amplifikation und den qualitativen und/oder quantitativen Nachweis von Nukleinsäuren in einer Reaktionskammer, wobei der Nachweis von molekularen Wechselwirkungen bzw. Hybridisierungen nach Abschluss einer zyklischen Amplifikationsreaktion erfolgen kann, ohne dass ein Austausch der Proben- bzw. Reaktionsflüssigkeiten erforderlich ist. Ferner gewährleistet das erfindungsgemäße Verfahren auch eine zyklische Detektion von Hybridisierungsereignissen bei der Amplifikation, d.h. einen Nachweis der Hybridisierung auch während der zyklischen Amplifikationsreaktion. Schließlich können mit Hilfe des erfindungsgemäßen Verfahrens die Amplifikationsprodukte während der Amplifikationsreaktion sowie nach Ablauf der Amplifikationsreaktion quantifiziert werden.

Die Amplifikation erfolgt üblicherweise durch herkömmliche PCR-Methoden oder durch ein wie vorstehend beschriebenes Verfahren zur parallelen Durchführung von Amplifikation der zu analysierenden Zielmoleküle durch PCR und Nachweis durch Hybridisierung der Zielmoleküle mit dem Substanzbibliothekenträger.

Bei einer weiteren Ausführungsform wird die Amplifikation als Multiplex-PCR in einem zweistufigen Prozess ausgeführt (siehe auch WO 97/45559). In einer ersten Stufe wird eine Multiplex-PCR durchgeführt, indem Fusionsprimer eingesetzt werden, deren 3'-Enden genspezifisch sind und deren 5'-Enden eine universelle Region darstellen. Letztere ist bei allen in der Multiplex-Reaktion eingesetzten forward- und reverse-Primern gleich. In dieser ersten Stufe ist die Primermenge limitierend. Dadurch können alle Multiplex-Produkte bis zu einem einheitlichen molaren Niveau amplifiziert werden, vorausgesetzt, dass die Zyklenzahl hinreichend ist, um für alle Produkte Primerlimitation zu erreichen. In einer zweiten Stufe sind universelle Primer zugegen, die identisch mit den 5'-Regionen der Fusionsprimer sind. Es erfolgt Amplifikation bis zur gewünschten DNA-Menge.

Bei einer weiteren bevorzugten Ausführungsform des erfindungsgemäßen Verfahrens erfolgt die Detektion während der zyklischen Amplifikationsreaktion und/oder nach Abschluss der zyklischen Amplifikationsreaktion. Vorzugsweise erfolgt die Detektion während der Amplifikationsreaktion bei jedem Amplifikationszyklus. Alternativ kann die Detektion aber auch bei jedem zweiten Zyklus oder jedem dritten Zyklus oder in beliebigen anderen Intervallen bestimmt werden.

Bei der Durchführung einer linearen Vervielfältigungsreaktion, bei der sich die Target-Menge mit jedem Schritt um ein bestimmte Menge erhöht, oder einer exponentiellen Vervielfältigungsreaktion, z.B. einer PCR, bei der sich die DNA-Target-Menge mit jedem Schritt vervielfältigt, in der Prozesseinheit kann somit nach jedem Vervielfältigungsschritt der Chip an die Detektionsebene gedrückt werden und damit die Detektion durchgeführt werden. Damit ist es möglich, eine Online-Überwachung der Vervielfältigungsreaktion durchzuführen. Insbesondere bei nichtlinearen Vervielfältigungsreaktionen ist es dadurch möglich, die Ausgangskonzentration der DNA-Target-Menge zu bestimmen.

Des Weiteren lässt sich so die Anzahl der Vervielfältigungsschritte online optimieren. Hat die DNA-Target-Menge eine bestimmte Konzentration erreicht, bricht man die Vervielfältigung ab. Ist die Target-Start-Konzentration klein, erhöht man die Zahl der Vervielfältigungsschritte, um eine sichere Analyse der Produkte durchführten zu können. Bei verminderter Reaktionszeit von Positiv-Kontrollen kann der Analyseprozess sehr früh abgebrochen werden.

Die für die Durchführung einer Amplifikationsreaktion erforderlichen Chemikalien wie z.B. Polymerase, Puffer, Magnesiumchlorid, Primer, markierte, insbesondere fluoreszenzmarkierte Primer, dNTP's etc. können in die Reaktionskammer beispielsweise gefriergetrocknet vorgelegt werden.

Vorzugsweise ist die zyklische Amplifikationsreaktion eine PCR. Bei der PCR werden für jeden PCR-Zyklus herkömmlicherweise drei Temperaturen durchfahren. Vorzugsweise lösen sich die hybridisierten Nukleinsäuren bei der höchsten Temperatur, d.h. der Denaturierungstemperatur von dem Mikroarray ab. Ein bevorzugter Wert für die Denaturierungstemperatur beträgt 95°C. Somit kann bei dieser Denaturierungstemperatur ein Hybridisierungssignal bestimmt werden, der als Nullwert bzw. Bezugswert für die bei dem jeweiligen PCR-Zyklus detektierten Nukleinsäuren dienen.

Bei der im PCR-Zyklus nachfolgenden Temperatur, einer Annealing-Temperatur von beispielsweise etwa 60°C, wird eine Hybridisierung zwischen nachzuweisenden Nukleinsäuren und den auf dem Substrat des Mikroarrays immobilisierten Nukleinsäuren ermöglicht. Bei einer Ausführungsform des erfindungsgemäßen Verfahrens erfolgt deswegen der Nachweis bzw. die Detektion von bei einem PCR-Zyklus vorliegenden Targetnukleinsäuren bei der Annealing-Temperatur.

Um die Empfindlichkeit des erfindungsgemäßen Verfahrens zu erhöhen, kann es ferner vorteilhaft sein, die Temperatur unter die Annealing-Temperatur zu senken, so dass die Detektion bevorzugt bei einer Temperatur unterhalb der Annealing-Temperatur eines Amplifikationszyklus erfolgt. Beispielsweise kann die Detektion bei einer Temperatur im Bereich von 25°C bis 50°C und vorzugsweise im Bereich von 30°C bis 45°C erfolgen.

Bei einer weiteren alternativen Ausführungsform des erfindungsgemäßen Verfahrens wird die Hybridisierung zwischen nachzuweisenden Nukleinsäuren und den auf dem Substrat des Mikroarrays immobilisierten Nukleinsäuren zunächst bei einer niedrigen Temperatur durchgeführt, um anschließend die Hybridisierungstemperatur zu erhöhen. Eine derartige Ausführungsform bietet den Vorteil, dass die Hybridisierungszeit gegenüber Hybridisierungen bei Temperaturen von über 50°C vermindert wird, ohne dabei an Spezifität in den Wechselwirkungen einzubüßen.

Wird von dem bei der bzw. unterhalb der Annealing-Temperatur bestimmten Messwert der bei der Denaturierungstemperatur bestimmte Nullwert bzw. Bezugswert abgezogen, so erhält man ein von Störeinflüssen freies Messergebnis, in dem Schwankungen und Drift eliminiert sind.

Üblicherweise werden die nachzuweisenden Targetmoleküle mit einem detektierbaren Marker versehen. Der Nachweis erfolgt bei dem erfindungsgemäßen Verfahren somit vorzugsweise dadurch, dass die gebundenen Targets mit mindestens einer Markierung versehen sind, die in Schritt b) detektiert wird.

Wie bereits vorstehend erwähnt, ist die Markierung, die an die Targets oder Sonden gekoppelt ist, vorzugsweise eine detektierbare Einheit oder eine über eine Ankergruppe an die Targets oder Sonden gekoppelte detektierbare Einheit. Hinsichtlich der Möglichkeiten der Detektion bzw. der Markierung ist das erfindungsgemäße Verfahren äußerst flexibel. So ist das erfindungsgemäße Verfahren mit einer Vielzahl physikalischer, chemischer oder biochemischer Detektionsverfahren kompatibel. Voraussetzung ist lediglich, dass die zu detektierende Einheit bzw. Struktur direkt an eine Sonde oder ein Target, beispielsweise ein Oligonukleotid gekoppelt bzw. über eine mit dem Oligonukleotid koppelbare Ankergruppe verknüpft werden kann.

Die Detektion der Markierung kann auf Fluoreszenz, Magnetismus, Ladung, Masse, Affinität, enzymatischer Aktivität, Reaktivität, einer Goldmarkierung u.dgl. beruhen. So basiert die Markierung vorzugsweise auf der Verwendung von Fluorophormarkierten Strukturen bzw. Bausteinen. In Verbindung mit der Fluoreszenz-Detektion kann die Markierung ein beliebiger an Targets oder Sonden während oder nach deren Synthese koppelbarer Farbstoff sein. Beispiele hierfür sind Cy-Farbstoffe (Amersham Pharmacia Biotech, Uppsala, Schweden), Alexa-Farbstoffe, Texas-Rot, Fluorescein, Rhodamin (Molecular Probes, Eugene, Oregon, USA), Lanthanide wie Samarium, Ytterbium und Europium (EG&G, Wallac, Freiburg, Deutschland).

Besonders bevorzugt ist dieser detektierbare Marker ein Fluoreszenzmarker. Wie bereits vorstehend erwähnt, gewährleistet der Einsatz der erfindungsgemäßen Vorrichtung in dem erfindungsgemäßen Verfahren das Detektieren der Fluoreszenzmarker mittels eines Fluoreszenzmikroskops ohne Autofokus, z.B. eines Fluoreszenzmikroskops mit Fixfokus.

Neben Fluoreszenz-Markern können im Rahmen der vorliegenden Erfindung als Markierung bzw. als Detektiereinheit, die mit den Targets bzw. Sonden gekoppelt ist, auch Lumineszenz-Marker, Metall-Marker, Enzym-Marker, radioaktive Marker und/oder polymere Marker eingesetzt werden.

Ebenso kann eine Nukleinsäure als Markierung (Tag) genutzt werden, die durch Hybridisierung mit einem markierten Reporter detektiert werden kann (Sandwich-Hybridisierung). Einsatz zum Nachweis des Tags finden diverse molekularbiologische Nachweisreaktionen wie Primer-Extension, Ligation und RCA.

Bei einer alternativen Ausführungsform des erfindungsgemäßen Verfahrens ist die detektierbare Einheit über eine Ankergruppe mit den Targets oder Sonden gekoppelt. Bevorzugt verwendete Ankergruppen sind Biotin, Digoxygenin u.dgl. Die Ankergruppe wird in einer anschließenden Reaktion mit spezifisch bindenden Komponenten, beispielsweise Streptavidin-Konjugaten oder Antikörper-Konjugaten umgesetzt, die selbst detektierbar sind oder eine detektierbare Reaktion auslösen. Bei Einsatz von Ankergruppen kann die Umsetzung der Ankergruppen in detektierbare Einheiten vor, während oder nach Zugabe der Probe umfassend die Targets bzw. ggf. vor, während oder nach der Spaltung einer selektiv spaltbaren Bindung in den Sonden erfolgen. Derartige selektiv spaltbare Bindungen in den Sonden sind z.B. in der internationalen Patentanmeldung WO 03/018838 beschrieben, auf deren diesbezüglichen Inhalt hiermit ausdrücklich Bezug genommen wird.

Die Markierung kann erfindungsgemäß auch durch Wechselwirkung eines markierten Moleküls mit den Sonden-Molekülen erfolgen. Beispielsweise kann die Markierung durch Hybridisierung eines wie vorstehend beschrieben markierten Oligonukleotids mit einer Oligonukleotid-Sonde bzw. einem Oligonukleotid-Target erfolgen.

Weitere im Rahmen der vorliegenden Erfindung geeignete Markierungsverfahren und Nachweissysteme sind beispielsweise in Lottspeich und Zorbas, Bioanalytik, Spektrum Akademischer Verlag, Heidelberg, Berlin, 1998, Kapitel 23.3 und 23.4 beschrieben.

Bei einer bevorzugten Ausführungsform des erfindungsgemäßen Verfahrens werden Nachweisverfahren eingesetzt, die im Ergebnis ein Addukt mit einem bestimmten Löslichkeitsprodukt, das eine Präzipitation zur Folge hat, liefern. Zur Markierung werden insbesondere Substrate bzw. Edukte eingesetzt, die in ein schwer lösliches, üblicherweise gefärbtes Produkt umgesetzt werden können. Beispielsweise können bei dieser Markierungsreaktion Enzyme verwendet werden, die den Umsatz eines Substrats in ein schwer lösliches Produkt katalysieren. Reaktionen, die geeignet sind, um zu einem Niederschlag an Array-Elementen zu führen, sowie Möglichkeiten für die Detektion des Niederschlags sind beispielsweise in der internationalen Patentanmeldung WO 00/72018 und in der internationalen Patentanmeldung WO 02/02810 beschrieben, auf deren diesbezüglichen Inhalt hiermit ausdrücklich Bezug genommen wird.

Bei einer besonders bevorzugten Ausführungsform des erfindungsgemäßen Verfahrens sind die gebundenen Targets mit einer Markierung versehen, die die Reaktion eines löslichen Substrats bzw. Edukts zu einem schwer löslichen Niederschlag auf dem Array-Element katalysiert, an dem eine Sonden/Target-Wechselwirkung stattgefunden hat bzw. die als Kristallisationskeim für die Umwandlung eines löslichen Substrats bzw. Edukts zu einem schwer löslichen Niederschlag auf dem Array-Element wirkt, an dem eine Sonden/Target-Wechselwirkung stattgefunden hat.

Der Einsatz des erfindungsgemäßen Verfahrens erlaubt auf diese Weise die simultane qualitative und quantitative Analyse einer Vielzahl von Sonden/Target-Wechselwirkungen, wobei einzelne Array-Elemente mit einer Größe von $\leq 1000\ \mu m$, vorzugsweise von $\leq 100\ \mu m$ und besonders bevorzugt von $\leq 50\ \mu m$ realisiert werden können.

In der Immunzytochemie und bei immunologischen Mikrotiterplatten-basierten Tests ist der Einsatz von enzymatischen Markierungen bekannt (siehe E. Lidell und I. Weeks, Antibody Technology, BIOS Scientific Publishers Limited, 1995). So katalysieren beispielsweise Enzyme den Umsatz eines Substrats in ein schwerlösliches, in aller Regel gefärbtes Produkt.

Besonders bevorzugt ist die zur Bildung eines Niederschlags an den Array-Elementen führende Reaktion eine durch ein Enzym katalysierte Umsetzung eines löslichen Substrats bzw. Edukts in ein schwer lösliches Produkt. Bei einer speziellen Ausführungsform ist die zur Bildung eines Niederschlags an den Array-Elementen führende Reaktion eine durch eine Peroxidase katalysierte Oxidation von 3,3',5,5'-Tetramethylbenzidin.

Vorzugsweise wird als Peroxidase für die Oxidation von 3,3', 5,5'-Tetramethylbenzidin Meerrettichperoxidase eingesetzt. Dem Fachmann sind jedoch weitere Peroxidasen bekannt, die zur Oxidation von 3,3',5,5'-Tetramethylbenzidin eingesetzt werden können.

Es wird angenommen, dass 3,3',5,5'-Tetramethylbenzidin unter der katalytischen Einwirkung einer Peroxidase in einem ersten Schritt zu einem blau gefärbten Radikalkation oxidiert wird (siehe z.B. Gallati und Pracht, J. Clin. Chem. Clin. Biochem. 1985, 23, 8, 454). Dieses blau gefärbte Radikalkation wird mittels eines Polyanions wie z.B. Dextransulfat als Komplex ausgefällt. Die Fällungsreaktion durch Peroxidase-katalysierte Oxidation von 3,3',5,5'-Tetramethylbenzidin ist beispielsweise in EP 0 456 782 beschrieben.

Die nachfolgende Tabelle 1 gibt, ohne den Anspruch zu erheben, vollständig zu sein, einen Überblick über eine Reihe von in Frage kommenden Reaktionen, die geeignet sind, um zu einem Niederschlag an Array-Elementen zu führen, an denen eine Wechselwirkung zwischen Target und Sonde erfolgt ist:

Tabelle 1

| Katalysator bzw. Kristallisationskeim | Substrat bzw. Edukt |
|---|---|
| Meerrettichperoxidase | DAB (3,3'-Diaminobenzidin) 4-CN (4-Chlor-1-Napthol) AEC (3-Amino-9-Ethylcarbazol) HYR (p-Phenylendiamin-HCl und Pyrocatechol) TMB (3,3',5,5'-Tetramethylbenzidin) Naphtol/Pyronin |
| Alkalische Phosphatase | Bromchlorindoylphosphat (BCIP) und Nitrotetrazoliumblau (NBT) |
| Glucoseoxidase | t-NBT und m-PMS (Nitrotetrazoliumblauchlorid und Phenazinmethosulfat) |
| Goldpartikel | Silbernitrat Silbertartrat |

Der Nachweis und/oder die Detektion von Sonde/Target-Wechselwirkungen über unlösliche Präzipitate sind insbesondere in WO 02/02810 beschrieben.

Im Folgenden werden Ausführungsformen der vorliegenden Erfindung beschrieben, die zur Überwindung von generell bei der Detektion von molekularen Wechselwirkungen auf festen Trägern möglicherweise auftretenden Problemen wie beispielsweise zur Verhinderung der etwaigen Ausbildung von Newtonschen Ringen zwischen Detektionsebene und Sondenarray dienen können.

Die Ausprägung von Newtonschen Ringen wird im Wesentlichen durch die Art der Beleuchtung, die Wellenlänge des zur Detektion eingesetzten Lichtes, den Abstand zwischen Detektionsebene und Sondenarray sowie den Brechungsindex der in der Kammer befindlichen Lösung bestimmt. Derartige Newtonsche Ringe können beispielsweise vermieden werden durch Änderung der Wellenlänge des zur Detektion eingesetzten Lichtes, Verwendung einer Lösung mit gleichem oder ähnlichem Brechungsindex wie dem der Detektionsebene und/oder des Sondenarrays und/oder die Verwendung einer Immersionsflüssigkeit zwischen Detektionsebene und Sondenarray.

Ferner können Newtonsche Ringe durch Aufbringen von Abstandshaltern auf dem Chip und/oder der dem Chip zugewandten Seite der Detektionsfläche verhindert werden.

Ferner können Newtonsche Ringe durch Aufbringen des Sondenarrays auf eine raue Trägeroberfläche verhindert werden.

Ferner können Newtonsche Ringe durch Aufbringen des Sondenarrays auf eine lichtabsorbierende Oberfläche verhindert werden.

Als weitere Möglichkeit kann während der Detektion der Anpressdruck, mit welchem der Chip relativ zur Detektionsfläche geführt wird, permanent variiert werden. Dadurch wird die Spaltdicke zwischen Chip und Detektionsfläche und so auch die Lage der Newtonschen Ringe verändert. Durch die Integration des zu detektierenden Fluoreszenzsignals über die Zeit wird auf diese Weise eine Verfälschung der Messwerte der Spots relativ zueinander vermieden.

Eine weitere besonders bevorzugte Möglichkeit zur Verhinderung von Newtonschen Ringen ist die Verwendung mehrerer Lichtquellen aus unterschiedlichen Richtungen zur Beleuchtung und damit zur Anregung der Fluorophore der gebundenen Targets.

Hintergrundfluoreszenz, die durch die Fluorophore ungebundener Targets in der verdrängten Flüssigkeit hervorgerufen wurde, kann zu einer Verfälschung des detektierten Signals führen. Dies kann vorzugsweise durch Einsatz einer Blende verhindert werden, die z.B. auf die Detektionsfläche oder den Chip aufgebracht und/oder um den Chip herum oder in der Abbildungsoptik angeordnet wird und derart ausgestaltet ist, dass nur die Fläche des Sondenarrays beleuchtet bzw. abgebildet wird.

Bei der Verwendung entsprechender Beleuchtungsquellen wie z.B. Laser kann es, bedingt durch die Kohärenz des Lichtes, zu Inhomogenitäten in der Beleuchtung kommen. Derartige Inhomogenitäten können durch Einsatz von Wellenleitern und/oder Mischfiltern und/oder Licht verschiedener Wellenlängen verringert bzw. vermieden werden. Ebenso ist eine Bewegung der Beleuchtungsquelle zur Beseitigung derartiger Effekte denkbar.

Durch Einsatz einer organischen oder anorganischen lichtabsorbierenden und im gewählten Wellenlängenbereich nicht fluoreszierenden Schicht auf dem Träger des Sondenarrays kann Fluoreszenzhintergrundsignal, das durch den Sondenträger und/oder dahinter befindliche Elemente verursacht wird, verringert bzw. verhindert werden. Vorzugsweise wird eine Schwarzchromschicht als Schutzschicht eingesetzt.

Bei sämtlichen vorstehend beschriebenen Ausführungsformen des erfindungsgemäßen Verfahrens ist eine Voramplifikation des zu analysierenden Materials nicht erforderlich. Von dem aus Bakterien, Blut oder anderen Zellen extrahierten Probenmaterial können gezielte Teilbereiche mit Hilfe einer PCR (Polymerase-Kettenreaktion) insbesondere in Anwesenheit der erfindungsgemäßen Vorrichtung bzw. des Substanzbibliothekenträgers wie in DE 102 53 966 beschrieben amplifiziert und an den Träger hybridisiert werden. Dies stellt eine wesentliche Vereinfachung des Arbeitsaufwands dar.

Das erfindungsgemäße Verfahren ist somit insbesondere zur parallelen Durchführung von Amplifikation der zu analysierenden Zielmoleküle durch PCR und Nachweis durch Hybridisierung der Zielmoleküle mit dem Substanzbibliothekenträger geeignet. Dabei wird die nachzuweisende Nukleinsäure zunächst durch eine PCR amplifiziert, wobei der Reaktion zu Anfang vorzugsweise mindestens ein Kompetitor zugesetzt wird, der die Bildung eines der beiden durch die PCR amplifizierten Template-Stränge inhibiert. Insbesondere wird bei der PCR ein DNA-Molekül zugesetzt, das mit einem der zur PCR-Amplifikation des Templates verwendeten Primer um die Bindung an das Template konkurriert, und nicht enzymatisch verlängert werden kann. Die durch die PCR amplifizierten einzelsträngigen Nukleinsäuremoleküle werden dann durch Hybridisierung mit einer komplementären Sonde nachgewiesen. Alternativ wird die nachzuweisende Nukleinsäure zunächst im Einzelstrangüberschuss durch eine PCR amplifiziert und durch eine anschließende Hybridisierung mit einer komplementären Sonde nachgewiesen, wobei der PCR-Reaktion zu Anfang ein Kompetitor zugesetzt wird, bei dem es sich um ein DNA-Molekül oder ein Molekül eines Nukleinsäure-Analogons handelt, das an einen der beiden Stränge des Templates hybridisieren kann, aber nicht an den Bereich, der durch die Sonden-Hybridisierung nachgewiesen wird, und das enzymatisch nicht verlängerbar ist.

Als Kompetitor in der PCR kann jedes Molekül eingesetzt werden, das eine bevorzugte Amplifikation nur eines der beiden in der PCR-Reaktion vorhandenen Template-Stränge bewirkt. Bei Kompetitoren kann es sich daher erfindungsgemäß um Proteine, um Peptide, um DNA-Liganden, um Interkalatoren, um Nukleinsäuren oder deren Analoga handeln. Als Kompetitoren werden bevorzugt Proteine bzw. Peptide eingesetzt, die in der Lage sind, einzelsträngige Nukleinsäuren mit Sequenz-Spezifität zu binden und über die oben definierten Eigenschaften verfügen. Besonders bevorzugt werden als Sekundärstrukturbrecher Nukleinsäuremoleküle und Nukleinsäure-Analoga-Mo leküle eingesetzt.

Durch anfängliche Zugabe des Kompetitors zur PCR während der Amplifikation wird die Bildung eines der beiden Template-Stränge im Wesentlichen inhibiert. "Im Wesentlichen inhibiert" bedeutet, dass im Rahmen der PCR ein ausreichender Einzelstrangüberschuss und eine ausreichende Menge des anderen Template-Strangs hergestellt werden, um einen effizienten Nachweis des amplifizierten Strangs durch die Hybridisierung zu gewährleisten. Die Amplifikation erfolgt damit nicht einer exponentiellen Kinetik der Form $2^n$ (mit n = Anzahl der Zyklen), sondern einer gedämpften Amplifikationskinetik der Form $<2^n$.

Der durch die PCR erzielte Einzelstrangüberschuss beträgt gegenüber dem nichtamplifizierten Strang den Faktor 1,1 bis 1000, bevorzugt den Faktor 1,1 bis 300, ebenfalls bevorzugt den Faktor 1,1 bis 100, besonders bevorzugt den Faktor 1,5 bis 100, ebenfalls besonders bevorzugt den Faktor 1,5 bis 50, insbesondere bevorzugt den Faktor 1,5 bis 20 und am meisten bevorzugt den Faktor 1,5 bis 10.

Typischerweise wird die Funktion eines Kompetitors darin bestehen, dass er selektiv an einen der beiden Template-Stränge bindet und damit die Amplifikation des entsprechenden komplementären Stranges behindert. Als Kompetitoren kommen daher einzelsträngige DNA- oder RNA-bindende Proteine mit Spezifität für einen der beiden in einer PCR zu amplifizierenden Template-Stränge in Frage. Ebenso kann es sich um Aptamere handeln, die Sequenzspezifisch nur an bestimmte Bereiche eines der beiden zu amplifizierenden Template-Stränge binden.

Bevorzugt werden Nukleinsäuren oder Nukleinsäure-Analoga als Kompetitoren eingesetzt. Üblicherweise werden die Nukleinsäuren bzw. Nukleinsäure-Analoga dadurch als Kompetitor der PCR wirken, dass sie entweder mit einem der zur PCR verwendeten Primer um die Primer-Bindungsstelle konkurrieren oder, aufgrund einer Sequenz-Komplementarität mit einem Bereich eines nachzuweisenden Template-Strangs hybridisieren können. Bei diesem Bereich handelt es sich nicht um die Sequenz, die durch die Sonde nachgewiesen wird. Solche Nukleinsäure-Kompetitoren sind enzymatisch nicht verlängerbar.

Bei den Nukleinsäure-Analoga kann es sich z.B. um sogenannte Peptid-Nukleinsäuren (peptide nucleic acids, PNA) handeln. Bei Nukleinsäure-Analoga kann es sich aber auch um Nukleinsäuremoleküle handeln, bei denen die Nukleotide über eine Phosphothioat-Bindung anstelle einer Phosphat-Bindung miteinander verknüpft sind. Ebenso kann es sich um Nukleinsäure-Analoga handeln, bei denen die natürlich vorkommenden Zuckerbausteine Ribose bzw. Deoxyribose gegen alternative Zucker wie z.B. Arabinose oder Trehalose etc. ausgetauscht wurden. Weiterhin kann es sich bei dem Nukleinsäurederivat um "locked nucleic acid" (LNA) handeln. Weitere übliche Nukleinsäure-Analoga sind dem Fachmann bekannt.

Bevorzugt werden als Kompetitoren DNA- oder RNA-Moleküle, insbesondere bevorzugt DNA- oder RNA-Oligonukleotide bzw. deren Analoga eingesetzt.

Abhängig von der Sequenz der als Kompetitoren eingesetzten Nukleinsäuremoleküle bzw. Nukleinsäure-Analoga beruht die Inhibierung der Amplifikation eines der beiden Template-Stränge im Rahmen der PCR-Reaktion auf unterschiedlichen Mechanismen. Dies wird im Folgenden beispielhaft anhand eines DNA-Moleküls diskutiert.

Wenn als Kompetitor z.B. ein DNA-Molekül verwendet wird, kann dies eine Sequenz aufweisen, die mit der Sequenz eines der zu PCR verwendeten Primer zumindest teilweise derart identisch ist, dass eine spezifische Hybridisierung des DNA-Kompetitor-Moleküls mit dem entsprechenden Template-Strang unter stringenten Bedingungen möglich ist. Da das zur Kompetition verwendete DNA-Molekül erfindungsgemäß in diesem Fall nicht durch eine DNA-Polymerase verlängerbar ist, kompetiert das DNA-Molekül mit dem jeweiligen Primer während der PCR-Reaktion um die Bindung an das Template. Je nach Mengenverhältnis des DNA-Kompetitor-Moleküls zum Primer kann auf diese Weise die Amplifikation des durch den Primer definierten Template-Strangs derart inhibiert werden, dass die Herstellung dieses Template-Strangs deutlich reduziert ist. Die PCR verläuft dabei nach einer exponentiellen Kinetik, die höher ist, als bei den verwendeten Kompetitor-Mengen zu erwarten wäre. Auf diese Weise entsteht ein Einzelstrangüberschuss in einer Menge, die ausreichend für einen effizienten Nachweis der amplifizierten Target-Moleküle durch Hybridisierung ist.

Bei dieser Ausführungsform dürfen die zur Kompetition verwendeten Nukleinsäuremoleküle bzw. Nukleinsäureanaloga enzymatisch nicht verlängerbar sein. "Enzymatisch nicht verlängerbar" bedeutet, dass die zur Amplifikation verwendete DNA- oder RNA-Polymerase den Nukleinsäure-Kompetitor nicht als Primer verwenden kann, d.h. nicht in der Lage ist, 3' von der durch den Kompetitor definierten Sequenz den jeweiligen Gegenstrang zum Template zu synthetisieren.

Alternativ zu der oben dargestellten Möglichkeit kann das DNA-Kompetitor-Molekül auch über eine Sequenz verfügen, die zu einem Bereich des nachzuweisenden Template-Strangs komplementär ist, der nicht durch eine der Primer-Sequenzen adressiert wird, und die enzymatisch nicht verlängerbar ist. Im Rahmen der PCR wird das DNA-Kompetitor-Molekül dann an diesem Template-Strang hybridisieren und die Amplifikation dieses Stranges entsprechend blockieren.

Dem Fachmann ist bekannt, dass die Sequenzen von DNA-Kompetitor-Molekülen oder allgemein Nukleinsäure-Kompetitor-Molekülen entsprechend gewählt werden können. Wenn die Nukleinsäure-Kompetitor-Moleküle eine Sequenz aufweisen, die nicht mit der Sequenz eines der zur PCR verwendeten Primer im Wesentlichen identisch, sondern zu einem anderen Bereich des nachzuweisenden Template-Strangs komplementär ist, ist diese Sequenz so zu wählen, dass sie nicht in den Bereich der Template-Sequenz fällt, der im Rahmen der Hybridisierung mit einer Sonde nachgewiesen wird. Dies ist deswegen notwendig, da zwischen der PCR und der Hybridisierungsreaktion keine Aufarbeitungsreaktion stattfinden muss. Würde als Kompetitor ein Nukleinsäuremolekül verwendet, das in den nachzuweisenden Bereich fällt, würde dies mit dem einzelsträngigen Target-Molekül um die Bindung an die Sonde kompetieren.

Bevorzugt hybridisieren solche Kompetitoren in der Nähe der Template-Sequenz, die durch die Sonde nachgewiesen wird. Die Positionsangabe "in der Nähe" ist dabei erfindungsgemäß so zu verstehen, wie sie für Sekundärstrukturbrecher angegeben ist. Allerdings können die erfindungsgemäßen Kompetitoren auch in unmittelbarer Nachbarschaft der nachzuweisenden Sequenz hybridisieren, d.h. exakt ein Nukleotid von der nachzuweisenden Target-Sequenz entfernt.

Wenn als kompetitierende Moleküle enzymatisch nicht verlängerbare Nukleinsäuren oder Nukleinsäure-Analoga verwendet werden, sind diese hinsichtlich ihrer Sequenz oder Struktur so zu wählen, dass sie nicht enzymatisch durch DNA- oder RNA-Polymerasen verlängert werden können. Bevorzugt ist das 3'-Ende eines Nukleinsäure-Kompetitors so ausgelegt, dass es keine Komplementarität zum Template aufweist und/oder anstelle der 3-OH-Gruppe am 3'-Ende einen anderen Substituenten trägt.

Weist das 3'-Ende des Nukleinsäure-Kompetitors keine Komplementarität zum Template auf, unabhängig davon, ob der Nukleinsäure-Kompetitor an eine der Primer-Bindungsstellen des Templates oder an eine der durch die PCR zu amplifizierenden Sequenzen des Templates bindet, kann der Nukleinsäure-Kompetitor wegen der fehlenden Basen-Komplementarität am 3'-Ende nicht durch die gängigen DNA-Polymerasen verlängert werden. Diese Art der Nicht-Verlängerbarkeit von Nukleinsäure-Kompetitoren durch DNA-Polymerasen ist dem Fachmann bekannt. Bevorzugt weist der Nukleinsäure-Kompetitor an seinem 3'-Ende bezüglich der letzten 4 Basen, besonders bevorzugt bezüglich der letzten 3 Basen, insbesondere bevorzugt bezüglich der letzten 2 Basen und am meisten bevorzugt bezüglich der letzten Base keine Komplementarität zu seiner Zielsequenz auf. Solche Kompetitoren können an den genannten Positionen auch nicht-natürliche Basen aufweisen, die keine Hybridisierung erlauben.

Nukleinsäure-Kompetitoren, die enzymatisch nicht verlängerbar sind, können auch eine 100%-ige Komplementarität zu ihrer Zielsequenz aufweisen, wenn sie in ihrem Rückgrat oder an ihrem 3'-Ende derart modifiziert sind, dass sie enzymatisch nicht verlängerbar sind.

Weist der Nukleinsäure-Kompetitor an seinem 3'-Ende eine andere Gruppe als die OH-Gruppe auf, handelt es sich bei diesen Substituenten bevorzugt um eine Phosphat-Gruppe, um ein Wasserstoff-Atom (Dideoxynukleotid), eine Biotingruppe oder eine Aminogruppe. Diese Gruppen können durch die gängigen Polymerasen nicht verlängert werden.

Besonders bevorzugt wird bei einem derartigen Verfahren als Kompetitor ein DNA-Molekül verwendet, das mit einem der beiden zur PCR verwendeten Primer um die Bindung an das Template kompetiert und welches am 3'-Ende während der chemischen Synthese mit einem Aminolink versehen wurde. Solche Kompetitoren können 100%-ige Komplementarität zu ihrer Zielsequenz haben.

Nukleinsäure-Analoga-Kompetitoren wie z.B. PNAs müssen dagegen nicht über eine blockierte 3'-OH-Gruppe oder eine nicht-komplementäre Base an ihrem 3'-Ende verfügen, da sie aufgrund des durch die Peptid-Bindung veränderten Rückgrats nicht durch die DNA-Polymerasen erkannt und somit auch nicht verlängert werden. Entsprechende andere Modifikationen der Phosphatgruppe, die durch die DNA-Polymerasen nicht erkannt werden, sind dem Fachmann bekannt. Dazu gehören u.a. Nukleinsäuren mit Rückgratmodifikationen wie z.B. 2'-5' Amid-Bindungen (Chan et al. (1999) J. Chem. Soc., Perkin Trans. 1, 315-320), Sulfid-Bindungen (Kawai et al. (1993) Nucleic Acids Res., 1 (6), 1473-1479), LNA (Sorensen et al. (2002) J. Am. Chem. Soc., 124 (10), 2164-2176) und TNA (Schoning et al. (2000) Science, 290 (5495), 1347-1351).

Es können auch mehrere Kompetitoren, die an unterschiedliche Bereiche des Templates (z.B. u.a. die Primer-Bindungsstelle) hybridisieren, gleichzeitig in einer PCR eingesetzt werden. Wenn die Kompetitoren über Sekundärstrukturbrechereigenschaften verfügen, kann dadurch die Effizienz der Hybridisierung zusätzlich gesteigert werden.

In einer alternativen Ausführungsform kann das DNA-Kompetitor-Molekül über eine zu einem der Primer komplementäre Sequenz verfügen. Solche z.B. Antisense-DNA-Kompetitor-Moleküle können dann je nach Mengenverhältnis zwischen Antisense-DNA-Kompetitor-Molekül und Primer dazu verwendet werden, den Primer in der PCR-Reaktion zu titrieren, so dass dieser nicht mehr mit dem jeweiligen Template-Strang hybridisiert und entsprechend nur der durch den anderen Primer definierte Template-Strang amplifiziert wird. Dem Fachmann ist bewusst, dass bei dieser Ausführungsform der Erfindung der Nukleinsäure-Kompetitor enzymatisch verlängerbar sein kann, aber nicht muss.

Wenn im Rahmen dieser Erfindung von Nukleinsäure-Kompetitoren gesprochen wird, schließt dies Nukleinsäure-Analoga-Kompetitoren mit ein, wenn sich nicht aus dem Kontext etwas anderes ergibt. Der Nukleinsäure-Kompetitor kann an den entsprechenden Strang des Templates reversibel oder irreversibel binden. Die Bindung kann durch kovalente bzw. nicht kovalente Wechselwirkungen erfolgen.

Bevorzugt erfolgt die Bindung des Nukleinsäure-Kompetitors über nicht-kovalente Wechselwirkungen und ist reversibel. Insbesondere bevorzugt erfolgt die Bindung an das Template durch Ausbildung von Watson-Crick Basenpaarungen.

Die Sequenzen der Nukleinsäure-Kompetitoren richten sich in der Regel nach der Sequenz des Template-Strangs, der nachgewiesen werden soll, bei antisense-Primern dagegen nach den zu titrierenden Primer-Sequenzen, die aber wiederum durch die Template-Sequenzen definiert sind.

Bei der PCR-Amplifikation von Nukleinsäuren handelt es sich um eine Labor-Standardmethode, mit deren vielfältigen Variations- und Ausgestaltungsmöglichkeiten der Fachmann vertraut ist. Prinzipiell ist eine PCR dadurch charakterisiert, dass das doppelsträngige Nukleinsäure-Template, üblicherweise ein doppelsträngiges DNA-Molekül, zuerst einer Hitze-Denaturierung für 5 Minuten bei 95° C unterworfen wird, wodurch die beiden Stränge voneinander getrennt werden. Nach einer Abkühlung auf die sogenannte "annealing"-Temperatur (definiert durch den Primer mit der niedrigeren Schmelztemperatur) lagern sich die in der Reaktionslösung vorhandenen "forward"- und "reverse"-Primer an die zu ihrer Sequenz komplementären Stellen in den jeweiligen Template-Strängen an. Die "annealing"-Temperatur der Primer richtet sich dabei nach der Länge und Basenzusammensetzung der Primer. Sie kann aufgrund theoretischer Überlegungen kalkuliert werden. Angaben zur Kalkulation von "annealing"-Temperaturen finden sich z.B. in Sambrook et al. (*vide supra*).

Nach dem Annealen der Primer, das typischerweise in einem Temperaturbereich von 40-75 °C, bevorzugt von 45-72 °C und insbesondere bevorzugt von 50-72 °C erfolgt, folgt ein Elongationsschritt, bei dem durch die Aktivität der in der Reaktionslösung vorhandenen DNA-Polymerase Desoxyribonukleotide mit dem 3'-Ende der Primer verknüpft werden. Die Identität der eingefügten dNTPs richtet sich dabei nach der Sequenz des mit dem Primer hybridisierten Template-Strangs. Da in der Regel thermostabile DNA-Polymerasen eingesetzt werden, läuft der Elongationsschritt üblicherweise zwischen 68-72° C ab.

Bei der symmetrischen PCR wird durch eine Wiederholung dieses beschriebenen Zyklus aus Denaturierung, Annealing der Primer und Elongation der Primer eine exponentielle Vermehrung des durch die Primersequenzen definierten Nukleinsäureabschnitts des Targets erreicht. Hinsichtlich der Pufferbedingungen bei der PCR, der verwendbaren DNA-Polymerasen, der Herstellung von doppelsträngigen DNA-Templates, des Designs von Primern, der Wahl der Annealing-Temperatur und Variationen der klassischen PCR steht dem Fachmann zahlreiche Literatur zur Verfügung.

Dem Fachmann ist geläufig, dass als Template auch z.B. einzelsträngige RNA, wie z.B. mRNA, eingesetzt werden kann. Diese wird in der Regel vorher durch eine Reverse Transkription in eine doppelsträngige cDNA überführt.

In einer bevorzugten Ausführungsform wird als Polymerase eine thermostabile DNA-abhängige DNA-Polymerase verwendet. In einer besonders bevorzugten Ausführungsform wird eine thermostabile DNA-abhängige DNA-Polymerase ausgewählt aus der Gruppe bestehend aus Taq-DNA-Polymerase (Eppendorf, Hamburg, Deutschland sowie Qiagen, Hilden, Deutschland), Pfu-DNA-Polymerase (Stratagene, La Jolla, USA), Tth-DNA-Polymerase (Biozym Epicenter Technol., Madison, USA), Vent-DNA-Polymerase, DeepVent-DNA-Polymerase (New England Biolabs, Beverly, USA), Expand-DNA-Polymerase (Roche, Mannheim, Deutschland) verwendet.

Die Verwendung von Polymerasen, die aus natürlich vorkommenden Polymerasen durch gezielte oder evolutive Veränderung optimiert worden sind, ist ebenfalls bevorzugt. Bei der Durchführung der PCR in Gegenwart des Substanzbibliothekenträgers ist insbesondere die Verwendung der Taq-Polymerase der Firma Eppendorf (Hamburg, Deutschland) bzw. des Advantage-cDNA-Polymerase-Mix von Clontech (Palo Alto, CA, USA) bevorzugt.

Ein weiterer Aspekt der vorliegenden Erfindung betrifft die Verwendung der erfindungsgemäßen Vorrichtung zur Durchführung von Mikroarray-basierten Tests.

Im Folgenden sind spezielle Ausgestaltungen der erfindungsgemäßen Vorrichtung bzw. des erfindungsgemäßen Verfahren dargestellt.

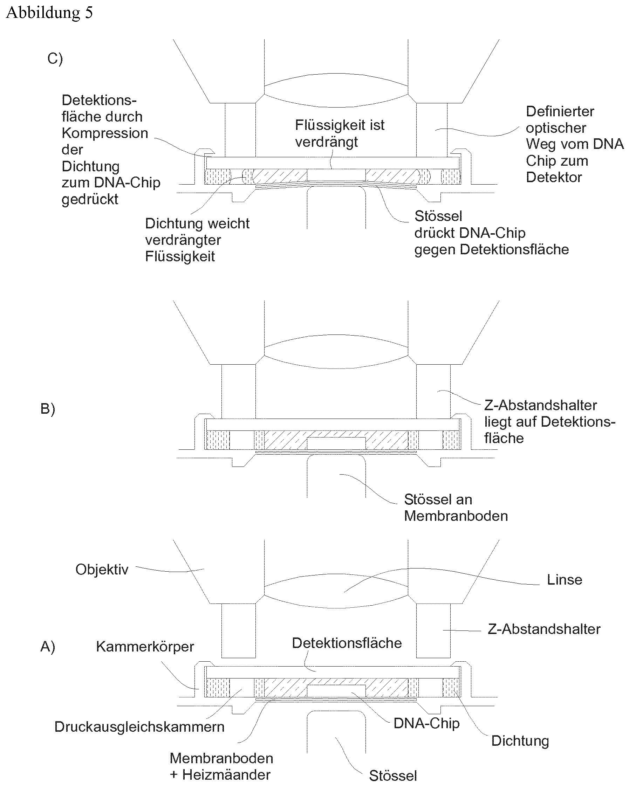

In Abbildung 5 wird gezeigt, dass die erste Fläche, hier eine elastische Membran, in die vorzugsweise eine Heizvorrichtung integriert ist, durch einen Stift bzw. einen Stößel deformiert und dadurch der Chip in Richtung der Detektionsebene gedrückt wird. Ferner wird durch einen Abstandshalter auf der zweiten Fläche die Detektionsebene in die Reaktionskammer gedrückt und nähert sich damit von oben dem DNA-Chip, bis die Flüssigkeit zwischen DNA-Chip und Detektionsebene nahezu vollständig verdrängt wird. Durch das Führen der Detektionsfläche in Richtung des Chips werden die die Reaktionskammer abdichtenden elastischen Dichtungen komprimiert. Die verdrängte Flüssigkeit deformiert die Dichtung derart, dass die Luft in Druckausgleichskammern komprimiert wird.

Die Prozesseinheit kann aber auch so gestaltet werden, dass entweder nur die erste Fläche, z.B. in Form einer elastischen Membran, verformt wird oder aber nur die Detektionsebene in die Kammer gedrückt wird.

In Abbildung 6 ist eine weitere technische Ausgestaltung zur Kompression der Prozesseinheit dargestellt. Die Reaktionskammer ist seitlich und auf der der Detektionsebene gegenüberliegenden Seite von einer Dichtungsmembran umschlossen, auf der der DNA-Chip befestigt ist. Die Dichtungsmembran verschließt auf der Höhe des DNA-Chips ein Loch an der Unterseite des Kammerkörpers. Das Loch ist etwas kleiner als der DNA-Chip. Bei der Durchführung einer PCR in der Reaktionskammer wird durch den aufgrund der mit der PCR verbundenen höheren Temperaturen entstehenden Innendruck in der Kammer das Loch fest verschlossen. Die Kammer ist also trotz labiler Dichtungsmembran druckfest (Prinzip des selbstschließenden Ventils). Zur Detektion wird ein Stift bzw. ein Stößel durch das Unterseitenloch geschoben. Die Dichtungsmembran wird angehoben und der DNA-Chip gegen die Detektionsebene gedrückt. Um die notwendige Elastizität der Dichtungsmembran zu gewährleisten, kann die Membran mit einer Ausgleichsfalte versehen werden. Durch die verdrängte Flüssigkeit werden auch bei dieser Ausgestaltung die Druckausgleichskammern komprimiert.

Die folgenden Beispiele dienen der Erläuterung der Erfindung und sollen in nicht einschränkender Weise ausgelegt werden.

Beispiele

Beispiel 1: Aufbau einer Reaktionskartusche ohne integrierte Heizung

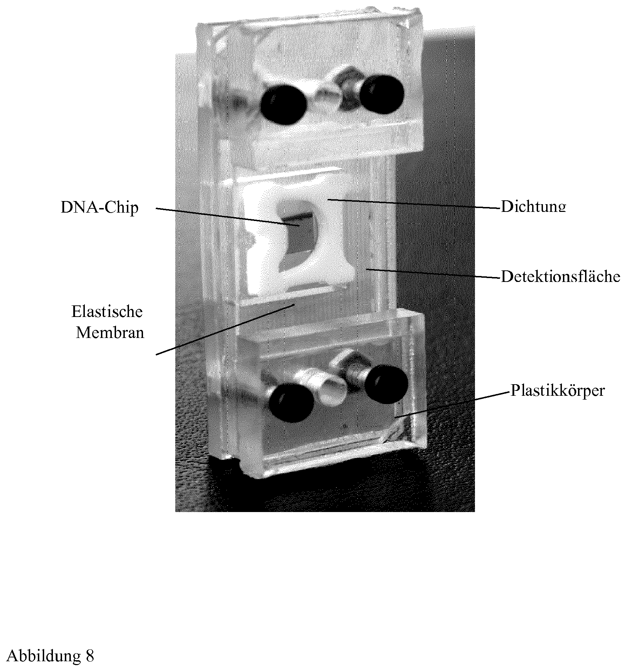

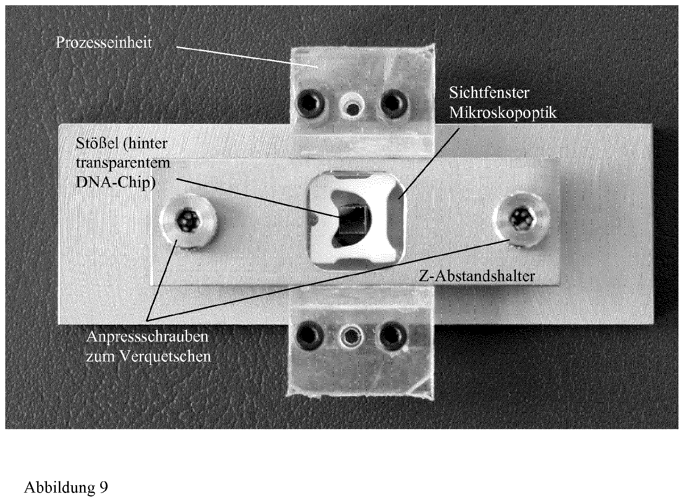

In den Abbildungen 8 und 9 ist ein Ausführungsbeispiel einer Prozessierungseinheit ohne integrierte Heizung sowie eine Vorrichtung zum Führen des DNA-Chips gegen die Detektionsebene dargestellt. Der DNA-Chip in der gezeigten Vorrichtung kann durch ein konventionelles Fluoreszenzmikroskop (z.B. Axioskop, Zeiss, Jena, Deutschland) ausgelesen werden.

Beispiel 2: Aufbau einer Reaktionskartusche mit Silizium-Heizsubstrat

Bei der in den Abbildungen 10 und 11 dargestellten Variante der Prozessierungseinheit der erfindungsgemäßen Vorrichtung handelt es sich um eine miniaturisierte Reaktionskartusche mit einem integrierten Sonden-Array (DNA-Chip), einem Silizium-Heizsubstrat mit integriertem Temperatursensor ("Heizsubstrat") zur Einstellung distinkter Temperaturen in der Reaktionskammer sowie einer Leiterplatte mit optionalem EPROM zur elektrischen Kontaktierung des Heizsubstrates. Die einzelnen Bestandteile sind in zwei aus Kunststoff gefertigten Halbschalen eingebettet. Die gesamte Einheit stellt ein räumlich geschlossenes System dar, in dem alle erforderlichen Reaktionen (z.B. PCR) z.B. temperaturgesteuert vorgenommen werden können.

In die untere Halbschale wird als erstes die Leiterplatte (mit dem EPROM nach unten) in den vorgesehenen Schacht eingelegt. Auf der Oberseite der Leiterplatte sind drei elektrische Kontaktierungsflächen ("Kontaktpads") angeordnet, welche die elektrische Verbindung mit dem nachfolgend eingelegten Heizsubstrat gewährleisten, welches ebenfalls drei Kontaktpads trägt. Dieses Heizsubstrat hat eine Größe 8 mm x 6 mm und eine Dicke von etwa 0,6 mm.

Das Heizsubstrat gewährleistet eine genaue Einstellung verschiedener Temperaturen (z.B. von 40°C-95°C) innerhalb der durchgeführten Untersuchung. Die Messung der Temperatur in der Reaktionskammer kann dabei entweder über den im Heizsubstrat integrierten Sensor oder aber über eine externe Messeinheit, welche die Temperatur direkt auf der Oberfläche des Heizsubstrates misst, erfolgen. In letzterem Falle kann auf den integrierten Sensor im Heizsubstrat verzichtet werden. Die zum Heizen und/oder zur Temperaturmessung verwendeten integrierten Bauelemente können zum Beispiel Dioden oder auch Transistoren sein. Die dem Reaktionsraum zugewandte Fläche des Silizium-Heizsubstrates enthält keinerlei elektrische Systeme und ist mit einer $SiO_2$-Passivierungsschicht überzogen.

Als nächstes Bauteil folgt eine elastische Dichtung, welche den Reaktionsraum seitlich begrenzt.

In der Mitte des Reaktionsraumes wird der DNA-Chip so befestigt, dass das Sonden-Array der Detektionsebene zugewandt ist. Nach dem Einbau der Detektionsebene in Form einer Glasfläche ragt diese noch 0,2 mm aus der unteren Halbschale hervor. Durch das nachfolgende Anfügen der von Passstiften geführten oberen Halbschale wird die Glasfläche gegen die Dichtung gepresst und gewährleistet auf diese Weise eine optimale Abdichtung der Reaktionskammer.

Anschließend kann die Reaktionskammer mit Reaktionslösung befüllt werden. Dabei ist zu beachten, dass nur der Innenraum mit dem Chip, nicht aber die äußeren Kammern befüllt werden. Die benötigten Flüssigkeiten werden mit Kanülen über die vorgesehene Kanülenführung in den Reaktionsraum eingespritzt.

Anschließend können über das Silizium-Heizsubstrat gesteuerte biochemische Reaktionen, wie z.B. PCR und/ oder Hybridisierung in der Reaktionskammer durchgeführt werden.

Zur Detektion der Zwischenergebnisse oder des Endresultats wird die Detektionsebene mittels der Abstandshalter der Detektionseinheit von oben gegen den DNA-Chip gedrückt, bis der Abstand zwischen Detektionsebene und Sondenarray etwa null ist. Dabei wird die umliegende Flüssigkeit in die äußeren Kammern verdrängt, wo sie die dortige Luft komprimiert. Dieser Vorgang ist reversibel und kann zum Beispiel nach jedem PCR-Zyklus vorgenommen werden.

Durch das kompakte Design sowie die interne Leiterplatte mit EPROM und das integrierte Heizsubstrat ist diese Variante der erfindungsgemäßen Vorrichtung besonders für den mobilen Einsatz geeignet.

Beispiel 3: Nachweis der Abnahme des Hintergrundsignals durch Verdrängung des Analyten

Alle in diesem Beispiel beschriebenen Fluoreszenzmessungen wurden mit einem Fluoreszenzmikroskop (Zeiss, Jena, Deutschland) vorgenommen. Die Anregung erfolgte im Auflicht mit einer Weißlichtquelle und einem für Cyanine 3 geeigneten Filtersatz. Die Signale wurden mit einer CCD-Kamera (PCO-Sensicam, Kehlheim, Deutschland) aufgezeichnet. Die Spaltbreite bezeichnet im Folgenden den Abstand zwischen Mikroarray und Detektionsebene.

a) Messung des Fluoreszenzsignals des Analyten in Abhängigkeit von der Spaltbreite

Es wurden Kanalmasken mit definierter Kanaltiefe (5 $\mu$m, 10 $\mu$m, 28 $\mu$m) aus Sylgard abgegossen. Die Kanäle wiesen eine Breite von 125 $\mu$m auf. Ein Glaschip wurde über die unterschiedlich tiefen Kanäle gelegt. Die Kanäle wurden dann mit einer 200 nM-Lösung eines Cy3-markierten Oligonukleotidesin 2 x SSC + 0,2 %SDS- befüllt und das Signal bei einer Belichtungszeit von 1,5 s gemessen.

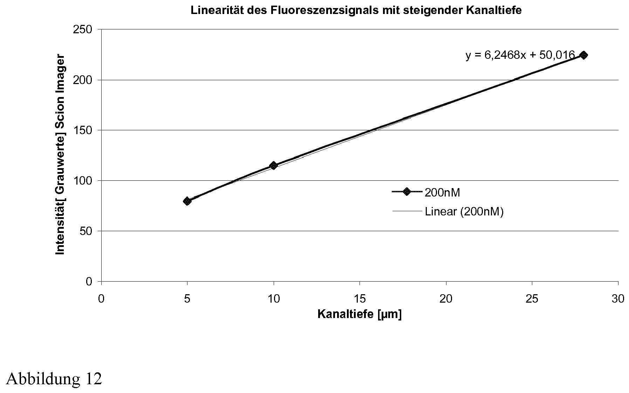

In Abbildung 12 sind die Messergebnisse dargestellt. Mit steigender Kanaltiefe steigt das Signal linear an. Es konnte eine Regressionsgerade errechnet werden (Gleichung 1)

$$(\text{Gleichung } 1) \qquad F(x) = 6{,}2468x + 50{,}016$$

Mit Hilfe der erhaltenen Regressionsgleichung (Gleichung 1) können nun die Schichtdicken zwischen DNA-Chip und Detektionsfläche anhand des Hintergrundfluoreszenzsignals bestimmt werden.

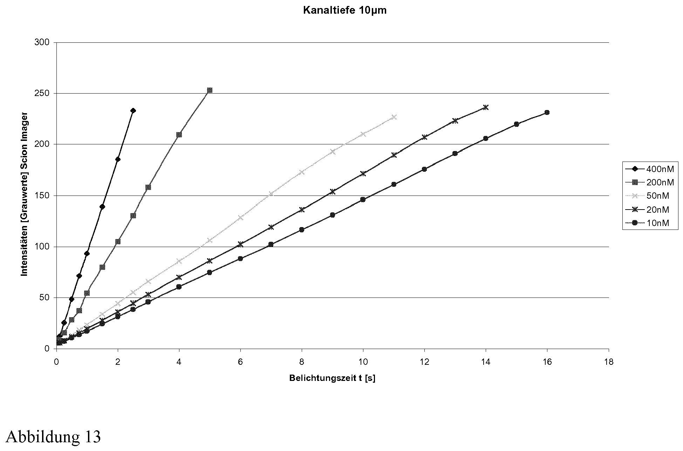

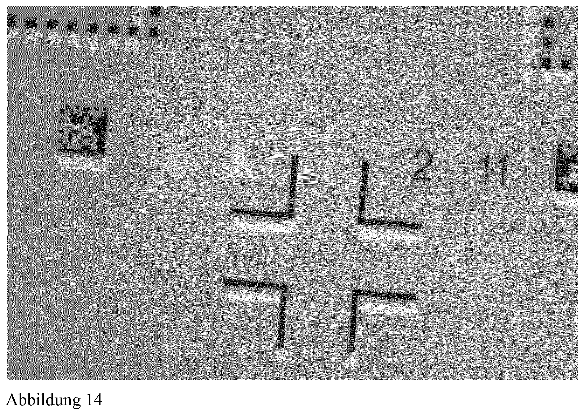

Dies wurde überprüft, indem zwei Glasflächen (Chips) aufeinander gelegt wurden, die auf ihrer Oberseite strukturierte Marken trugen (Kreuze, Zahlen und Datamatrix in Abbildung 14), auf welche fokussiert werden konnte. Die Chips wurden so aufeinander gelegt, dass die strukturierten Marken zueinander orientiert und nur durch eine dünne Flüssigkeitsschicht voneinander getrennt waren. Als Flüssigkeit wurde eine 200 nM-Lösung eines Cy3-markierten Oligonukleotides in 2 x SSC + 0,2 %SDS verwendet. Mit Hilfe der mit einer Skalierung versehenen Fokussierungseinrichtung des Mikroskops konnten der Abstand zwischen den Marken und damit die Schichtdicke des Flüssigkeitfilms direkt bestimmt werden. Die Intensität des Hintergrundes beträgt 158 Grauwerte bei einer Belichtungszeit von 0,75 s. Die am Fluoreszenzmikroskop gemessene Spaltbreite beträgt 40 $\mu$m. Unter der Annahme, dass sich die Messgrauwerte linear zur Belichtungszeit verhalten (siehe Abbildung 13), erhält man mit Gleichung 1 eine Spaltbreite von 42,6 $\mu$m. Die so ermittelten Werte für die Dicke der Flüssigkeitsschicht stimmen gut überein.

b) Experimente zur Verringerung beziehungsweise Eliminierung der Hintergrundfluoreszenz durch Komprimierung der Prozesseinheit

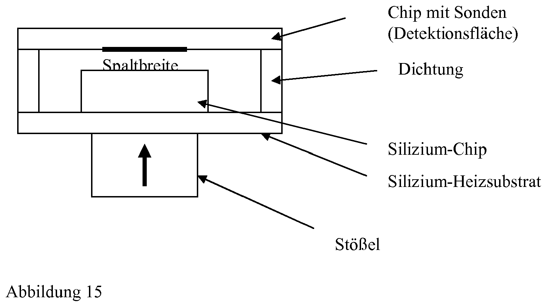

Bei diesen Experimenten wurde das Hybridisierungssignal in Abhängigkeit der Verdrängung des fluoreszierenden Analyten durch Andrücken eines Stößels gemessen. Der experimentelle Aufbau ist in Abbildung 15 skizziert. Durch das Andrücken des Stößels wurde der Silizium-Chip (3,15 x 3,15 mm) gegen einen Sonden-Chip (DNA-Chip) gedrückt und dabei die zwischen den beiden Flächen liegende Flüssigkeit verdrängt.

Zur Durchführung des Experimentes wurde die Kammer mit einer Hybridisierungslösung befüllt, die ein Modellsystem für die Verhälnisse bei einer PCR-Hybridisierung darstellt. Die Hybridisierungslösung enthielt ein Cy3-markiertes Oligonukleotid (Endkonzentration 2 nM in 2 x SSC + 0,2 % SDS), welches Komplementarität zum Sonden-Array aufwies. Zusätzlich enthielt die Hybridisierungslösung ein ebenfalls Cy3-markiertes Oligonukleotid, welches nicht mit dem Sondenarray hybridisiert und daher lediglich zum FluoreszenzHintergrundsignal in der Lösung, nicht aber zu den spezifischen Signalen an den Spots beiträgt.

Die Hybridisierung erfolgte für 10 min. Beim anschließenden Auslesen der Hybridisierungssignale wurde eine feste Belichtungszeit von 1,5 s gewählt. Am Versuchsaufbau wurde zwischen jeder Aufnahme der Stößel weiter an den Sonden-Array (Detektionsfläche) gedrückt, so dass sich der mit Hybridisierungslösung gefüllte Spalt zwischen Array und 2. Fläche verringerte.

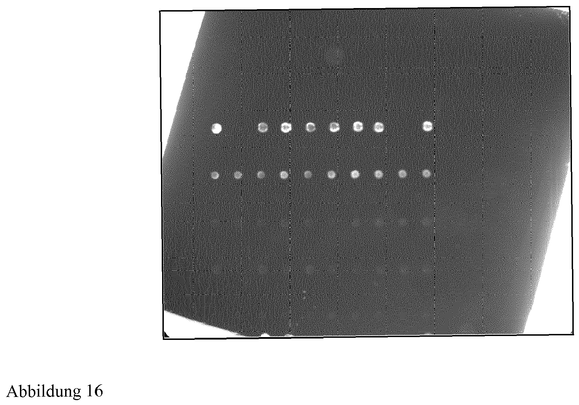

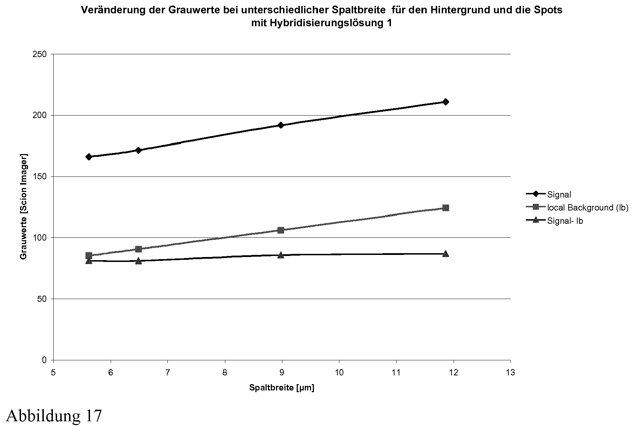

Abbildung 16 zeigt eine Aufnahme des Hybridisierungssignals bei einer Spaltbreite von 10 $\mu$m. Die Messergebnisse für Hintergrundsignal und Hybridisierungssignal an den Spots sind in Abbildung 17 dargestellt. Beide Signale verhalten sich erwartungsgemäß linear zur Spaltbreite. Daher verändert sich das um den Hintergrund korrigierte Spotsignal nicht mit der Spaltbreite.

Bei Erreichen eines Grauwertes von 255 ist das Messinstrument übersteuert. Das heißt, mit einer Spaltbreite von etwa 17 $\mu$m ist eine Messung der Spotintensität nur durch Verringerung der Belichtungszeit möglich. Damit sinkt dann die Messempfindlichkeit.

Durch Verringerung der Spaltbreite erhöht sich somit der dynamische Meßbereich. Durch Hintergrundbereinigung der Spotsignale (Differenzbildung) kann die Spaltbreite über einen weiten Bereich ohne eine Beeinträchtigung der Messung und Messergebnisse.variiert werden Bei sehr großen Spaltbreiten (>20 $\mu$m) wird die Messung durch Übersteuerung des Detektors stark beeinträchtigt.

c) Amplifikation, Hybridisierung und Detektion als Eintopfreaktion

Es wurden zwei Prozesseinheiten mit einem Aufbau entsprechend Abbildung 15 montiert und durchnumeriert.
Es wurden zwei identische Reaktionsansätze mit folgender Zusammensetzung hergestellt:

|  | Reaktionsansatz: |  |
| --- | --- | --- |
| 20mM dNTPs | | 0,5 $\mu$l |
| 1 M Kaliumacetat (Kaac) | | 3 $\mu$l |
| 25mM Mg-acetat Eppendorf | | 5 $\mu$l |
| Clontech C-DNA PCR Puffer | | 5 $\mu$l |
| Eppendorf Taq-Polymerase | | 3 $\mu$l |
| 10$\mu$M Primer CMV_DP_Cy3 | | 1 $\mu$l |
| Cy3_5'TGAGGCTGGGAARCTGACA3' 10$\mu$M Primer CMV_UP_NH2 | | 0,66 $\mu$l |
| 5'GGGYGAGGAYAACGAAATC3'_NH2 10$\mu$M Primer CMV_UP | | 0,33 $\mu$l |
| 5'GGGYGAGGAYAACGAAATC3' 10$\mu$M Primer Entero_DP_Cy3 | | 1 $\mu$l |
| Cy3_5'CCCTGAATGCGGCTAAT3 ' 10$\mu$M Primer Entero_UP_NH2 | | 0,66 $\mu$l |
| 5'ATTGTCACCATAAGCAGCC3'_NH2 10$\mu$M Primer Entero_UP | | 0,33 $\mu$l |
| 5'ATTGTCACCATAAGCAGCC3' 10$\mu$M Prime HSV1_DP_Cy3 | | 1 $\mu$l |
| Cy3_5'CTCGTAAAATGGCCCCTCC3' 10$\mu$M Primer HSV1_UP_NH2 | | 0,66 $\mu$l |
| 5'CGGCCGTGTGACACTATCG3'_NH2 10$\mu$M Primer HSV1_UP | | 0,33 $\mu$l |
| 5'CGGCCGTGTGACACTATCG 10$\mu$M Primer HSV2_UP_Cy3 | | 1 $\mu$l |
| Cy3_5'CGCTCTCGTAAATGCTTCCCT3 ' 10$\mu$M Primer HSV2_DP_NH2 | | 0,66 $\mu$l |
| 5'TCTACCCACAACAGACCCACG3'_NH2 10$\mu$M Primer HSV2_DP | | 0,33 $\mu$l |
| 5 'TCTACCCACAACAGACCCACG3' 10$\mu$M Primer VZV_DP_Cy3 | | 1 $\mu$l |
| Cy3_5'TCGCGTGCTGCGGC 10$\mu$M Primer VZV_UP_NH2 | | 0,66 $\mu$l |
| 5'CGGCATGGCCCGTCTAT3'_NH2 10$\mu$M Primer VZV_UP | | 0,33 $\mu$l |

(fortgesetzt)

| | |
|---|---|
| 5'CGGCATGGCCCGTCTAT Template CMV | 1 µl |
| PCR-Grade Water | 22,5 µl |
| total | 50 µl |

Die Prozesseinheiten wurden mit je 50 µl Reaktionsansatz befüllt. und nach folgendem Temperatur-Zeit-Regime prozessiert.

| | | |
|---|---|---|
| 1 Denaturieren | 95 °C | |
| Dauer | 300 s | |
| 2 Denaturieren | 95 °C | |
| Dauer | 10 s | |
| 3 Annealing/Extension | 60 °C | |
| Dauer | 20 s | |

Wiederholung von Schritt 2 bis 3 35 mal

| | |
|---|---|
| 4 Denaturierung | 95 °C |
| Dauer | 300 s |
| 5 Hybridisierung | 40 °C |
| Dauer | 3600 s |

Anschließend wurden die beiden Prozesseinheiten unterschiedlichen Behandlungen unterzogen. Im ersten Fall (Prozesseinheit 1) wurde die Hintergrundfluoreszenz durch Verdrängung des Analyten verringert. Dies wurde gewährleistet, indem der Stößel nach oben in Richtung der Detektionsfläche gedrückt wurde, so dass sich der mit der Reaktionslösung gefüllte Spalt weitestgehend verkleinert.

Im zweiten Fall (Prozesseinheit 2) wurde der Analyt gegen eine nicht fluoreszierende Lösung ausgetauscht. Der Austausch der Lösung erfolgte mit 2 x SSC-Puffer bei einer Flussrate von 300 µl/min und einem Spülvolumen von 900 µl. Diese Vorgehensweise entspricht dem Stand der Technik.

Anschließend wurden die beiden Strategien zur Verringerung der Hintergrundfluoreszenz miteinander verglichen. Dazu wurden die Hybridisierungssignale in beiden Prozesseinheiten mit Hilfe des beschriebenen Fluoreszenzmikroskop-Kamera-Aufbaus detektiert.

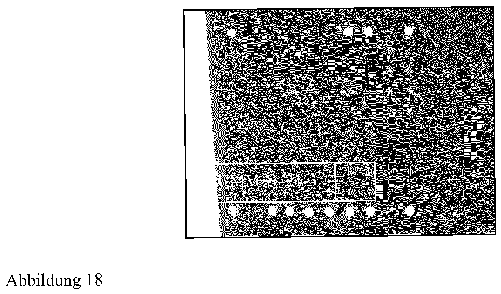

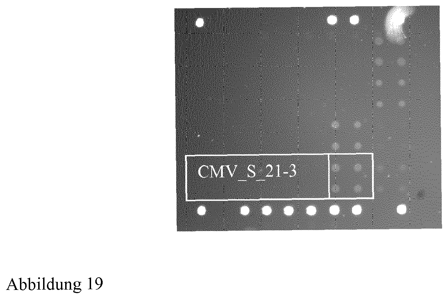

Die Belichtungszeit betrug 5 s (siehe Abbildung 18 und Abbildung 19). Der Vergleich der Spotintensitäten erfolgte anhand des Spots mit der Substanz CMV_S_21-3 (5'-NH$_2$TGTTGGGCAACCACCGCACTG-3'). Der Ort der Sonden ist in den Abbildungen 18 und 19 gekennzeichnet.

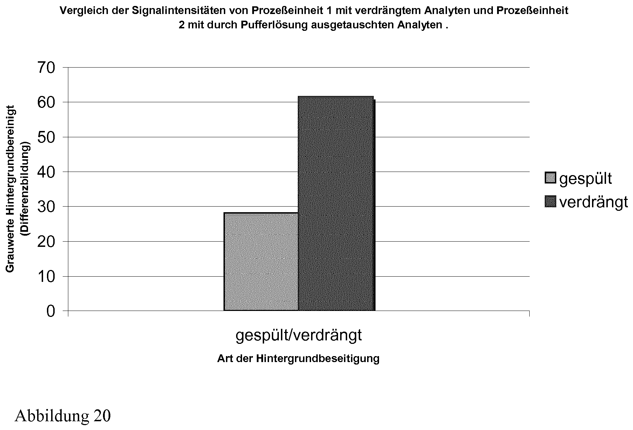

In Abbildung 20 ist das Ergebnis des Experimentes zusammengefasst. Durch die Spülung der Reaktionskammer in der Prozesseinheit 2 verringert sich das Hybridisierungssignal gegenüber der Verdrängung in Prozesseinheit 1. Es wird angenommen, dass eine Ausblutung der Sonden dafür verantwortlich ist.

Die Methode der Analytverdrängung durch das erfindungsgemäße Verfahren ist somit dem Austausch der Lösungen vorzuziehen.

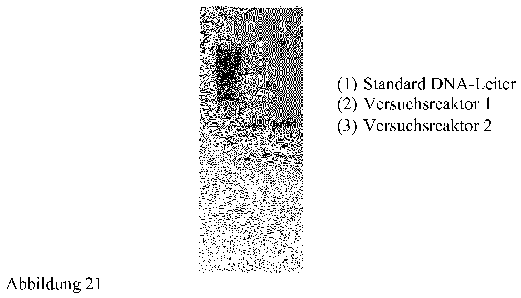

Um einen Nachweis über Menge und Integrität des Amplifikationsproduktes zu erhalten, wurden zusätzlich 5 µl jeder Reaktionslösung auf einem 2 %-igen Agarosegel analysiert. Das Ergebnis (Ethidiumbromid-gefärbtes Gel detektiert auf eine UV Transilluminator) ist in Abbildung 21 zu sehen.

Beispiel 4: Gerät zur Prozessierung und Detektion von erfindungsgemäßen Reaktionskartuschen

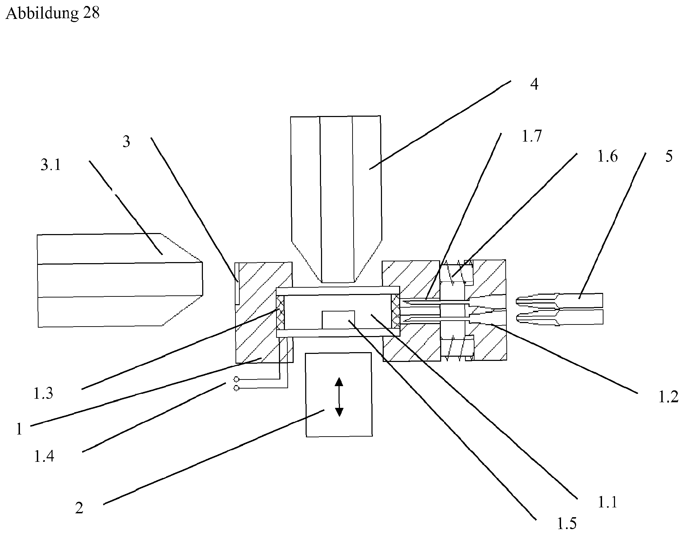

Ein Gerät zur Prozessierung und Detektion von erfindungsgemäßen Reaktionskartuschen gemäß diesem Ausführungsbeispiel ist in Abbildung 28 gezeigt. Das Gerät zur Durchführung von Mikroarray-basierten Tests mit erfindungsgemäßen Reaktionskartuschen besteht üblicherweise aus mehreren Komponenten, welche in einem Gerät vereint, aber auch modular aus mehreren Teilgeräten zusammengestellt sein können. Die Vorrichtung kann dabei wahlweise über einen integrierten Rechner oder eine Schnittstelle zu einem externen Rechner angesteuert werden. Der Aufbau der Vorrichtung ist in Abbildung 28 veranschaulicht.

Ein beispielhafter Ablauf findet wie folgt statt:

Das Fluid-Interface der Reaktionskartusche wird vom Anwender manuell in die Befüllstellung gebracht, bei der die Kanülen die Dichtung des Kammerkörpers durchstechen. Anschließend füllt der Anwender das Reaktionsgemisch mit Hilfe einer Standard-Laborpipette in die Reaktionskammer ein. Beide Schritte können auch von einer entsprechend ausgeführten Vorrichtung übernommen werden. Das Fluidinterface wird nun wieder in die Ausgangsstellung bewegt, wobei auch dieser Vorgang von einer entsprechend ausgeführten Vorrichtung durchgeführt werden kann.

Die Reaktionskartusche wird daraufhin in das Gerät eingelegt. Ein in der Vorrichtung angeordneter Datamatrix-Reader erkennt die auf der Reaktionskartusche angebrachte eineindeutige Datamatrix und lädt anhand eines vom Nutzer übermittelten Datensatzes die Kenndaten für die Kartusche sowie für den durchzuführenden Test in den Steuerrechner. Dieser steuert dann die einzelnen Prozessschritte, welche beispielsweise eine Amplifikation und Hybridisierung umfassen können. Über den integrierten Drückmechanismus wird anschließend zur Detektion der Kapillarspalt in der Reaktionskammer erfindungsgemäß verringert.

Die Detektion kann mit herkömmlichen fluoreszenzoptischen bild- oder nichtbildgebenden Systemen erfolgen. Die gewonnenen Daten werden anschließend an einen Steuerrechner übermittelt, welcher diese auswertet und auf einer internen oder externen Schnittstelle präsentiert oder speichert.

Anschließend kann die Reaktionskartusche durch den Anwender aus dem Gerät entnommen und entsorgt werden.

Beispiel 5: Reaktionskartusche aus elektrisch leitfähigem Kunststoff

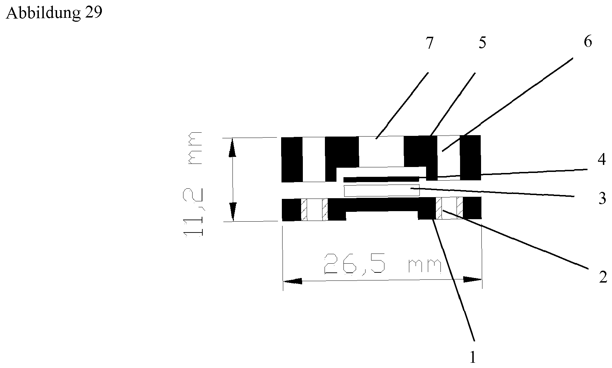

Eine wie in Abbildung 29 dargestellte Reaktionskartusche wird hergestellt.

Die untere Halbschale (1) der Reaktionskartusche besteht aus elektrisch leitfähigem Kunststoff als Boden der Reaktionskammer (Conduct 2, RKT, Deutschland). Auf der Unterseite des Kammerbodens wird ein Folien-Pt100 Temperatursensor mit Hilfe eines geeigneten Klebers, z.B. Loctite 401 (Loctite, Deutschland) fixiert. Die untere Halbschale bildet gemeinsam mit der Dichtung (3) und dem Deckglas (4) die Reaktionskammer der erfindungsgemäßen Kartusche.

Die Kartusche weist ferner eine Gewindebohrung (2) zum Einsetzen von Schrauben zur elektrischen Kontaktierung, eine obere Halbschale (5) der Reaktionskartusche, z.B. aus Acryl, eine Bohrung (6) zur Befestigung der oberen Halbschale sowie ein Detektionsfenster (7) in der oberen Halbschale auf.

Ein Standard-PCR-Reaktionsmix wird hergestellt:

30,5 $\mu$l deionisiertes Wasser
5 $\mu$l 10xPCR-Puffer (z.B. 10 x cDNA PCR Reaction Buffer, Clontech, Deutschland)
5 $\mu$l Mg-Acetat, 25 mM (z.B. Eppendorf, Deutschland)
0,5 $\mu$l dNTP, 20 mM each
1 $\mu$l 16sfD1 (5'-AGAGTTTGATCCTGGCTCAG-3'), 10 mM
1 $\mu$l 16sRa (5'-TACCGTCACCATAAGGCTTCGTCCCTA-3'), 10 mM
3 $\mu$l Taq DNA Polymerase (z.B. Genaxxon, Deutschland)
1 $\mu$l Template

Mit Hilfe einer Insulinspritze (Becton Dickinson, Deutschland) wird die Reaktionskammer mit dem Reaktionsgemisch gefüllt. Zum Entlüften während des Befüllvorganges wird eine zweite Kanüle durch die Dichtung des Kammerkörpers gestochen. Nach der Befüllung werden Entlüftungskanüle und Insulinspritze fachgerecht entsorgt.

Die Kammer wird anschließend über die beiden dafür vorgesehenen Schrauben an einer Regeleinheit angeschlossen (CLONDIAG chip technologies GmbH, Deutschland) Ebenso wird der Temperatursensor auf der Unterseite der unteren Halbschale an diese Regeleinheit angeschlossen. Diese Regeleinheit ist in der Lage, nach einem vorgegebenen Programm bestimmte Temperaturen in der unteren Halbschale zu regeln.

Auf diese Weise wird das nachfolgende PCR-Programm durchgeführt: 5 min 95°C, 30 x (30 s 95°C, 30 s 62°C, 50 s 72°C).

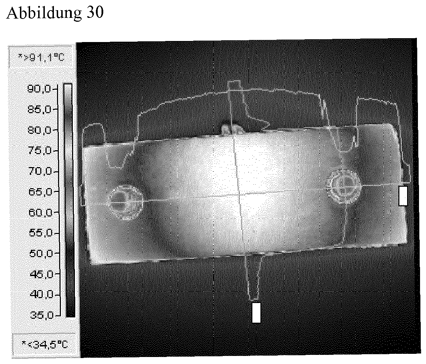

Abbildung 30 zeigt eine Aufnahme der Reaktionskartusche mit einer Wärmebildkamera bei einer Temperatur von 95°C.

Nach Abschluss des Programms wird das Reaktionsprodukt mit Hilfe einer Insulinspritze aus der Reaktionskammer entfernt. Zur Belüftung während der Leerung der Reaktionskammer wird analog zur Befüllung eine Kanüle durch die Dichtung des Kammerkörpers gestochen.

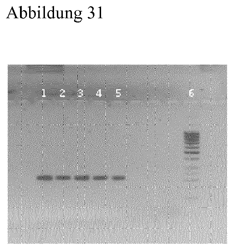

Das Reaktionsprodukt wird nun durch Agarosegel-Elektrophorese analysiert. Dazu werden 5$\mu$l der Reaktionslösung mit einem geeigneten Puffer (z.B. 5 $\mu$l 250 mM in 50% Glycerin, Bromphenolblau) in die Tasche eines 2% Agarosegels aufgetragen und eine Elektrophorese durchgeführt. Das Resultat ist in Abbildung 31 dargestellt.

Wie deutlich zu erkennen ist, konnte in allen Fällen ein Amplifikationsprodukt der korrekten Größe und in zur Positivkontrolle vergleichbarer Menge erhalten werden.

Abbildungen

Abbildung 1:
Übersicht über die erfindungsgemäße Vorrichtung umfassend eine Auslesegerät und die Prozesseinheit.

Abbildung 2:
Darstellung der erfindungsgemäßen Prozesseinheit.

Abbildung 3:
Explosionszeichnung der erfindungsgemäßen Prozesseinheit umfassend die Detektionsfläche, Dichtung, DNA-Chip und Kammerkörper. Der Kammerkörper weist eine reversibel deformierbare elastische Membran auf.

Abbildung 4:
Darstellung des Kammerkörpers mit kunstoffumspritzten Heizmeander in der elastischen Membran.

Abbildung 5:
Darstellung des Zustandes der erfindungsgemäßen Prozesseinheit im Auslesegerät A) während der PCR, B) vor der Detektion, und C) während der Detektion.

Abbildung 6:
Darstellung der Funktionsweise der erfindungsgemäßen Prozesseinheit mit Membrandichtung, Ausgleichsfalte und Unterseitenloch. In A) ist die Prozesseinheit in der Normalstellung zu sehen. In B) ist die Prozesseinheit in der komprimierten Form zu sehen, bei der die fluoreszierende Lösung zwischen DNA-Chip und Detektionsfläche verdrängt ist.

Abbildung 7:
Darstellung eines Drehtellers, auf dem vier Temperaturblöcke installiert sind. Die Temperaturblöcke sind auf jeweils eine Temperatur thermostatisiert. Durch Drehbewegung des Tellers und/oder der Prozesseinheit lässt sich die Temperatur in der Reaktionskammer wechseln.

Abbildung 8:
Abbildung eines Ausführungsbeispiels einer gefrästen und verschraubten Prozesseinheit.

Abbildung 9: Abbildung eines Ausführungsbeispiels einer Komprimierungs- bzw. Verquetschungsapparatur für die erfindungsgemäße Prozesseinheit zur Detektion der Hybridisierungssignale in einem konventionellen Fluoreszenzmikroskop.

Abbildung 10:
Darstellung einer erfindungsgemäßen Prozesseinheit mit einer Platine als elektrischem Anschluss für Heizer und Temperatursensor. Der Heizer ist als Halbleiterbauteil ausgeführt.

Abbildung 11:
Explosionzeichnung der in Abbildung 10 gezeigten Prozesseinheit.

Abbildung 12:
Darstellung der Regressionsgeraden zur Ermittlung der Breite eines mit Fluorophor befüllten Spaltes.

Abbildung 13:
Darstellung des linearen Verlaufs des Fluoreszenzsignals mit steigender Belichtungszeit über den gemessenen Bereich.

Abbildung 14:
Fluoreszenz-Aufnahme zweier übereinander gelegter, im Zwischenraum mit 200 nM Cy3-Fluorophor befüllter Chips. Die Intensität des Hintergrundes beträgt 158 Grauwerte bei einer Belichtungszeit von 0,75 s. Die am Fluoreszenzmikroskop gemessene Spaltbreite beträgt 40,00 $\mu$m. Unter der Annahme, dass sich die Messgrauwerte linear zur Belichtungszeit verhalten (siehe Abbildung 13), erhält man mit Gleichung 1 eine Spaltbreite von 42,6 $\mu$m. Die so ermittelten Schichtdickenwerte stimmen gut überein.

Abbildung 15:
Darstellung des Versuchsaufbaus zur spülungsfreien Detektion von DNA-Arrays.

Abbildung 16:
Fluoreszenzaufnahme eines Arrays mit angedrücktem Chip. An den weißen Rändern ist die Hintergrundstrahlung durch die verdrängte Probenlösung zu erkennen.

Abbildung 17:
Abnahme der absoluten Intensitäten von Signal und Hintergrund bei Verringerung der Spaltbreite. Die Differenz beider Werte bleibt über den Messbereich konstant.

Abbildung 18.
Detektion der Sondensignale durch Verdrängung der Hintergrundfluoreszenz. Am linken Rand ist die nicht verdrängte Flüssigkeit zu erkennen.

Abbildung 19:
Detektion der Sondensignale eines durch Spülung hintergrundbereinigten DNA-Arrays.

Abbildung 20:
Zusammenfassung der Messergebnisse zum experimentellen Vergleich zwischen dem Verdrängen und dem Austausch des Analyten.

Abbildung 21:
Referenzanalytik der PCR in einer Prozesseinheit mittels Gelelektrophorese.

Abbildung 22:
Schematische Darstellung einer abnehmbaren Befülleinheit zur Befüllung von Reaktionskartuschen mit reaktiven Substanzen oder Puffern. Hierfür werden folgende Bezugszeichen verwendet:

| | |
|---|---|
| 1 | Befülleinheit |
| 1.1 | mechanisches Interface Befülleinheit- Kartusche |
| 2 | Kartusche |
| 2.1 | mechanisches Interface Kartusche-Befülleinheit |
| 2.2 | Dichtung |
| 2.3 | Reaktionskammer |
| 2.4 | Bevorzugte Öffnung für die Kanülen in der Kartusche |
| 3 | Befüllungskanal |
| 3.1 | Fluidisches und mechanisches Interface zu probenbeaufschlagenden Werkzeugen |
| 3.2 | Befüllungskanüle |
| 4 | Abfallkanal mit Abfallbehälter |
| 4.1 | Entlüftungsloch |
| 4.2 | Abfallkanüle |

Abbildung 23:
Darstellung des Ablaufs zur Befüllung einer Reaktionskartusche mit einer modularen Befülleinheit.

Abbildung 24:
Schematische Darstellung einer integrierten Befülleinheit zur Befüllung von Reaktionskartuschen mit reaktiven Substanzen oder Puffern in der Vorzugsstellung ohne Penetration der Dichtung des Kammerkörpers. Hierfür werden folgende Bezugszeichen verwendet:

| | |
|---|---|
| 1 | Befülleinheit- Kartusche |
| 1.1 | mechanisches Interface Kartusche-Befülleinheit |
| 2 | Reaktionskartusche |

2.1 mechanisches Interface Kartusche-Befülleinheit
2.2 Dichtung
2.3 Reaktionsraum
2.4 Bevorzugte Öffnung für die Kanülen im Kartuschengehäuse-Gehäuse
3 Befüllungskanal
3.1 Fluidisches und mechanisches Interface zu probenzuführenden Werkzeugen
3.2 Befüllungskanüle
4 Abfallkanal mit Abfallbehälter
4.1 Fluidisches und mechanisches Interface zu probenabführenden Einheiten
4.2 Abfallkanüle
5 Einrichtung für Vorzugsstellung, hier Feder

Abbildung 25:
Darstellung des Ablaufs zur Befüllung einer Reaktionskartusche mit einer integrierten Befülleinheit.

Abbildung 26:
Schematische Darstellung einer integrierten Befülleinheit mit integriertem Abfallbehälter zur Befüllung von Reaktionskartuschen mit reaktiven Substanzen oder Puffern in der Vorzugsstellung ohne Penetration der Dichtung des Kammerkörpers. Hierfür werden zusätzlich zu den Bezugszeichen aus Abbildung 24 folgende Bezugszeichen verwendet:

4 Abfallkanal mit Abfallbehälter
4.1 Entlüftungsloch

Abbildung 27:

a) Befüllung des Reaktionsraumes bei Abführung der überschüssigen Flüssigkeit in einen Abfallbehälter oder -kanal
b) Abführung überschüssiger Flüssigkeit bei der Verringerung des Reaktionsraumes zur Detektion

Hierbei werden folgende Bezugszeichen verwendet:

1 Reaktionskammer
2 Dichtung
3 Drückmechanismus
4 Fluidinterface
4.1 abführende Kanüle
4.2 zuführende Kanüle

Abbildung 28:
Gerät zur Prozessierung und Detektion von erfindungsgemäßen Reaktionskartuschen gemäß dem Ausführungsbeispiel 4. Hierbei werden folgende Bezugszeichen verwendet:

1 Reaktionkartusche
1.1 Reaktionskammer mit Micro-Array
1.2 Fluidsystem-Interface
1.3 Dichtung des Kammerkörpers
1.4 Elektrische Anschlüsse für Heizsystem, evtl. auch Temperatursensoren
1.5 Chip
1.6 Lagesicherungssystem zur Realisierung einer Vorzugslage und Führung der Kanülen
1.7 Kanülen
2 Drückmechanismus
3 Identifikationssystem, z.B. Barcode oder Datamatrix
3.1 Identifikationsoptik, z.B. Barcode- oder Datamatrix-Reader
4 Detektionsoptik
5 Fluidanschlüsse

Abbildung 29:

Reaktionskartusche gemäß Ausführungsbeispiel 5.

Abbildung 30:
Aufnahme der Reaktionskartusche gemäß Ausführungsbeispiel 5 mit einer Wärmebildkamera bei einer Temperatur von 95°C.

Abbildung 31:
Analyse des Reaktionsprodukts gemäß Ausführungsbeispiel 5 durch Agarosegel-Elektrophorese. Dabei bedeuten:

   1, 5: Positivkontrolle aus dem Thermocycler
   2-4: Reaktionsprodukten aus Kartuschen
   6: 100bp Standard

Einige Ausführungsformen der Erfindung betreffen:

1. Vorrichtung zum qualitativen und/oder quantitativen Nachweis von molekularen Wechselwirkungen zwischen Sonden- und Targetmolekülen, enthaltend:

   a) einen Mikroarray mit einem Substrat, auf dem auf Array-Elementen Sondenmoleküle immobilisiert sind, wobei der Mikroarray auf einer ersten Fläche der Vorrichtung angeordnet ist; und
   b) eine Reaktionskammer, die zwischen der ersten Fläche mit dem darauf angeordneten Mikroarray und einer zweiten Fläche gebildet ist,

wobei der Abstand zwischen dem Mikroarray und der zweiten Fläche veränderbar ist.

2. Vorrichtung nach Ausführungsform 1,
wobei der Abstand zwischen dem Mikroarray und der zweiten Fläche in einem Bereich von etwa 0 bis etwa 1 mm veränderbar ist

3. Vorrichtung nach Ausführungsform 1 oder 2,
wobei die Vorrichtung zusätzlich eine Temperatursteuerungs- und/oder -regeleinheit zur Steuerung und/oder Regelung der Temperatur in der Reaktionskammer umfasst.

4. Vorrichtung nach Ausführungsform 3,
wobei die Temperatursteuerungs- und -regeleinheit in die erste Fläche integriert ist.

5. Vorrichtung nach Ausführungsform 3,
wobei die Vorrichtung Temperatursteuerungs- und regeleinheit Temperaturblöcke umfasst, die jeweils auf eine definierte Temperatur vorgeheizt sind.

6. Vorrichtung nach Ausführungsform 5,
wobei die Temperaturblöcke linear oder auf einem Drehteller angeordnet sind.

7. Vorrichtung nach einer der vorhergehenden Ausführungsformen,
wobei die Vorrichtung ein Detektionssystem umfasst.

8. Vorrichtung nach Ausführungsform 7,
wobei das Detektionssystem ein optisches System, vorzugsweise ein fluoreszenzoptisches System ist.

9. Vorrichtung nach Ausführungsform 8,
wobei das fluoreszenzoptische System ein Fluoreszenzmikroskop ohne Autofokus ist.

10. Vorrichtung nach einer der Ausführungsformen 7 bis 9,
wobei das Detektionssystem mit einem Abstandshalter verbunden ist, der bei Auflage auf der zweiten Fläche einen Abstand zwischen dem Detektionssystem und der zweiten Fläche einstellt.

11. Vorrichtung nach einer der vorhergehenden Ausführungsformen,
wobei für den zwischen der ersten und zweiten Fläche gebildeten Reaktionsraum seitlich begrenzende Ausgleichsbereiche vorgesehen sind, die bei Verringerung des Abstands zwischen Mikroarray und zweiter Fläche das Volumen in der Reaktionskammer im Wesentlichen konstant halten.

12. Vorrichtung nach einer der vorhergehenden Ausführungsformen,
wobei die zweite Fläche aus einem optisch durchlässigen Material, vorzugsweise Glas ausgestaltet ist.

13. Vorrichtung nach einer der vorhergehenden Ausführungsformen,
wobei der zwischen der ersten und zweiten Fläche gebildete Reaktionsraum seitlich durch elastische Dichtungen begrenzt ist.

14. Vorrichtung nach einer der vorhergehenden Ausführungsformen,
wobei die erste Fläche zumindest im Bereich unterhalb des Mikroarrays derart ausgestaltet ist, dass der Mikroarray relativ zur zweiten Fläche so führbar ist, dass der Abstand zwischen dem Mikroarray und der zweiten Fläche veränderbar ist.

15. Vorrichtung nach Ausführungsform 14,
wobei die erste Fläche zumindest im Bereich unterhalb des Mikroarrays elastisch verformbar ist.

16. Vorrichtung nach Ausführungsform 15,
wobei die erste Fläche aus einem elastischen Kunststoff ausgestaltet ist.

17. Vorrichtung nach einer der Ausführungsformen 12 bis 14,
wobei die erste Fläche mittels zweier übereinander liegender Schichten ausgestaltet ist, wobei eine äußere Schicht der beiden übereinander liegenden Schichten zumindest im Bereich unterhalb des Mikroarrays eine Aussparung aufweist.

18. Vorrichtung nach Ausführungsform 17,
wobei eine innere der beiden übereinander liegenden Schichten aus einer elastischen Dichtung gebildet ist.

19. Vorrichtung nach einer der Ausführungsformen 14 bis 18,
wobei die Vorrichtung mindestens ein Mittel umfasst, mit dem der Mikroarray relativ zur zweiten Fläche führbar ist.

20. Vorrichtung nach Ausführungsform 19,
wobei der Mikroarray durch Druck und/oder Zug des Mittels auf die erste Fläche relativ zur zweiten Fläche führbar ist.

21. Vorrichtung nach Ausführungsform 19 oder 20,
wobei die erste Fläche durch das Mittel in Vibration versetzbar ist.

22. Vorrichtung nach einer der vorhergehenden Ausführungsformen,
wobei die zweite Fläche relativ zur ersten Fläche so führbar ist, dass der Abstand zwischen dem Mikroarray und der zweiten Fläche veränderbar ist.

23. Vorrichtung nach einer der Ausführungsformen 10 bis 22,
wobei die zweite Fläche durch Druck und/oder Zug des Abstandshalters auf die zweite Fläche relativ zur ersten Fläche so führbar ist, dass der Abstand zwischen dem Mikroarray und der zweiten Fläche veränderbar ist.

24. Vorrichtung nach einer der vorhergehenden Ausführungsformen,
wobei die erste Fläche und die zweite Fläche so führbar sind, dass der Abstand zwischen dem Mikroarray und der zweiten Fläche veränderbar ist.

25. Vorrichtung nach einer der vorangehenden Ausführungsformen,
wobei die Reaktionskammer ein Kapillarspalt zwischen dem Kammerträger und dem Mikroarray ist.

26. Vorrichtung nach Ausführungsform 25,
wobei der Kapillarspalt eine Dicke im Bereich von etwa 0 $\mu$m bis etwa 100 $\mu$m aufweist.

27. Vorrichtung nach einer der vorhergehenden Ausführungsformen,
wobei die Reaktionskammer mindestens zwei Unterkammern umfasst, wobei in einem ersten nicht komprimierten Zustand die Unterkammern fluidisch miteinander verbunden sind und in einem zweiten komprimierten Zustand keine fluidische Verbindung zwischen den Unterkammern besteht.

28. Vorrichtung nach Ausführungsform 27,
wobei jede Unterkammer einem definierten Bereich des Mikroarrays zugeordnet ist.

29. Vorrichtung nach Ausführungsform 27 oder 28,
wobei der Mikroarray und/oder die zweite Fläche mit Kavitäten versehen sind, die als Wände zwischen den Unterkammern dienen.

30. Vorrichtung nach einer der Ausführungsformen 27 bis 29,
wobei die Wände zwischen den Unterkammern durch elastische Dichtungen gebildet sind.

31. Vorrichtung nach einer der vorhergehenden Ausführungsformen,
wobei die Vorrichtung zusätzlich eine Befülleinheit und/oder Aufarbeitungseinheit zur Reinigung und/oder Aufkonzentration einer Probenlösung und/oder Steuerung des Be- und/oder Entladens der Reaktionskammer mit Fluiden umfasst.

32. Vorrichtung nach Ausführungsform 31,
wobei die Reaktionskammer und die Befüll- bzw. Aufarbeitungseinheit über zwei Kanülen miteinander verbunden sind, wobei die Kanülen so angeordnet sind, dass eine erste Kanüle die Zuführung von Fluiden aus der Befüll- bzw. Aufarbeitungseinheit in die Reaktionskammer gewährleistet und eine zweite Kanüle das Entweichen der durch die zugeführten Fluide aus der Reaktionskammer verdrängten Luft gewährleistet.

33. Vorrichtung nach einer der vorhergehenden Ausführungsformen, wobei die Vorrichtung eine mit dem Detektionssystem verbundene Einheit zur Verarbeitung von durch das Detektionssystem aufgenommenen Signalen umfasst.

34. Vorrichtung nach einer der vorhergehenden Ausführungsformen, wobei die Vorrichtung zusätzlich eine Schnittstelle für externe Rechner aufweist.

35. Vorrichtung nach einer der vorhergehenden Ausführungsformen, wobei die Vorrichtung mit einer Codierung, vorzugsweise einer Datenmatrix oder einem Barcode, versehen ist, die Informationen über die Substanzbibliothek und/oder die Durchführung der Vervielfältigungs- und/oder Nachweisreaktion enthält.

36. Vorrichtung nach einer der vorhergehenden Ausführungsformen, wobei die Sonden- und/oder Targetmoleküle Biopolymere sind, die ausgewählt sind aus der Gruppe bestehend aus Nukleinsäuren, Peptiden, Proteinen, Antigenen, Antikörpern, Kohlenhydraten und/oder deren Analoga und/oder Mischpolymeren der vorstehend genannten Biopolymere.

37. Vorrichtung nach Ausführungsform 36, wobei die Sonden- und/oder Targetmoleküle Nukleinsäuren und/oder Nukleinsäureanaloga sind.

38. Vorrichtung nach einer der vorhergehenden Ausführungsformen, wobei die Vorrichtung einen Kammerkörper aus elektrisch leitfähigem Material aufweist.

39. Vorrichtung nach Ausführungsform 38, wobei das elektrisch leitfähige Material elektrisch leitfähiger Kunststoff ist.

40. Vorrichtung nach Ausführungsform 39, wobei der elektrisch leitfähige Kunststoff ausgewählt ist aus der Gruppe bestehend aus Polyamid mit 5-30% Kohlefasern, Polycarbonat mit 5-30% Kohlefasern, Polyamid mit 2-20% rostfreien Stahlfasern und PPS mit 5-40% Kohlenstofffaser.

41. Verfahren zum qualitativen und/oder quantitativen Nachweis von molekularen Wechselwirkungen zwischen Sonden- und Targetmolekülen, umfassend die folgenden Schritte:

a) Einbringen einer Probe enthaltend Targetmoleküle in eine Reaktionskammer einer Vorrichtung nach einer der Ausführungsformen 1 bis 40;
b) Detektieren einer Wechselwirkung zwischen den Targetmolekülen und den auf dem Substrat immobilisierten Sondenmolekülen.

42. Verfahren nach Ausführungsform 41,
wobei der Abstand zwischen Mikroarray und zweiter Fläche vor dem Detektieren in einer Position gehalten wird, die die Wechselwirkung zwischen den Targetmolekülen und den auf dem Substrat immobilisierten Sondenmolekülen ermöglicht.

43. Verfahren nach Ausführungsform 41 oder 42,
wobei in Schritt b) der Abstand zwischen dem Mikroarray und der zweiten Fläche verändert, vorzugsweise verringert wird.

44. Verfahren nach Ausführungsform 43,
wobei in Schritt b) der Abstand zwischen dem Mikroarray und der zweiten Fläche so verändert wird, dass die Probenlösung zwischen dem Mikroarray und der zweiten Fläche im Wesentlichen entfernt ist.

45. Verfahren nach einer der Ausführungsformen 41 bis 44,
wobei die Targetmoleküle mit einem detektierbaren Marker versehen werden.

46. Verfahren nach Ausführungsform 45,
wobei der detektierbare Marker ein Fluoreszenzmarker ist.

47. Verfahren nach Ausführungsform 46,
wobei das Detektieren der Fluoreszenzmarker mittels eines Fluoreszenzmikroskops ohne Autofokus erfolgt.

48. Verfahren nach Ausführungsform 47,
wobei das Detektieren der Fluoreszenzmarker mittels eines Fluoreszenzmikroskops mit Fixfokus erfolgt.

49. Verfahren nach einer der Ausführungsformen 41 bis 48,
wobei die Sonden- und/oder Targetmoleküle Nukleinsäuren und/oder Nukleinsäureanaloga sind.

50. Verfahren nach Ausführungsform 49,
wobei die Targetmoleküle in der Reaktionskammer mittels einer zyklischen Amplifikationsreaktion amplifiziert werden.

51. Verfahren nach Ausführungsform 50,
wobei das Detektieren nach einem oder mehreren Zyklen während der zyklischen Amplifikationsreaktion und/oder nach Abschluss der zyklischen Amplifikationsreaktion erfolgt.

52. Verwendung einer Vorrichtung nach einer der Ausführungsformen 1 bis 40 zur Durchführung von Mikroarray-basierten Tests.

53. Verfahren zum qualitativen und/oder quantitativen Nachweis von molekularen Wechselwirkungen zwischen Sonden- und Targetmolekülen, umfassend die folgenden Schritte:

a) Einbringen einer Probe enthaltend Targetmoleküle in eine Reaktionskammer, die einen Mikroarray aufweist, wobei der Mikroarray ein Substrat mit darauf auf Array-Elementen immobilisierten Sondenmoleküle umfasst;
b) Detektieren einer Wechselwirkung zwischen den Targetmolekülen und den auf dem Substrat immobilisierten Sondenmolekülen,

wobei zwischen dem Einbringen der Probe enthaltend Targetmoleküle in die Reaktionskammer und dem Detektieren kein Austauschen von Lösungen in der Reaktionskammer und/oder Entfernen von Lösungen aus der Reaktionskammer erfolgt.

54. Verfahren nach Ausführungsform 53, wobei das Verfahren ferner wie in einer der Ausführungsformen 41 bis 51 beschrieben ausgeführt wird.

**Patentansprüche**

1. Verfahren zum qualitativen und/oder quantitativen Nachweis von molekularen Wechselwirkungen zwischen Sonden- und Targetmolekülen, wobei die Sonden- und Targetmoleküle Nukleinsäuren und/oder Nukleinsäureanaloga sind, umfassend die folgenden Schritte:

   a) Einbringen einer Probe enthaltend Targetmoleküle in eine Reaktionskammer (1.1), die zwischen einer ersten Fläche mit einem darauf angeordneten Mikroarray (1.5) und einer zweiten Fläche gebildet ist, wobei der Mikroarray (1.5) ein Substrat mit darauf auf Array-Elementen immobilisierten Sondenmolekülen umfasst;
   b) Amplifizieren der Targetmoleküle in der Reaktionskammer (1.1) mittels einer zyklischen Amplifikationsreaktion;
   c) Detektieren einer Wechselwirkung zwischen den Targetmolekülen und den auf dem Substrat immobilisierten Sondenmolekülen bei einer Temperatur unterhalb der Annealing-Temperatur eines Amplifikationszykus, wobei in Schritt c) der Abstand zwischen dem Mikroarray (1.5) und der zweiten Fläche so verändert wird, dass die Probenlösung zwischen dem Mikroarray (1.5) und der zweiten Fläche im Wesentlichen entfernt ist.

2. Verfahren nach Anspruch 1,
   wobei der Abstand zwischen Mikroarray und zweiter Fläche vor dem Detektieren in einer Position gehalten wird, die die Wechselwirkung zwischen den Targetmolekülen und den auf dem Substrat immobilisierten Sondenmolekülen ermöglicht.

3. Verfahren nach einer der Ansprüche 1 oder 2,
   wobei die Targetmoleküle mit einem detektierbaren Marker versehen werden.

4. Verfahren nach Anspruch 3,
   wobei der detektierbare Marker ein Fluoreszenzmarker ist.

5. Verfahren nach Anspruch 4,
   wobei das Detektieren der Fluoreszenzmarker mittels eines Fluoreszenzmikroskops ohne Autofokus erfolgt.

6. Verfahren nach Anspruch 4,
   wobei das Detektieren der Fluoreszenzmarker mittels eines Fluoreszenzmikroskops mit Fixfokus erfolgt.

7. Verfahren nach einem der Ansprüche 1-6,
   wobei das Detektieren nach einem oder mehreren Zyklen während der zyklischen Amplifikationsreaktion und/oder nach Abschluss der zyklischen Amplifikationsreaktion erfolgt.

8. Verfahren nach einem der Ansprüche 1-7,
   wobei die zyklische Amplifikationsreaktion eine PCR ist.

9. Verfahren nach einem der Ansprüche 1-8,
   wobei die Detektion bei einer Temperatur unterhalb von 60°C erfolgt.

10. Verfahren nach einem der Ansprüche 1-9,
    wobei die Detektion bei einer Temperatur im Bereich von 25°C bis 50°C, optional im Bereich von 30°C bis 45°C, erfolgt.

Ausgabemonitor

Bedienfeld

AP-Eingabeschacht

Auslesegerät

Prozeßeinheit

Detektionfenster

Aufarbeitungseinheit

Ap500

Probenaufgabe

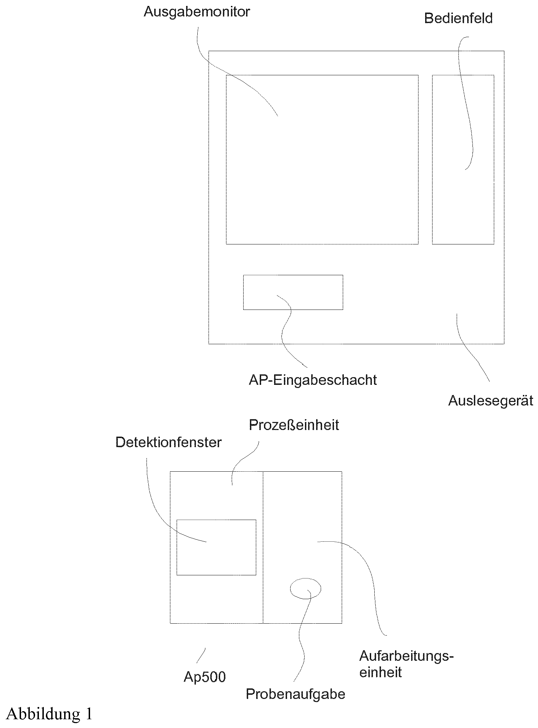

Abbildung 1

Kammerkörper     Dichtung     Druckausgleichskammern

Glashalterung

Reaktionsraum(Kammer)

Heizer/Fühler-
kontakte

Detektionsflächel

DNA-Chip

Kanülenführung

Abbildung 2

Detektionsfläche

Dichtung

DNA-Chip

Elastische Membran

Kammerkörper

Abbildung 3

Abbildung 4

Abbildung 5

C)

Detektionsfläche durch
Kompression
der
Dichtung
zum DNA-Chip
gedrückt

Flüssigkeit ist
verdrängt

Definierter
optischer
Weg vom DNA
Chip zum
Detektor

Dichtung weicht
verdrängter
Flüssigkeit

Stössel
drückt DNA-Chip
gegen Detektionsfläche

B)

Z-Abstandshalter
liegt auf Detektionsfläche

Stössel an
Membranboden

Objektiv

Linse

Z-Abstandshalter

A)          Kammerkörper

Detektionsfläche

Druckausgleichskammern

DNA-Chip

Dichtung

Membranboden
+ Heizmäander

Stössel

B)

Flüssigkeit ist
verdrängt

Ausbreiten
des verdrängten
Flüssikigkeitsvolumens

Dehnung
der
Ausgleichsfalte

Andrücken
des DNA-Chips
an
Detektionsfläche
durch Stössel

A)

Unterseitenloch

Membrandichtung

Ausgleichsfalte

Membranboden
+ Heizmäander
+ Unterseitenloch

Dichtungsmembran

Stössel

Abbildung 6

Temperaturblock
Anealing

Drehteller

Temperaturblock
Denaturierung

Z-Bewegung zum
Wechsel der Position

Drehachse

Temperaturblock
4. Temperatur

Temperaturblock
Extension

Ap500-
Prozeßeinheit

Drehbewegung zum Wechsel der
Position und Temperatur in der
Prozeßeinheit

Abbildung 7

DNA-Chip — Dichtung
— Detektionsfläche
Elastische
Membran
Plastikkörper

Abbildung 8

Abbildung 9

Kammerkörper

Detektionsebene

Elektrische
Anschlüsse für
Heizer, Sensor,
Eprom

Kanülenführung

Abbildung 10

Kammerkörper

Detektionsfläche

DNA-Chip

Dichtung

Heiz/Sensorsubstrat (+Eprom)

Anschlussplatine

Klemmverbinder

Abbildung 11

**Linearität des Fluoreszenzsignals mit steigender Kanaltiefe**

Abbildung 12

**Kanaltiefe 10μm**

Abbildung 13

Abbildung 14

Chip mit Sonden
(Detektionsfläche)

Dichtung

Spaltbreite

Silizium-Chip

Silizium-Heizsubstrat

Stößel

Abbildung 15

Abbildung 16

**Veränderung der Grauwerte bei unterschiedlicher Spaltbreite für den Hintergrund und die Spots mit Hybridisierungslösung 1**

Abbildung 17

Abbildung 18

Abbildung 19

**Vergleich der Signalintensitäten von Prozeßeinheit 1 mit verdrängtem Analyten und Prozeßeinheit 2 mit durch Pufferlösung ausgetauschten Analyten .**

Abbildung 20

(1) Standard DNA-Leiter
(2) Versuchsreaktor 1
(3) Versuchsreaktor 2

Abbildung 21

Abbildung 22

Abbildung 23a

| | |
|---|---|
| | 1.<br> Kontaktierung der Kartusche mit der modularen Befülleinheit bei gleichzeitiger Durchdringung der Dichtung des Kammerkörpers |
| | 2.<br>Einbringen der reaktiven Lösungen in die Reaktionskammer durch geeignete Werkzeuge, wie z.B. eine Standard-Laborpipette |

| | 3.<br>Abtrennung und Entsorgung der Befülleinheit |
|---|---|

Abbildung 23b

Abbildung 24

Abbildung 25a

| | |
|---|---|
| | 1.<br>Einlegen in die Apperatur, Reaktionskartusche und Befülleinheit in Ausgangslage |
| | 2.<br>Penetration der Dichtung und Kontaktierung von Einfüll- und Abfallkanal |

| | |
|---|---|
| | 3.<br>Befüllung des<br>Reaktionsraumes |
| | 4.<br>Trennung der Kanäle von<br>dem Reaktionsraum und<br>Durchführung von<br>Amplifikationsraktionen/<br>Hybridisierung, Detektion<br><br>Wenn für die weitere<br>Prozessierung (Detektion)<br>die Kanäle bzw. externe<br>Abfallbehälter kontaktiert<br>werden sollen, nocheinmal<br>in Stellung wie 3.) |

Abbildung 25b

Abbildung 26

Abbildung 27a

Abbildung 27b

Abbildung 28

Abbildung 29

Abbildung 30

Abbildung 31

Europäisches
Patentamt

European
Patent Office

Office européen
des brevets

**EUROPÄISCHER RECHERCHENBERICHT**

Nummer der Anmeldung

EP 18 18 9028

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WO 01/54814 A2 (MOTOROLA INC [US]; MCGARRY MARK W [US] ET AL.) 2. August 2001 (2001-08-02) * Seiten 29,30; Abbildungen 1-4 * * Seite 34 * ----- | 1-10 | INV. B01L3/00 C12Q1/68 G01N21/03 G01N21/64 |
| Y | US 6 632 641 B1 (BRENNAN THOMAS M [US] ET AL) 14. Oktober 2003 (2003-10-14) * Spalte 53; Beispiel 9 * ----- | 1-10 | ADD. B01L7/00 |
| Y | US 2004/018523 A1 (HAWKINS GEORGE W) 29. Januar 2004 (2004-01-29) * Absätze [0004], [0005], [0013] - [0018] * * Absätze [0019] - [0022], [0048] - [0076]; Abbildungen 7-10 * ----- | 1-10 | |
| A | US 5 863 502 A (SOUTHGATE ET AL) 26. Januar 1999 (1999-01-26) * Spalte 24, Zeile 66 - Spalte 25, Zeile 21 * * Spalte 18, Zeile 55 - Spalte 19, Zeile 53 * * Spalte 11, Zeile 27 - Spalte 12, Zeile 7 * ----- | 1-10 | RECHERCHIERTE SACHGEBIETE (IPC) G01N B01L C12Q |

Der vorliegende Recherchenbericht wurde für alle Patentansprüche erstellt

| Recherchenort | Abschlußdatum der Recherche | Prüfer |
|---|---|---|
| Den Haag | 12. Juni 2019 | Tiede, Ralph |

KATEGORIE DER GENANNTEN DOKUMENTE

X : von besonderer Bedeutung allein betrachtet
Y : von besonderer Bedeutung in Verbindung mit einer
anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes
Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 18 18 9028

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

12-06-2019

| Im Recherchenbericht angeführtes Patentdokument | Datum der Veröffentlichung | Mitglied(er) der Patentfamilie | Datum der Veröffentlichung |
|---|---|---|---|
| WO 0154814 A2 | 02-08-2001 | AU 3459301 A<br>AU 2001234593 B2<br>CA 2398271 A1<br>EP 1251963 A2<br>JP 2003520972 A<br>US 6569674 B1<br>US 2003190744 A1<br>WO 0154814 A2 | 07-08-2001<br>29-09-2005<br>02-08-2001<br>30-10-2002<br>08-07-2003<br>27-05-2003<br>09-10-2003<br>02-08-2001 |
| US 6632641 B1 | 14-10-2003 | AU 1075701 A<br>CA 2386791 A1<br>EP 1235932 A2<br>US 6632641 B1<br>US 2003232382 A1<br>WO 0127327 A2 | 23-04-2001<br>19-04-2001<br>04-09-2002<br>14-10-2003<br>18-12-2003<br>19-04-2001 |
| US 2004018523 A1 | 29-01-2004 | US 6589778 B1<br>US 2004018523 A1 | 08-07-2003<br>29-01-2004 |
| US 5863502 A | 26-01-1999 | AU 1825197 A<br>US 5863502 A<br>WO 9727324 A1 | 20-08-1997<br>26-01-1999<br>31-07-1997 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0012575 A
- US 5412087 A
- WO 9836827 A
- US 5143854 A
- US 5304810 A
- US 5459325 A
- US 5192980 A
- US 5834758 A
- US 5324633 A
- US 6027880 A
- US 5585639 A
- WO 0012759 A
- WO 0072018 A

- WO 0202810 A
- WO 0025113 A
- WO 9627025 A
- US 5760950 A
- WO 9857151 A
- WO 9927140 A
- WO 2004087951 A
- WO 03004699 A
- WO 0102094 A
- WO 9745559 A
- WO 03018838 A
- EP 0456782 A
- DE 10253966

**In der Beschreibung aufgeführte Nicht-Patentliteratur**

- **D. J. LOCKHART ; E. A. WINZELER.** Genomics, gene expression and DNA arrays. *Nature,* 2000, vol. 405, 827-836
- **A.A. LEITCH ; T. SCHWARZACHER ; D. JACKSON ; I. J. LEITCH.** vitro Hybridisierung. Spektrum Akademischer Verlag, 1994
- **C. E. HOOPER et al.** Quantitative Photon Imaging in the Life Sciences Using Intensified CCD Cameras. *Journal of Bioluminescence and Chemiluminescence,* 1990, 337-344
- **A. MARSHALL ; J. HODGSON.** DNA Chips: An array of possibilities. *Nature Biotechnology,* 1998, vol. 16, 27-31
- **G. RAMSAY.** DNA Chips: State of the Art. *Nature Biotechnology,* 16. Januar 1998, 40-44
- **I. GRYCZCYNSKI et al.** Polarisation sensing with visual detection. *Anal. Chem.,* 1999, vol. 71, 1241-1251
- **M. KWIATOWSKI et al.** Solid-phase synthesis of chelate-labelled oligonucleotides: application in triple-color ligase-mediated gene analysis. *Nucleic Acids Research,* 1994, vol. 22, 13
- **M. P. BRUCHEZ.** Semiconductor nanocrystals as fluorescent biological labels. *Science,* 1998, vol. 281, 2013
- **SAMBROOK et al.** Molecular Cloning: A laboratory manual. Cold Spring Harbor Laboratory Press, 2001
- Confocal and 2-photon-microscopy: Foundations. **A. DIASPRO.** Applications and Advances. Wiley-Liss, 2002

- **C. E. HOOPER et al.** Quantitative Photone Imaging in the Life Sciences Using Intensified CCD Cameras. *Journal of Bioluminescence and Chemoluminescence,* 1990, 337-344
- **Z. GUTTENBERG et al.** *Lab Chip.,* 2005, vol. 5 (3), 308-17
- **X. ZHU et al.** *Lab Chip.,* 2004, vol. 4 (6), 581-7
- **J. LIU et al.** *Anal Chem.,* 2005, vol. 77 (9), 2756-2761
- **J. WANG.** *Anal Chem.,* 2003, vol. 75 (15), 3941-5
- **S.M. RADKE et al.** *Biosens Bioelectron.,* 2005, vol. 20 (8), 1662-7
- **B. LIU et al.** *PNAS,* 2005, vol. 102 (3), 589-593
- **K. USUI et al.** *Mol Divers.,* 2004, vol. 8 (3), 209-18
- **J.A. CRUZ-AGUADO et al.** *Anal Chem.,* 2004, vol. 76 (14), 4182-8
- **J. SZOLLOSI et al.** *J Biotechnol.,* 2002, vol. 82 (3), 251-66
- **A. MARSHALL ; J. HODGSON.** DNA chips: An array of possibilities. *Nature Biotechnology,* 1998, vol. 16, 27-31
- **G. RAMSAY.** DNA Chips: State of the art. *Nature Biotechnology,* 1998, vol. 16, 40-44
- **F. LOTTSPEICH ; H. ZORBAS.** Bioanalytik. Spektrum Akademischer Verlag, 1998
- **LOTTSPEICH ; ZORBAS.** Bioanalytik. Spektrum Akademischer Verlag, 1998
- **E. LIDELL ; I. WEEKS.** Antibody Technology. BIOS Scientific Publishers Limited, 1995

- **GALLATI ; PRACHT.** *J. Clin. Chem. Clin. Biochem.,* 1985, vol. 23 (8), 454
- **CHAN et al.** *J. Chem. Soc., Perkin Trans.,* 1999, vol. 1, 315-320
- **KAWAI et al.** *Nucleic Acids Res.,* 1993, vol. 1 (6), 1473-1479
- **SORENSEN et al.** *J. Am. Chem. Soc.,* 2002, vol. 124 (10), 2164-2176
- **SCHONING et al.** *Science,* 2000, vol. 290 (5495), 1347-1351

SEQUENZPROTOKOLL

<110>  CLONDIAG GmbH

<120>  Vorrichtung und Verfahren zum Nachweis von molekularen
        Wechselwirkungen

<130>  C08892EPD1/MH

<140>
<141>

<150>  DE 10 2004 022 263.0
<151>  06.05.2004

<160>  18

<170>  PatentIn version 3.3

<210>  1
<211>  19
<212>  DNA
<213>  Artificial

<220>
<223>  Primer CMV_DP_Cy3

<400>  1
tgaggctggg aarctgaca                                          19


<210>  2
<211>  19
<212>  DNA
<213>  Artificial

<220>
<223>  Primer CMV_UP_NH2

<400>  2
gggygaggay aacgaaatc                                          19


<210>  3
<211>  19
<212>  DNA
<213>  Artificial

<220>
<223>  Primer CMV_UP

<400>  3
gggygaggay aacgaaatc                                          19


<210>  4
<211>  17
<212>  DNA
<213>  Artificial

<220>
<223>  Primer Entero_DP_Cy3

```
<400>  4
ccctgaatgc ggctaat                                                    17


<210>  5
<211>  19
<212>  DNA
<213>  Artificial

<220>
<223>  Primer Entero_UP_NH2

<400>  5
attgtcacca taagcagcc                                                  19


<210>  6
<211>  19
<212>  DNA
<213>  Artificial

<220>
<223>  Primer Entero_UP

<400>  6
attgtcacca taagcagcc                                                  19


<210>  7
<211>  19
<212>  DNA
<213>  Artificial

<220>
<223>  Primer HSV1_DP_Cy3

<400>  7
ctcgtaaaat ggcccctcc                                                  19


<210>  8
<211>  19
<212>  DNA
<213>  Artificial

<220>
<223>  Primer HSV1_UP_NH2

<400>  8
cggccgtgtg acactatcg                                                  19


<210>  9
<211>  19
<212>  DNA
<213>  Artificial

<220>
<223>  Primer HSV1_UP

<400>  9
cggccgtgtg acactatcg                                                  19
```

```
<210>  10
<211>  21
<212>  DNA
<213>  Artificial

<220>
<223>  Primer HSV2_UP_Cy3

<400>  10
cgctctcgta aatgcttccc t                                                    21


<210>  11
<211>  21
<212>  DNA
<213>  Artificial

<220>
<223>  Primer HSV2_DP_NH2

<400>  11
tctacccaca acagacccac g                                                    21


<210>  12
<211>  21
<212>  DNA
<213>  Artificial

<220>
<223>  Primer HSV2_DP

<400>  12
tctacccaca acagacccac g                                                    21


<210>  13
<211>  14
<212>  DNA
<213>  Artificial

<220>
<223>  Primer VZV_DP_Cy3

<400>  13
tcgcgtgctg cggc                                                            14


<210>  14
<211>  17
<212>  DNA
<213>  Artificial

<220>
<223>  Primer VZV_UP_NH2

<400>  14
cggcatggcc cgtctat                                                         17


<210>  15
<211>  17
```

```
<212>  DNA
<213>  Artificial

<220>
<223>  Primer VZV_UP

<400>  15
cggcatggcc cgtctat                                                    17


<210>  16
<211>  21
<212>  DNA
<213>  Artificial

<220>
<223>  Probe CMV_S_21-3

<400>  16
tgttgggcaa ccaccgcact g                                              21


<210>  17
<211>  20
<212>  DNA
<213>  Artificial

<220>
<223>  Primer 16sfD1

<400>  17
agagtttgat cctggctcag                                                20


<210>  18
<211>  27
<212>  DNA
<213>  Artificial

<220>
<223>  Primer 16sRa

<400>  18
taccgtcacc ataaggcttc gtcccta                                        27
```